# EUROPÄISCHE PATENTSCHRIFT

(11) **EP 0 763 534 B1**
(45) Veröffentlichungstag und Bekanntmachung des Hinweises auf die Patenterteilung: **26.02.2003**
(21) Anmeldenummer: 96114211.4
(22) Anmeldetag: 05.09.1996
(51) Int. Cl.: C07D 401/12, C07D 403/12, C07D 413/12, A61K 31/54, A61K 31/50, A61K 31/42

(54) **Arylalkyl-diazinone als Phosphodiesterase IV-Hemmer**
Arylalkyl-diazinone derivatives as phosphodiesterase IV inhibitors
Dérivés d'arylalkyl-diazinone en tant qu'inhibiteurs de la phosphodiestérase de type IV

(30) Priorität: 14.09.1995 DE 19533975
(43) Veröffentlichungstag der Anmeldung: 19.03.1997
(73) Patentinhaber: MERCK PATENT GmbH, 64293 Darmstadt (DE)
(72) Erfinder: Jonas, Rochus Dr., 64291 Darmstadt (DE); Wolf, Michael Dr., 64297 Darmstadt (DE); Beier, Norbert Dr., 64354 Reinheim (DE)

(56) Entgegenhaltungen:
- EP-A- 0 351 213
- EP-A- 0 618 201
- EP-A- 0 721 950
- EP-A- 0 723 962
- EP-A- 0 738 715
- DE-A- 2 243 667

## Beschreibung

Die Erfindung betrifft Arylalkyl-diazinonderivate der Formel I 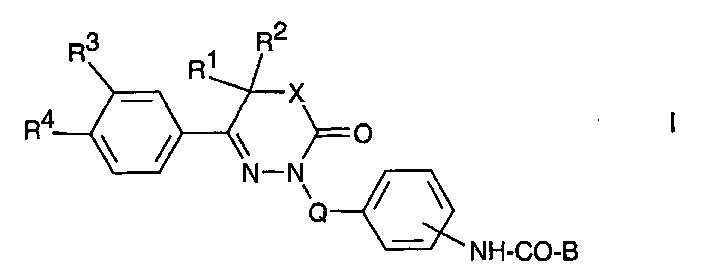 worin
- B: 2- oder 3-Furyl, 2- oder 3-Thienyl, 1-, 2- oder 3-Pyrrolyl, 1-, 2-, 4- oder 5-lmidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 2-, 3-, 4-, 5- 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl,
- Q: fehlt oder Alkylen mit 1-6 C-Atomen,
- X: CH_{2,} S oder O,
- R¹ und R²: jeweils unabhängig voneinander H oder A,
- R³ und R⁴: jeweils unabhängig voneinander -OH, OR⁵, -S-R⁵_{,} -SO-R⁵, -SO₂-R⁵, Hal, Methylendioxy, -NO₂, -NH₂, - NHR⁵ oder -NR⁵R⁶,
- R⁵ und R⁶: jeweils unabhängig voneinander A, Cycloalkyl mit 3-7 C-Atomen, Methylencycloalkyl mit 4-8 C-Atomen oder Alkenyl mit 2-8 C-Atomen,
- A: Alkyl mit 1 bis 10 C-Atomen, das durch 1 bis 5 F- und/oder Cl-Atome substituiert sein kann und
- Hal: F, Cl, Br oder I
bedeuten,
sowie deren physiologisch unbedenklichen Salze.

Thiadiazinonderivate sind beispielsweise aus DE 41 34 893 bekannt. Andere Diazinonderivate sind in EP 0 618 201 A1, EP 0 351 213 A2, DE 2 243 667, EP 0 723 962 A1, EP 0 721 950, EP 0 738 715 A2, EP 0 679 651 A2, EP 0 129 791 A2, Drugs Future **17**, 799 (1992) und in Drug News Perspect **6**, 203 (1993) beschrieben.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen.
Insbesondere zeigen sie eine Phosphodiesterase IV-Hemmung und können zur Behandlung von asthmatischen Erkrankungen eingesetzt werden. Die antiasthmatische Wirkung kann z. B. nach der Methode von T. Olsson, Acta allergologica 26, 438-447 (1971), bestimmt werden.

Die Verbindungen zeigen außerdem eine hemmende Wirkung auf die Bildung von TNF (Tumor Nekrose Faktor) und eignen sich daher zur Behandlung von allergischen und entzündlichen Krankheiten, Autoimmunkrankheiten und Transplantatabstoßungsreaktionen. Sie können ferner zur Behandlung von Gedächtnisstörungen eingesetzt werden.

Die Verbindungen der Formel I können als Arzneimittelwirkstoffe in der Human- und Veterinärmedizin eingesetzt werden. Ferner können sie als Zwischenprodukte zur Herstellung weiterer Arzneimittelwirkstoffe eingesetzt werden.

Gegenstand der Erfindung sind dementsprechend die Verbindungen der Formel I sowie ein Verfahren zur Herstellung von Verbindungen der Formel I sowie ihrer Salze, dadurch gekennzeichnet, daß man eine Verbindung der Formel II 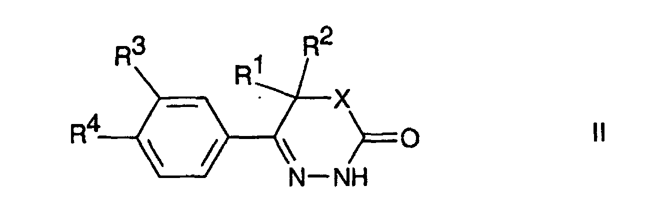 worin
- R¹, R², R³, R⁴ und X: die angegebenen Bedeutungen haben,
mit einer Verbindung der Formel III 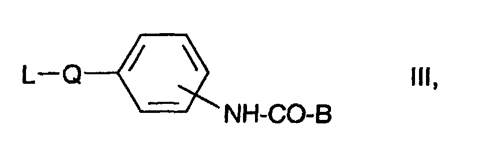 worin
- B und Q: die angegebenen Bedeutungen haben, und
- L: Cl, Br, OH oder eine reaktionsfähige veresterte OH-Gruppe bedeutet,
umsetzt,
oder
daß man eine Verbindung der Formel IV 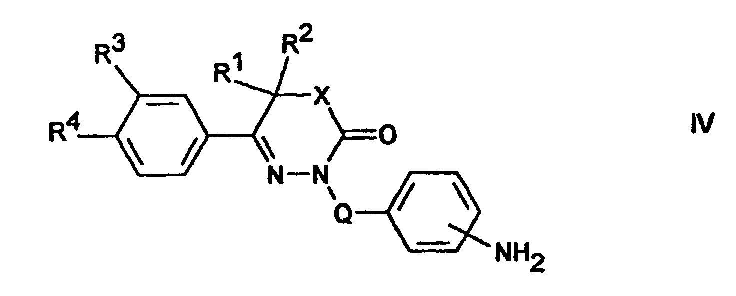 worin
R¹, R², R³, R⁴, Q und X die angegebenen Bedeutungen haben, mit einer Verbindung der Formel V

L-CO-B V

worin
- B: die angegebene Bedeutung hat, und
- L: Cl, Br, OH oder eine reaktionsfähige veresterte OH-Gruppe bedeutet,
umsetzt,
und/oder daß man eine basische Verbindung der Formel I durch Behandeln mit einer Säure in eines ihrer Salze überführt.

Vor- und nachstehend haben die Reste R¹, R², R³, R⁴, B, Q und X die bei den Formeln I, II, III, IV und V angegebenen Bedeutungen, sofern nicht . ausdrücklich etwas anderes angegeben ist.

A bedeutet vorzugsweise Alkyl, weiter bevorzugt durch 1 bis 5 Fluor-und/Oder Chloratome substituiertes Alkyl.

In den vorstehenden Formeln ist Alkyl vorzugsweise unverzweigt und hat 1, 2, 3, 4, 5, 6, 7, 8, 9 oder 10 C-Atome, vorzugsweise 1, 2, 3, 4 oder 5 C-Atome und bedeutet vorzugsweise Methyl, Ethyl, Trifluormethyl, Pentafluorethyl oder Propyl, weiterhin bevorzugt Isopropyl, Butyl, Isobutyl, sek.-Butyl oder tert-Butyl, aber auch n-Pentyl, neo-Pentyl oder Isopentyl.

Cycloalkyl hat vorzugsweise 3-7 C-Atome und steht bevorzugt für Cyclopropyl und Cyclobutyl, weiterhin bevorzugt für Cyclopentyl oder Cyclo-hexyl, femer auch für Cycloheptyl.

Methylencycloalkyl hat vorzugsweise 4-8 C-Atome und steht bevorzugt für Methylencyclopropyl und Methylencyclobutyl, weiterhin bevorzugt für Methylencyclopentyl und Methylencyclohexyl, femer auch für Methylencycloheptyl.

Alkenyl steht vorzugsweise für Vinyl, 1- oder 2-Propenyl, 1-Butenyl, Isobutenyl, sek.-Butenyl, femer bevorzugt ist 1-Pentenyl, iso-Pentenyl oder 1-Hexenyl.

Alkylen ist vorzugsweise unverzweigt und bedeutet bevorzugt Methylen oder Ethylen, femer bevorzugt Propylen oder Butylen.

Von den Resten R¹ und R² steht einer vorzugsweise für H, während der andere bevorzugt Propyl oder Butyl, besonders bevorzugt aber Ethyl oder Methyl bedeutet Femer bedeuten R¹ und R² auch zusammen bevorzugt jeweils Wasserstoff.

Hal bedeutet vorzugsweise F, Cl oder Br, aber auch 1.

Die Reste R³ und R⁴ können gleich oder verschieden sein und stehen in der 3- oder 4-Position des Phenylrings. Sie bedeuten beispielsweise unabhängig voneinander Hydroxy, -S-CH₃, -SO-CH₃, -SO₂CH₃, F, Cl, Br oder I oder zusammen Methylendioxy. Besonders bevorzugt stehen sie aber jeweils für Methoxy, Ethoxy, Propoxy, Cyclopentoxy, oder aber für Fluor-, Difluor-, Trifluormethoxy, 1-Fluor-, 2-Fluor-, 1,2-Difluor-, 2,2-Difluor-, 1,2,2-Trifluor- oder 2,2,2-Trifluorethoxy.

Der Rest B ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4-oder 5-Oxazolyl, 3-, 4- oder 5-Isoxazolyl, 2-, 4- oder 5-Thiazolyl, 3-, 4-oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3-oder 5-yl, 1- oder 5-Tetrazolyl, 1,2,3-Oxadiazol-4- oder -5-yl, 1,2,4-Oxadiazol-3-azol-3- oder -5-yl, 1,3,4-Thiadiazol-2- oder -5-yl, 1,2,4-Thiadiazol-3- oder-5-yl, 1,2,3-Thiadiazol-4- oder -5-yl, 3- oder 4-Pyridazinyl, Pyrazinyl, 2-, 3-, 4-, 5- 6- oder 7-Benzofuryl, 2-, 3-, 4-, 5-, 6- oder 7-Benzothienyl. 1-, 2-, 3-, 4-, 5-, 6- oder 7-Indolyl, 1-, 2-, 4- oder 5-Benzimidazolyl, 1-, 3-, 4-, 5-, 6-oder 7-Benzopyrazolyl, 2-, 4-, 5-, 6- oder 7-Benzoxazolyl, 3-, 4-, 5-, 6-oder 7- Benzisoxazolyl, 2-, 4-, 5-, 6- oder 7-Benzthiazolyl, 2-, 4-, 5-, 6-oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl.

Für die gesamte Erfindung gilt, daß sämtliche Reste, die mehrfach auftreten, gleich oder verschieden sein können, d.h. unabhängig voneinander sind.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformen la bis If ausgedrückt werden, die der Formel I entsprechen und worin die nicht näher bezeichneten Reste die bei der Formel I angegebene Bedeutung haben, worin jedoch
in Ia
   - R¹: H,
   - R²: H oder A,
   - R³: OA und
   - X: S
   bedeuten;
in Ib
   - R¹: H,
   - R²: Methyl oder Ethyl,
   - R³ und R⁴: jeweils unabhängig voneinander OA und
   - X: S
   bedeuten;
in Ic
   - R¹: H,
   - R²: Methyl oder Ethyl.
   - R³: OA
   - R⁴: mono-, di- oder trifluorsubstituiertes Alkyl mit 1 bis 6 C-Atomen und
   - X: S
   bedeuten;
in Id
   - R¹: H,
   - R²: Methyl oder Ethyl,
   - R³ und R⁴: jeweils unabhängig voneinander OR⁵ und
   - B: einen Pyridylrest
   bedeuten;
in Ie
   - R¹ und R²: H,
   - R³ und R⁴: jeweils unabhängig voneinander OA
   - B: einen Pyridylrest und
   - X: S oder CH₂
   bedeuten.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Veriag, Stuttgart; insbesondere aber in der DE 19502699.3), beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

In den Verbindungen der Formeln II und IV haben R¹, R², R³ und R⁴ die angegebenen Bedeutungen, insbesondere die angegebenen bevorzugten Bedeutungen.

In den Verbindungen der Formeln III und V steht Q vorzugsweise für Methylen oder Ethylen, femer bevorzugt für Propylen oder Butylen.

B hat in den Verbindungen der Formeln III und V die angegebenen bevorzugten Bedeutungen, während L Cl, Br, OH oder eine reaktionsfähige veresterte OH-Gruppe bedeutet

Falls L eine reaktionsfähige veresterte OH-Gruppe bedeutet, so ist diese vorzugsweise Alkylsulfonyloxy mit 1-6 C-Atomen (bevorzugt Methyl-sulfonyloxy) oder Arylsulfonyloxy mit 6-10 C-Atomen (bevorzugt Phenyloder p-Tolylsulfonyloxy, femer auch 2-Naphthalinsulfonyloxy).

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.
Andererseits ist es möglich, die Reaktion stufenweise durchzuführen.

Die Verbindungen der Formel I können vorzugsweise erhalten werden, indem man Verbindungen der Formel II mit Verbindungen der Formel III umsetzt.

Die Ausgangsstoffe der Formeln II und III sind teilweise bekannt. Sofern sie nicht bekannt sind, können sie nach an sich bekannten Methoden hergestellt werden.

Pyridazinone der Formel II sind z. B. in Eur. J. Med. Chem. - Chim. Therapeut. 9, 644-650 (1977) beschrieben.

Thiadiazinone der Formel II und ihre Herstellung sind z. B. in der deutschen Patentanmeldung P 41 34 893 beschrieben.

6-Ethyl-1,3,4-oxadiazin-2-one der Formel II können z. B. ausgehend von Butyroveratron, in einer Bromierung über α-Brombutyroveratron, einer Substitution des Br-Atoms durch eine OH-Gruppe mit Hilfe von Kaliumformiat in Methanol, der anschließenden Umsetzung mit Carbomethoxy-hydrazin zum entsprechenden Hydrazonderivat, und Cyclisierung mit Hilfe von Kaliumcarbonat in Toluol erhalten werden.

Im einzelnen erfolgt die Umsetzung der Verbindungen der Formel II mit den Verbindungen der Formel III in Gegenwart oder Abwesenheit eines inerten Lösungsmittels bei Temperaturen zwischen etwa -20 und etwa 150°, vorzugsweise zwischen 20 und 100°.

Als inerte Lösungsmittel eignen sich z.B. Kohlenwasserstoffe wie Hexan, Petrolether, Benzol, Toluol oder Xylol; chlorierte Kohlenwassertoffe wie Trichlorethylen, 1,2 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmono-methyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethytformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Verbindungen der Formel I können weiterhin erhalten werden, indem man Verbindungen der Formel IV mit Verbindungen der Formel V umsetzt.
Die Ausgangsverbindungen der Formeln IV und V sind in der Regel bekannt. Sind sie nicht bekannt, so können sie nach an sich bekannten Methoden hergestellt werden.
Verbindungen der Formel IV sind insbesondere aus DE 19502699 und DE 19514568 bekannt.

In den Verbindungen der Formel V bedeutet der Rest -CO-L eine voraktivierte Carbonsäure, vorzugsweise ein Carbonsäurehalogenid.

Die Umsetzung der Verbindungen der Formel IV mit Verbindungen der Formel V erfolgt unter den gleichen Bedingungen, betreffend die Reaktionszeit. Temperatur und Lösungsmittel, wie dies für die Umsetzung der Verbindungen der Formel II mit Verbindungen der Formel III beschrieben ist.

Eine Base der Formel 1 kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, femer organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansutfonsäure, Ethandisutfonsäure, 2 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und loder Aufreinigung der Verbindungen der Formel I verwendet werden.

Andererseits können, falls gewünscht, die freien Basen der Formel I aus ihren Salzen mit Basen (z.B. Natrium- oder Kaliumhydroxid oder-carbonat) in Freiheit gesetzt werden.

Verbindungen der Formel I können ein oder mehrere Asymmetriezentren enthalten. In diesem Fall liegen sie gewöhnlich in racemischer Form vor. Erhaltene Racemate können nach an sich bekannten Methoden mechanisch oder chemisch in ihre Enantiomeren getrennt werden. Vorzugsweise werden aus dem racemischen Gemisch durch Umsetzung mit einem optisch-aktiven Trennmittel Diastereomere gebildet.

Natürlich ist es auch möglich, optisch aktive Verbindungen der Formel I nach den oben beschriebenen Methoden zu erhalten, indem man Ausgangsstoffe verwendet, die bereits optisch aktiv sind.

Die Formel I umschließt alle Stereoisomeren und deren Gemische, z.B. die Racemate.

Gegenstand der Erfindung ist ferner die Verwendung der Verbindungen der Formel I undloder ihrer physiologisch unbedenklichen Salze zur Herstellung pharmazeutischer Zubereitungen, insbesondere auf nichtchemischem Wege. Hierbei können sie zusammen mit mindestens einem festen, flüssigen undloder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind auch Arzneimittel der Formel I und ihre physiologisch unbedenklichen Salze als Phosphodiesterase IV-Hemmer.

Gegenstand der Erfindung sind femer pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungsund/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die Verbindungen der Formel I und ihre physiologisch unbedenklichen Salze können bei der Bekämpfung von Krankheiten, bei denen eine Erhöhung des cAMP(cyclo-Adenosin-monophosphat)-Spiegels zu Entzündungshemmung oder -verhinderung und Muskelentspannung führt, eingesetzt werden. Besondere Verwendung können die erfindungsgemäßen Verbindungen bei der Behandlung von Allergien, Asthma, chronischer Bronchitis, atopischer Dermatitis, Psoriasis und anderer Hautkrankheiten und Autoimmunerkrankungen finden.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und loder durch Kristallisation.
- Massenspektrometrie (MS):: EI (Elektronenstoß-Ionisation) M⁺ FAB (Fast Atom Bombardment) (M+H)⁺

### Beispiel 1

Eine Suspension von 4,70 g 6-(3,4-Dimethoxyphenyl)-2,3,4,5-tetrahydropyridazin-3-on ("A") in 150 ml THF wird mit 2,24 g Kalium-tert-butylat versetzt und 30 Minuten gerührt. Man gibt 7,32 g 4-Nicotinoylaminobenzylchlorid dazu und rührt 10 Stunden bei Raumtemperatur nach. Das Lösungsmittel wird entfernt und wie üblich aufgearbeitet. Man erhält 2-(4-Nicotinoylamino-benzyl)-6-(3,4-dimethoxyphenyl)-2,3,4,5-tetrahydropyridazin-3-on.

Analog erhält man durch Umsetzung von "A"
mit 4-lsonicotinoylamino-benzylchlorid:
   2-(4-lsonicotinoylamino-benzyl)-6-(3,4-dimethoxyphenyl)-2,3,4,5-tetrahydro-pyridazin-3-on.

### Beispiel

Eine Lösung von 3,4 g 2-(4-Aminobenzyl)-6-(3,4-dimethoxyphenyl)-2,3,4,5-tetrahydro-pyridazin-3-on, F. 184° und 0,75 ml Pyridin in 100 ml Dichlormethan wird mit 1,4 g Nicotinoylchlorid versetzt und eine Stunde nachgerührt Man entfernt das Lösungsmittel und arbeitet wie üblich auf. Nach Umkristallisation erhält man 2-(4-Nicotinoylamino-benzyl)+(3,4-dimethoxyphenyl)-2,3,4,5-tetrahydro-pyridazin-3-on.

Analog erhält man durch Umsetzung der nachstehenden "Aminderivate"
2-(3-Aminobenzyl)-6-(3,4-dimethoxyphenyl)-2,3,4,5-tetrahydro-pyridazin-3-on, F. 140°
2-(2-Aminobenzyl)-6-(3,4-dimethoxyphenyl)-2,3,4,5-tetrahydro-pyridazin-3-on
2-(4-Aminobenzyl)-6-(3,4-dimethoxyphenyl)-5-ethyl-2,3,4,5-tetrahydro-pyridazin-3-on;
2-(3-Aminobenzyl)-6-(3,4-dimethoxyphenyl)-5-ethyl-2,3,4,5-tetrahydro-pyridazin-3-on, F. 49°
2-(2-Aminobenzyl)-6-(3,4-dimethoxyphenyl)-5-ethyl-2,3,4,5-tetrahydro-pyridazin-3-on
2-(4-Aminobenzyl)-6-(3-methoxy-4-trifluormethoxyphenyl)-5-ethyl-2,3,4,5-tetrahydro-pyridazin-3-on
2-(4-Aminobenzyl)-6-(3-methoxy-4-difluormethoxyphenyl)-5-ethyl-2,3,4,5-tetrahydro-pyridazin-3-on
2-(4-Aminobenzyl)-6-(3-methoxy-4-fluormethoxyphenyl)-5-ethyl-2,3,4,5-tetrahydro-pyridazin-3-on
2-(4-Aminobenzyl)-6-(3-difluonnethoxy-4-methoxyhenyl)-5-ethyl-2,3,4,5-tetrahydro-pyridazin-3-on
2-(4-Aminobenzyl)-6-(3-trifluormethoxy-4-methoxyphenyl)-5-ethyl-2,3,4,5-tetrahydro-pyridazin-3-on
2-(4-Aminobenzyl)-6-(3-fluormethoxy-4-methoxyphenyl)-5-ethyl-2,3,4,5-tetrahydro-pyridazin-3-on
2-(4-Aminobenzyl)-6-(3-methoxy-4-ethoxyphenyl)-5-ethyl-2,3,4,5-tetrahydro-pyridazin-3-on
2-(4-Aminobenzyl)-6-(3-ethoxy-4-methoxyphenyl)-5-ethyl-2,3,4,5-tetrahydro-pyridazin-3-on
2-(4-Aminobenzyl)-6-(3-hydroxy-4-methoxyphenyl)-5-ethyl-2,3,4,5-tetrahydro-pyridazin-3-on
2-(4-Aminobenzyl)-6-(4-methylsulfonylphenyl)-5-ethyl-2,3,4,5-tetrahydro-pyridazin-3-on
2-(4-Aminobenzyl)-6-(4-methylenoxyphenyl)-5-ethyl-2,3,4,5-tetra-hydro-pyridazin-3-on
2-(4-Aminobenzyl)-6-(3-cyclopentyloxy-4-methoxyphenyl)-5-ethyl-2,3,4,5-tetrahydro-pyridazin-3-on
2-(3-Aminobenzyl)-6-(3-cyclopentyloxy-4-methoxyphenyl)-5-ethyl-2,3,4,5-tetrahydro-pyridazin-3-on, F. 109°
2-(4 Aminophenethyl)-6-(3,4-dimethoxyphenyl)-2,3,4,5-tetrahydro-pyridazin-3-on
2-(4-Aminophenethyl)-6-(3,4-dimethoxyphenyl)-5-ethyl-2,3,4,5-tetra-hydro-pyridazin-3-on
3-(4-Aminobenzyl)-5-(3,4-dimethoxyphenyl)-3,6-dihydro-1,3,4-thiadiazin-2-on
3-(3-Aminobenzyl)-5-(3,4-dimethoxyphenyl)-3,6-dihydro-1,3,4-thiadiazin-2-on
3-(2-Aminobenzyl)-5-(3,4-dimethoxyphenyl)-3,6-dihydro-1,3,4-thiadiazin-2-on
3-(4-Aminobenzyl)-5-(3,4-dimethoxyphenyl)-6-ethyl-3,6-dihydro-1,3,4-thiadiazin-2-on, F. 105°
3-(3-Aminobenzyl)-5-(3,4-dimethoxyphenyl)-6-ethyl-3,6-dihydro-1,3,4-thiadiazin-2-on. F. 112°
3-(2-Aminobenzyl)-5-(3,4-dimethoxyphenyl)-6-ethyl-3,6-dihydro-1,3,4-thiadiazin-2-on
3-(4-Aminobenzyl)-5-(3-methoxy-4 trifluormethoxyphenyl)-6-ethyl-3,6-dihydro-1,3,4-thiadiazin-2-on
3-(4-Aminobenzyl)-5-(3-methoxy-4-difluormethoxyphenyl)-6-ethyl-3,6-dihydro-1,3,4-thiadiazin-2-on
3-(4-Aminobenzyl)-5-(3-methoxy-4-fluormethoxyphenyl)-6-ethyl-3,6-dihydro-1,3,4-thiadiazin-2-on
3-(4-Aminobenzyl)-5-(3-difluormethoxy-4-methoxyphenyl)-6-ethyl-3,8-dihydro-1,3,4-thiadiazin-2-on
3-(4-Aminobenzyl)-5-(3-trifluormethoxy-4-methoxyphenyl)-6-ethyl-3,6-dihydro-1,3,4-thiadiazin-2-on
3-(4-Aminobenzyl)-5-(3-fluormethoxy-4-Methoxyphenyl)-6-ethyl-3,6-dihydro-1,3,4-thiadiazin-2-on
3-(4-Aminobenzyl)-5-(3-methoxy-4-ethoxyphenyl)-6-ethyl-3,6-dihydro-1,3,4-thiadiazin-2-on
3-(4-Aminobenzyl)-5-(3-ethoxy-4-methoxyphenyl)-6-ethyl-3,6-dihydro-1,3,4-thiadiazin-2-on
3-(4-Aminobenzyl)-5-(3-hydroxy-4-methoxyphenyl)-6-ethyl-3,6-dihydro-1,3,4-thiadiazin-2-on
3-(4-Aminobenzyl)-5-(4-methylsulfonylphenyl)-6-ethyl-3,6-dihydro-1,3,4-thiadiazin-2-on
3-(4-Aminobenzyl)-5-(4-methylenoxyphenyl)-6-ethyl-3,6-dihydro-1,3,4-thiadiazin-2-on
3-(4-Aminobenzyl)-5-(3-cyclopentyloxy-4-methoxyphenyl)-6-ethyl-3,6-dihydro-1,3,4-thiadiazin-2-on, F. 132°
3-(3-Aminobenzyl)-5-(3-cyclopentyloxy-4-methoxyphenyl)-6-ethyl-3,6-dihydro-1,3,4-thiadiazin-2-on
3-(4-Aminophenethyl)-5-(3,4-dimethoxyphenyl)-3,6-dihydro-1,3,4-thiadiazin-2-on
3-(4-Aminophenethyl)-5-(3,4-dimethoxyphenyl)-6-ethyl-3,6-dihydro-1,3,4-thiadiazin-2-on
3-(4-Aminobenzyl)-5-(3,4-dimethoxyphenyl)-3,6-dihydro-1,3,4-oxadiazin-2-on
3-(3-Aminobenzyl)-5-(3,4-dimethoxyphenyl)-3.6-dihydro -1,3,4-oxadjazin-2-on
3-(2-Aminobenzyl)-5-(3,4-dimethoxyphenyl)-3,6-dihydro-1,3,4-oxadiazin-2-on
3-(4-Aminobenzyl)-5-(3,4-dimethoxyphenyl)-6-ethyl-3,6-dihydro-1,3,4-oxadiazin-2-on
3-(3-Aminobenzyl)-5-(3,4-dimethoxyphenyl)-6-ethyl-3,6-dihydro-1,3,4-oxadiazin-2-on
3-(2-Aminobenzyl)-5-(3,4-dimethoxyphenyl)4-ethyl-3,6-dihydro-1,3,4-oxadiazin-2-on
3-(4-Aminobenzyl)-5-(3-methoxy-4-trifluormethoxyphenyl)-6-ethyl-3,6-dihydro-1,3,4-oxadiazin-2-on
3-(4-Aminobenzyl)-5-(3-methoxy-4-difluormethoxyphenyl)-6-ethyl-3,6-dihydro-1,3,4-oxadiazin-2-on
3-(4-Aminobenzyl)-5-(3-methoxy-4 fluormethoxyphenyl)-6-ethyl-3,6-dihydro-1,3,4-oxadiazin-2-on
3-(4-Aminobenzyl)-5-(3-difluormethoxy-4-methoxyphenyl)-6-ethyl-3,6-dihydro-1,3,4-oxadiazin-2-on
3-(4-Aminobenzyl)-5-(3-trifluormethoxy-4-methoxyphenyl)-6-ethyl-3,6-dihydro-1,3,4-oxadiazin-2-on
3-(4-Aminobenzyl)-5-(3-fluormethoxy-4-methoxyphenyl)-6-ethyl-3,6-dihydro-1,3,4-oxadiazin-2-on
3-(4-Aminobenzyl)-5-(3-Methoxy-4-ethoxyphenyl)-6-ethyl-3,6-dihydro-1,3,4-oxadiazin-2-on
3-(4-Aminobenzyl)-5-(3-Ethoxy-4-methoxyphenyl)-6-ethyl-3,6-dihydro-1,3,4-oxadiazin-2-on
3-(4-Aminobenzyl)-5-(3-hydroxy-4-methoxyphenyl)-6-ethyl-3,6-d(hydro-1,3,4-oxadiazin-2-on
3-(4-Aminobenzyl)-5-(4-methylsulfonylphenyl)-6-ethyl-3,6-dihydro-1,3,4-oxadiazin-2-on
3-(4-Aminobenzyl)-5-(4-methylenoxyphenyl)-6-ethyl-3,6-dihydro-1,3,4-oxadiazin-2-on
3-(4 Aminobenzyn-5-(3-cyclopentyloxy-4-methoxyphenyl)-6-ethyl-3,6-dihydro-1,3,4-oxadiazin-2-on
3-(3-Aminobenzyl)-5-(3-cydopentyloxy-4-methoxyphenyl)-6-ethyl-3,6-dihydro-1,3,4-oxadiazin-2-on
3-(4-Aminophenethyl)-5-(3,4-dimethoxyphenyl)-3,6-dihydro-1,3,4-oxadiazin-2-on
3-(4-Aminophenethyl)-5-(3,4-dimethoxyphenyl)-6-ethyl-3,6-dihydro-1,3,4-oxadiazin-2-on
mit Nicotinoylchlorid die nachstehenden Verbindungen
2-(3-Nicotinoylamino-benzyl)-6-(3,4-dimethoxyphenyl)-2,3,4,5-tetrahydro-pyridazin-3-on
2-(2-Nicotinoylamino-benzyl)-6-(3,4-dimethoxyphenyl)-2,3,4,5-tetrahydro-pyridazin-3-on
2-(4-Nicotinoylamino-benzyl)-6-(3,4-dimethoxyphenyl)-5-ethyl-2,3,4,5-tetrahydro-pyridazin-3-on;
2-(3-Nicotinoylamino-benzyl)-6-(3,4-dimethoxyphenyl)-5-ethyl-2,3,4,5-tetrahydro-pyridazin-3-on
2-(2-Nicotinoylamino-benzyl)-6-(3,4-dimethoxyphenyl)-5-ethyl-2,3,4,5-tetrahydro-pyridazin-3-on
2-(4-Nicotinoylamino-benzyl)-6-(3-methoxy-4-trifluormethoxyphenyl)-5-ethyl-2,3,4,5-tetrahydro-pyridazin-3-on
2-(4-Nicotinoylamino-benzyl)-6-(3-methoxy-4-difluormethoxyphenyl)-5-ethyl-2,3,4,5-tetrahydro-pyridazin-3-on
2-(4-Nicotinoylamino-benzyl)-6-(3-methoxy-4-fluormethoxyphenyl)-5-ethyl-2,3,4,5-tetrahydro-pyridazin-3-on
2-(4-Nicotinoylamino-benzyl)-6-(3-difluormethoxy-4-methoxyphenyl)-5-ethyl-2,3,4,5-tetrahydro-pyridazin-3-on
2-(4-Nicotinoylamino-benzyl)-6-(3-trifluormethoxy-4-methoxyphenyl)-5-ethyl-2,3,4,5-tetrahydro-pyridazin-3-on
2-(4-Nicotinoylamino-benzyl)-6-(3 fluormethoxy-4-methoxyphenyl)-5-ethyl-2,3,4,5-tetrahydro-pyridazin-3-on
2-(4-Nicotinoylamino-benzyl)-6-(3-methoxy-4-ethaxyphenyl)-5-ethyl-2,3,4,5-tetrahydro-pyridazin-3-on
2-(4-Nicotinoylamino-benzyl)-6-(3-Ethoxy-4-methoxyphenyl)-5-ethyl-2,3,4,5-tetrahydro-pyridazin-3-on
2-(4-Nicotinoylamino-benzyl)-6-(3-hydroxy-4-methoxyphenyl)-5-ethyl-2,3,4,5-tetrahydro-pyridazin-3-on
2-(4-Nicotinoylamino-benzyl)-6-(4-methylsulfonylphenyl)-5-ethyl-2,3,4,5-tetrahydro-pyridazin-3-on
2-(4-Nicotinoylamino-benzyl)-6-(4-methylenoxyphenyl)-5-ethyl-2,3,4,5-tetrahydro-pyridazin-3-on
2-(4-Nicotinoylamino-benzyl)-6-(3-cyclopentyloxy-4-methoxyphenyl)-5-ethyl-2,3,4,5-tetrahydro-pyridazin-3-on
2-(3-Nicotinoylamino-benzyl)-6-(3-cyclopentytoxy-4-methoxyphenyl)-5-ethyl-2,3,4,5-tetrahydro-pyridazin-3-on
2-(4-Nicotinoylamino-phenethyl)-6-(3,4-dimethoxyphenyl)-2,3,4,5-ethyl-hydro-pyridazin-3-on
2-(4-Nicotinoylamino-phenethyl)-6-(3,4-dimethoxyphenyl)-5-ethyl-2,3,4,5-tetrahydro-pyridazin-3-on
3-(4-Nicotinoylamino-benzyl)-5-(3,4-dimethoxyphenyl)-3,6-dihydro-1,3,4-thiadiazin-2-on
3-(3-Nicotinoylamino-benzyl)-5-(3,4-dimethoxyphenyl)-3,6-dihydro-1,3,4-thiadiazin-2-on
3-(2-Nicotinoylamino-benzyl)-5-(3,4-dimethoxyphenyl)-3,6-dihydro-1,3,4-thiadiazin-2-on
3-(4-Nicotinoylamino-benzyl)-5-(3,4-dimethoxyphenyl)-6-ethyl-3,6-dihydro-1,3,4-thiadiazin-2-on
3-(3-Nicotinoylamino-benzyl)-5-(3,4-dimethoxyphenyl)-6-ethyl-3,6-dihydro-1,3,4-thiadiazin-2-on
3-(2-Nicotinoylamino-benzyl)-5-(3,4-dimethoxyphenyl)-6-ethyl-3,6-dihydro-1,3,4-thiadiazin-2-on
3-(4-Nicotinaylamino-benzyl)-5-(3-methoxy-4-trifluormethoxyphenyl)-6-ethyl-3,6-dihydro-1,3,4-thiadiazin-2-on
3-(4-Nicotinoylamino-benzyl)-5-(3-methoxy-4-difluormethoxyphenyl)-6-ethyl-3,6-dihydro-1,3,4-thiadiazin-2-on
3-(4-Nicotinoylamino-benzyl)-5-(3-methoxy-4-fluormethoxyphenyl)-6-ethyl-3,6-dihydro-1,3,4-thiadiazin-2-on
3-(4-Nicotinoylamino-benzyl)-5-(3-difluormethoxy-4-methoxyphenyl)-6-ethyl-3,6-dihydro-1,3,4-thiadiazin-2-on
3-(4-Nicotinoylamino-benzyl)-5-(3-trifluormethoxy-4-methoxyphenyl)-6-ethyl-3,6-dihydro-1,3,4-thiadiazin-2-on
3-(4-Nicotinoylamino-benzyl)-5-(3-fluormethoxy-4-methoxyphenyl)-6-ethyl-3,6-dihydro-1,3,4-thiadiazin-2-on
3-(4-Nicotinoylamino-benzyl)-5-(3-methoxy-4-ethoxyphenyl)-6-ethyl-3,6-dihydro-1,3,4-thiadiazin-2-on
3-(4-Nicotinoylamino-benzyl)-5-(3-ethoxy-4-methoxyphenyl)-6-ethyl-3,6-dihydro-1,3,4-thiadiazin-2-on
3-(4-Nicotinoylamino-benzyl)-5-(3-hydroxy-4-methoxyphenyl)-6-ethyl-3,6-dihydro-1,3,4-thiadiazin-2-on
3-(4-Nicotinoylamino-benzyl)-5-(4-methylsulfonylphenyl)-6-ethyl-3,6-dihydro-1,3,4-thiadiazin-2-on
3-(4-Nicotinoylamino-benzyl)-5-(4-methylenoxyphenyl)-6-ethyl-3,6-dihydro-1,3,4-thiadiazin-2-on
3-(4-Nicotinoylamino-benzyl)-5-(3-cyclopentyloxy-4-methoxyphenyl)-6-ethyl-3,6-dihydro-1,3,4-thiadiazin-2-on
3-(3-Nicotinoylamino-benzyl)-5-(3-cyclopentyloxy-4-methoxyphenyl)-6-ethyl-3,6-dihydro-1,3,4-thiadiazin-2-on
3-(4-Nicotinoylamino-phenethyl)-5-(3,4-dimethoxyphenyl)-3,6-dihydro-1,3,4-thiadiazin-2-on
3-(4-Nicotinoylamino-phenethyl)-5-(3,4-dimethoxyphenyl)-6-ethyl-3,6-dihydro-1,3,4-thiadiazin-2-on
3-(4-Nicotinoylamino-benzyl)-5-(3,4-dimethoxyphenyl)-3,6-dihydro-1,3,4-oxadiazin-2-on
3-(3-Nicotinoylamino-benzyl)-5-(3,4-dimethoxyphenyl)-3,6-dihydro-1,3,4-oxadiazin-2-on
3-(2-Nicotinoylamino-benzyl)-5-(3,4-dimethoxyphenyl)-3,6-dihydro-1,3,4-oxadiazin-2-on
3-(4-Nicotinoylamino-benzyl)-5-(3,4-dimethoxyphenyl)-6-ethyl-3,6-dihydro-1,3,4-oxadiazin-2-on
3-(3-Nicotinoylamino-benzyl)-5-(3,4-dimethoxyphenyl)-6-ethyl-3,6-dihydro-1,3,4-oxadiazin-2-on
3-(2-Nicotinoylamino-benzyl)-5-(3,4-dimethoxyphenyl)-6-ethyl-3,6-dihydro-1,3,4-oxadiazin-2-on
3-(4-Nicotinoylamino-benzyl)-5-(3-methoxy-4-trifluormethoxyphenyl)-6-ethyl-3,6-dihydro-1,3,4-oxadiazin-2-on
3-(4-Nicotinoylamino-benzyl)-5-(3-methoxy-4-difluormethoxyphenyl)-6-ethyl-3,6-dihydro-1,3,4-oxadiazin-2-on
3-(4-Nicotinoylamino-benzyl)-5-(3-methoxy-4-fluormethoxyphenyl)-6-ethyl-3,6-dihydro-1,3,4-oxadiazin-2-on
3-(4-Nicotinoylamino-benzyl)-5-(3-difluormethoxy-4-methoxyphenyl)-6-ethyl-3,6-dihydro-1,3,4-oxadiazin-2-on
3-(4-Nicotinoylamino-benzyl)-5-(3-frifluormethoxy-4-methoxyphenyl)-6-ethyl-3,6-dihydro-1,3,4-oxadiazin-2-on
3-(4-Nicotinoylamino-benzyl)-5-(3-fluormethoxy-4-methoxyphenyl)-6-ethyl-3,6-dihydro-1,3,4-oxadiazin-2-on
3-(4-Nicotinoylamino-benzyl)-5-(3-methoxy-4-ethoxyphenyl)-6-ethyl-3,6-dihydro-1,3,4-oxadiazin-2-on
3-(4-Nicotinoylamino-benzyl)-5-(3-ethoxy-4-methoxyphenyl)-6-ethyl-3,6-dihydro-1,3,4-oxadiazin-2-on
3-(4-Nicotinoylamino-benzyl)-5-(3-hydroxy-4-methoxyphenyl)-6-ethyl-3,6-dihydro-1,3,4-oxadiazin-2-on
3-(4-Nicotinoylamino-benzyl)-5-(4-methylsulfonylphenyl)-6-ethyl-3,6-dihydro-1,3,4-oxadiazin-2-on
3-(4-Nicotinoylamino-benzyl)-5-(4-methylenoxyphenyl)-6-ethyl-3,6-dihydro-1,3,4-oxadiazin-2-on
3-(4-Nicotinoylamino-benzyl)-5-(3-cyclopentyloxy-4-methoxyphenyl)-6-ethyl-3,6-dihydro-1,3,4-oxadiazin-2-on
3-(3-Nicotinoylamino-benzyl)-5-(3-cyclopentyloxy-4-methoxyphenyl)-6-ethyl-3,6-dihydro-1,3,4-oxadiazin-2-on
3-(4-Nicotinoylamino-phenethyl)-5-(3,4-dimethoxyphenyl)-3,6-dihydro-1,3,4-oxadiazin-2-on
3-(4-Nicotinoylamino-phenethyl)-5-(3,4-dimethoxyphenyl)-6-ethyl-3,6-dihydro-1,3,4-oxadiazin-2-on.

Analog erhält man durch Umsetzung der oben aufgeführten "Aminderivate" mit Isonicotinoylchlorid die nachstehenden Verbindungen
2-(4-Isonicotinoylamino-benzyl)-6-(3,4-dimethoxyphenyl)-2,3,4,5-tetrahydro-pyridazin-3-on
2-(3-Isonicotinoylamino-benzyl)-6-(3,4-dimethoxyphenyl)-2,3,4,5-tetrahydro-pyridazin-3-on
2-(2-Isonicotinoylamino-benzyl)-6-(3,4-dimethoxyphenyl)-2,3,4,5-tetrahydro-pyridazin-3-on
2-(4-lsonicotinoylamino-benzyl)-6-(3,4-dimethoxyphenyl)-5-ethyl-2,3,4,5-tetrahydro-pyridazin-3-on;
2-(3-Isonicotinoylamino-benzyl)-6-(3,4-dimethoxyphenyl)-5-ethyl-2,3,4,5-tetrahydro-pyridazin-3-on
2-(2-lsonicotinoylamino-benzyl)-6-(3,4-dimethoxyphenyl)-5-ethyl-2,3,4,5-tetrahydro-pyridazin-3-on
2-(4-Isonicotinoylamino-benzyl)-6-(3-methoxy-4-trifluomethoxyphenyl)-5-ethyl-2,3,4,5-tetrahydro-pyridazin-3-on
2-(4-Isonicotinoylamino-benzyl)-6-(3-methoxy-4-difluormethoxyphenyl)-5-ethyl-2,3,4,5-tetrahydro-pyridazin-3-on
2-(4-Isonicotinoylamino-benzyl)-6-(3-methoxy-4-fluormethoxyphenyl)-5-ethyl-2,3,4,5-tetrahydro-pyridazin-3-on
2-(4-Isonicotinoylamino-benzyl)-6-(3-difluormethoxy-4-methoxyphenyl)-5-ethyl-2,3,4,5-tetrahydro-pyridazin-3-on
2-(4-Isonicotinoylamino-benzyl)-6-(3-trifluormethoxy-4-methoxyphenyl)-5-ethyl-2,3,4,5-tetrahydro-pyridazin-3-on
2-(4-lsonicotinoylamino-benzyl)-6-(3-fluormethoxy-4-methoxyphenyl)-5-ethyl-2,3,4,5-tetrahydro-pyridazin-3-on
2-(4-Isonicotinoylamino-benzyl)-6-(3-Methoxy-4-ethoxyphenyl)-5-ethyl-2,3,4,5-tetrahydro-pyridazin-3-on
2-(4-Isonicotinoylamino-benzyl)-6-(3-ethoxy-4-methoxyphenyl)-5-ethyl-2,3,4,5-tetrahydro-pyridazin-3-on
2-(4-Isonicotinoylamino-benzyl)-6-(3-hydroxy-4-methioxyphenyl)-5-ethyl-2,3,4,5-tetrahydro-pyridazin-3-on
2-(4-lsonicotinoylamino-benzyl)-6-(4-methylsulfonylphenyl)-5-ethyl-2,3,4,5 tetrahydro-pyridazin-3-on
2-(4-Isonicotinoylamino-benzyl)-6-(4-methylenoxyphenyl)-5-ethyl-2,3,4,5-tetrahydro-pyridazin-3-on
2-(4-lsonicotinoylamino-benzyl)-6-(3-cyclopentyloxy-4-methoxyphenyl)-5-ethyl-2,3,4,5-tetrahydro-pyridazin-3-on
2-(3-isonicotinoylamino-benzyl)-6-(3-cyclopentyloxy-4-methoxyphenyl)-5-ethyl-2,3,4,5-tetrahydro-pyridazin-3-on
2-(4-Isonicotinoylamino-phenethyl)-6-(3,4-dimethoxyphenyl)-2,3,4,5-tetrahydro-pyridazin-3-on
2-(4-Isonicotinoylamino-phenethyl)-6-(3,4-dimethoxyphenyl)-5-ethyl-2,3,4,5-tetrahydro-pyridazin-3-on
3-(4-lsonicotinoylamino-benzyl)-5-(3,4-dimethoxyphenyl)-3,6-dihydro-1,3,4-thiadiazin-2-on
3-(3-Isonicotinoylamino-benzyl)-5-(3,4-dimethoxyphenyl)-3,6-dihydro-1,3,4-thiadiazin-2-on
3-(2-Isonicotinoylamino-benzyl)-5-(3,4-dimethoxyphenyl)-3,6-dihydro-1,3,4-thiadiazin-2-on
3-(4-Isonicotinoylamino-benzyl)-5-(3,4-dimethoxyphenyl)-6-ethyl-3,6-dihydro-1,3,4-thiadiazin-2-on
3-(3-Isonicotinoylamino-benzyl)-5-(3,4-dimethoxyphenyl)-6-ethyl-3,6-dihydro-1,3,4-thiadiazin-2-on
3-(2-Isonicotinoylamino-benzyl)-5-(3,4-dimethoxyphenyl)-6-ethyl-3,6-dihydro-1,3,4-thiadiazin-2-on
3-(4-Isonicotinoylamino-benzyl)-5-(3-methoxy-4-trifluormethoxyphenyl)-6-ethyl-3,6-dihydro-1,3,4-thiadiazin-2-on
3-(4-lsonicotinoylamino-benzyl)-5-(3-methoxy-4-difluormethoxyphenyl)-6-ethyl-3,6-dihydro-1,3,4-thiadiazin-2-on
3-(4-lsonicotinoylamino-benzyl)-5-(3-methoxy-4-fluormethoxyphenyl)-6-ethyl-3,6-dihydro-1,3,4-thiadiazin-2-on
3-(4-Isonicotinoylamino-benzyl)-5-(3-difluormethoxy-4-methoxyphenyl)-6-ethyl-3,6-dihydro-1,3,4-thiadiazin-2-on
3-(4-Isonicotinoylamino-benzyl)-5-(3-trifluormethoxy-4-methoxyphenyl)-6-ethyl-3,6-dihydro-1,3,4-thiadiazin-2-on
3-(4-Nicotinoylamino-benzyl)-5-(3-fluormethoxy-4-methoxyphenyl)-6-ethyl-3,6-dihydro-1,3,4-thiadiazin-2-on
3-(4-Isonicotinoylamino-benzyl)-5-(3-methoxy-4-ethoxyphenyl)-6-ethyl-3,6-dihydro-1,3,4 thiadiazin-2-on
3-(4-Isonicotinoylamino-benzyl)-5-(3-ethoxy-4-methoxyphenyl)-6-ethyl-3,6-dihydro-1,3,4-thiadiazin-2-on
3-(4-Isonicotinoylamino-benzyl)-5-(3-hydroxy-4-methoxyphenyl)-6-ethyl-3,6-dihydro-1,3,4-thiadiazin-2-on
3-(4-lsonicotinoylamino-benzyl)-5-(4-methylsulfonylphenyl)-6-ethyl-3,6-dihydro-1,3,4-thiadiazin-2-on
3-(4-Isonicotinoylamino-benzyl)-5-(4-methylenoxyphenyl)-6-ethyl-3,6-dihydro-1,3,4-thiadiazin-2-on
3-(4-Isonicotinoylamino-benzyl)-5-(3-cyclopentyloxy-4-methoxyphenyl)-6-ethyl-3,6-dihydro-1,3,4-thiadiazin-2-on
3-(3-Isonicotinoylamino-benzyl)-5-(3-cyclopentyloxy-4-methoxyphenyl)-6-ethyl-3,6-dihydro-1,3,4-thiadiazin-2-on
3-(4-Isonicotinoylamino-phenethyl)-5-(3,4-dimethoxyphenyl)-3,6-dihydro-1,3,4-thiadiazin-2-on
3-(4-Isonicotinoylamino-phenethyl)-5-(3,4-dimethoxyphenyl)-6-ethyl-3,6-dihydro-1,3,4-thiadiazin-2-on
3-(4-lsonicotinoylamino-benzyl)-5-(3,4-dimethoxyphenyl)-3,6-dihydro-1,3,4-oxadiazin-2-on
3-(3-Isonicotinoylamino-benzyl)-5-(3,4-dimethoxyphenyl)-3,6-dihydro-1,3,4-oxadiazin-2-on
3-(2-lsonicotinoylamino-benzyl)-5-(3,4-dimethoxyphenyl)-3,6-dihydro-1,3,4-oxadiazin-2-on
3-(4-Isonicotinoylamino-benzyl)-5-(3,4-dimethoxyphenyl)-6-ethyl-3,6-dihydro-1,3,4-oxadiazin-2-on
3-(3-lsonicotinoylamino-benzyl)-5-(3,4-dimethoxyphenyl)-6-ethyl-3,6-dihydro-1,3,4-oxadiazin-2-on
3-(2-Isonicotinoylamino-benzyl)-5-(3,4-dimethoxyphenyl)-6-ethyl-3,8-dihydro-1,3,4-oxadiazin-2-on
3-(4-Isonicotinoylamino-benzyl)-5-(3-methoxy-4-trifluormethoxyphenyl)-6-ethyl-3,6-dihydro-1,3,4-oxadiazin-2-on
3-(4-Isonicotinoylamino-benzyl)-5-(3-methoxy-4-difluormethoxyphenyl)-6-ethyl-3,6-dihydro-1,3,4-oxadiazin-2-on
3-(4-lsonicotinoylamino-benzyl)-5-(3-methoxy-4-fluormethoxyphenyl)-6-ethyl-3,6-dihydro-1,3,4-oxadiazin-2-on
3-(4-lsonicotinoylamino-benzyl)-5-(3-difluormethoxy-4-methoxyphenyl)-6-ethyl-3,6-dihydro-1,3,4-oxadiazin-2-on
3-(4-lsonicotinoylamino-benzyl)-5-(3-trifluormethoxy-4-methoxyphenyl)-6-ethyl-3,6-dihydro-1,3,4-oxadiazin-2-on
3-(4-lsonicotinoylamino-benzyl)-5-(3-fluormethoxy-4-methoxyphenyl)-6-ethyl-3,6-dihydro-1,3,4-oxadiazin-2-on
3-(4-Isonicotinoylamino-benzyl)-5-(3-methoxy-4-ethoxyphenyl)-6-ethyl-3,6-dihydro-1,3,4-oxadiazin-2-on
3-(4-Isonicotinoylamino-benzyl)-5-(3-ethoxy-4-methoxyphenyl)-6-ethyl-3,6-dihydro-1,3,4-oxadiazin-2-on
3-(4-lsonicotinoylamino-benzyl)-5-(3-hydroxy-4-methoxyphenyl)-6-ethyl-3,6-dihydro-1,3,4-oxadiazin-2-on
3-(4-Isonicotinoylamino-benzyl)-5-(4-methylsulfonylphenyl)-6-ethyl-3,6-dihydro-1,3,4-oxadiazin-2-on
3-(4-Isonicotinoylamino-benzyl)-5-(4-methylenoxyphenyl)-6-ethyl-3,6-dihydro-1,3,4-oxadiazin-2-on
3-(4-Isonicotinoylamino-benzyl)-5-(3-cyclopentyloxy-4-methoxyphenyl)-6-ethyl-3,6-dihydro-1,3,4-oxadiazin-2-on
3-(3-lsonicotinoylamino-benzyl)-5-(3-cyclopentyloxy-4-methoxyphenyl)-6-ethyl-3,6-dihydro-1,3,4-oxadiazin-2-on
3-(4-Isonicotinoylamino-phenethyl)-5-(3,4-dimethoxyphenyl)-3,6-dihydro-1,3,4-oxadiazin-2-on
3-(4-lsonicotinoylamino-phenethyl)-5-(3,4-dimethoxyphenyl)-6-ethyl-3,6-dihydro-1,3,4-oxadiazin-2-on.

Analog erhält man durch Umsetzung der oben aufgeführten "Aminderivate" mit Picolinsäurechlorid die nachstehenden Verbindungen
2-(4-Picolinoylamino-benzyl)-6-(3,4-dimethoxyphenyl)-2,3,4,5-tetrahydro-pyridazin-3-on
2-(3-Picolinoylamino-benzyl)-6-(3,4-dimethoxyphenyl)-2,3,4,5-tetrahydro-pyridazin-3-on
2-(2-Picolinoylamino-benzyl)-6-(3,4-dimethoxyphenyl)-2,3,4,5-tetrahydro-pyridazin-3-on
2-(4-Picolinoylamino-benzyl)-6-(3,4-dimethoxyphenyl)-5-ethyl-2,3,4,5-tetrahydro-pyridazin-3-on;
2-(3-Picolinoylamino-benzyl)-6-(3,4-dimethoxyphenyl)-5-ethyl-2,3,4,5-tetrahydro-pyridazin-3-on
2-(2-Picolinoylamino-benzyl)-6-(3,4-dimethoxyphenyl)-5-ethyl-2,3,4,5-tetrahydro-pyridazin-3-on
2-(4-Picolinoylamino-benzyl)-6-(3-methoxy-4-trifluormethoxyphenyl)-5-ethyl-2,3,4,5-tetrahydro-pyridazin-3-on
2-(4-Picolinoylamino-benzyl)-6-(3-methoxy-4-difluormethoxyphenyl)-5-ethyl-2,3,4,5-tetrahydro-pyridazin-3-on
2-(4-Picolinoylamino-benzyl)-6-(3-methoxy-4-fluormethoxyphenyl)-5-ethyl-2,3,4,5-tetrahydro-pyridazin-3-on
2-(4-Picolinoylamino-benzyl)-6-(3-difluormethoxy-4-methoxyphenyl)-5-ethyl-2,3,4,5-tetrahydro-pyridazin-3-on
2-(4-Picolinoylamino-benzyl)-6-(3-trifluormethoxy-4-methoxyphenyl)-5-ethyl-2,3,4,5-tetrahydro-pyridazin-3-on
2-(4-Picolinoylamino-benzyl)-6-(3-fluormethoxy-4-methoxyphenyl)-5-ethyl-2,3,4,5-tetrahydro-pyridazin-3-on
2-(4-Picolinoylamino-benzyl)-6-(3-methoxy-4-ethoxyphenyl)-5-ethyl-2,3,4,5-tetrahydro-pyridazin-3-on
2-(4-Picolinoylamino-benzyl)-6-(3-ethoxy-4-methoxyphenyl)-5-ethyl-2,3,4,5-tetrahydro-pyridazin-3-on
2-(4-Picolinoylamino-benzyl)-6-(3-hydroxy-4-methoxyphenyl)-5-ethyl-2,3,4,5-tetrahydro-pyridazin-3-on
2-(4-Picolinoylamino-benzyl)-6-(4-methylsulfonylphenyl)-5-ethyl-2,3,4,5-tetrahydro-pyridazin-3-on
2-(4-Picolinoylamino-benzyl)-6-(4-methylenoxyphenyl)-5-ethyl-2,3,4,5-tetrahydro-pyridazin-3-on
2-(4-Picolinoylamino-benzyl)-6-(3-cyclopentyloxy-4-methoxyphenyl)-5-ethyl-2,3,4,5-tetrahydro-pyridazin-3-on
2-(3-Picolinoylamino-benzyl)-6-(3-cyclopentyloxy-4-methoxyphenyl)-5-ethyl-2,3,4,5-tetrahydro-pyridazin-3-on
2-(4-Picolinoylamino-phenethyl)-6-(3,4-dimethoxyphenyl)-2,3,4,5-tetrahydro-pyridazin-3-on
2-(4-Picolinoylamino-phenethyl)-6-(3,4-dimethoxyphenyl)-5-ethyl-2,3,4,5-tetrahydro-pyridazin-3-on
3-(4-Picolinoylamino-benzyl)-5-(3,4-dimethoxyphenyl)-3,6-dihydro-1,3,4-thiadiazin-2-on
3-(3-Picolinoylamino-benzyl)-5-(3,4-dimethoxyphenyl)-3,6-dihydro-1,3,4-thiadiazin-2-on
3-(2-Picolinoylamino-benzyl)-5-(3,4-dimethoxyphenyl)-3,6-dihydro-1,3,4-thiadiazin-2-on
3-(4-Picolinoylamino-benzyl)-5-(3,4-dimethoxyphenyl)-6-ethyl-3,6-dihydro-1,3,4-thiadiazin-2-on
3-(3-Picolinoylamino-benzyl)-5-(3,4-dimethoxyphenyl)-6-ethyl-3,6-dihydro-1,3,4-thiadiazin-2-on
3-(2-Picolinoylamino-benzyl)-5-(3,4-dimethoxyphenyl)-6-ethyl-3,6-dihydro-1,3,4-thiadiazin-2-on
3-(4-Picolinoylamino-benzyl)-5-(3-methoxy-4-trifluormethoxyphenyl)-6-ethyl-3,6-dihydro-1,3,4-thiadiazin-2-on
3-(4-Picolinoylamino-benzyl)-5-(3-methoxy-4-difluormethoxyphenyl)-6-ethyl-3,6-dihydro-1,3,4-thiadiazin-2-on
3-(4-Picolinoylamino-benzyl)-5-(3-methoxy-4-fluormethoxyphenyl)-6-ethyl-3,6-dihydro-1,3,4-thiadiazin-2-on
3-(4-Picolinoylamino-benzyl)-5-(3-difluormethoxy-4-methoxyphenyl)-6-ethyl-3,6-dihydro-1,3,4-thiadiazin-2-on
3-(4-Picolinoylamino-benzyl)-5-(3-trifluormethoxy-4-methoxyphenyl)-6-ethyl-3,6-dihydro-1,3,4-thiadiazin-2-on
3-(4-Picolinoylamino-benzyl)-5-(3-fluormethoxy-4-methoxyphenyl)-6-ethyl-3,6-dihydro-1,3,4-thiadiazin-2-on
3-(4-Picolinoylamino-benzyl)-5-(3-methoxy-4-ethoxyphenyl)-6-ethyl-3,6-dihydro-1,3,4-thiadiazin-2-on
3-(4-Picolinoylamino-benzyl)-5-(3-ethoxy-4-methoxyphenyl)-6-ethyl-3,6-dihydro-1,3,4-thiadiazin-2-on
3-(4-Picolinoylamino-benzyl)-5-(3-hydroxy-4-methoxyphenyl)-6-ethyl-3,6-dihydro-1,3,4-thiadiazin-2-on
3-(4-Picolinoylamino-benzyl)-5-(4-methylsulfonylphenyl)-6-ethyl-3,6-dihydro-1,3,4-thiadiazin-2-on
3-(4-Picolinoylamino-benzyl)-5-(4-methylenoxyphenyl)-6-ethyl-3,6-dihydro-1,3,4-thiadiazin-2-on
3-(4-Picolinoylamino-benzyl)-5-(3-cyclopentyloxy-4-methoxyphenyl)-6-ethyl-3,6-dihydro-1,3,4-thiadiazin-2-on
3-(3-Picolinoylamino-benzyl)-5-(3-cyclopentyloxy-4-methoxyphenyl)-6-ethyl-3,6-dihydro-1,3,4-thiadiazin-2-on
3-(4-Picolinoylamino-phenethyl)-5-(3,4-dimethoxyphenyl)-3,6-dihydro-1,3,4-thiadiazin-2-on
3-(4-Picolinoylamino-phenethyl)-5-(3,4-dimethoxyphenyl)-6-ethyl-3,6-dihydro-1,3,4-thiadiazin-2-on
3-(4-Picolinoylamino-benzyl)-5-(3,4-dimethoxyphenyl)-3,6-dihydro-1 ,3,4-oxadiazin-2-on
3-(3-Picolinoylamino-benzyl)-5-(3,4-dimethoxyphenyl)-3,6-dihydro-1,3,4-oxadiazin-2-on
3-(2-Picolinoylamino-benzyl)-5-(3,4-dimethoxyphenyl)-3,6-dihydro-1,3,4-oxadiazin-2-on
3-(4-Picolinoylamino-benzyl)-5-(3,4-dimethoxyphenyl)-6-ethyl-3,6-dihydro-1,3,4-oxadiazin-2-on
3-(3-Picolinoylamino-benzyl)-5-(3,4-dimethoxyphenyl)-6-ethyl-3,6-dihydro-1,3,4-oxadiazin-2-on
3-(2-Picolinoylamino-benzyl)-5-(3,4-dimethoxyphenyl)-6-ethyl-3,6-dihydro-1,3,4-oxadiazin-2-on
3-(4-Picolinoylamino-benzyl)-5-(3-methoxy-4-trifluormethoxyphenyl)-6-ethyl-3,6-dihydro-1,3,4-oxadiazin-2-on
3-(4-Picolinoylamino-benzyl)-5-(3-methoxy-4-difluormethoxyphenyl)-6-ethyl-3,6-dihydro-1,3,4-oxadiazin-2-on
3-(4-Picolinoylamino-benzyl)-5-(3-methoxy-4-fluormethoxyphenyl)-6-ethyl-3,6-dihydro-1,3,4-oxadiazin-2-on
3-(4-Picolinoylamino-benzyl)-5-(3-difluormethoxy-4-methoxyphenyl)-6-ethyl-3,6-dihydro-1,3,4-oxadiazin-2-on
3-(4-Picolinoylamino-benzyl)-5-(3-trifluormethoxy-4-methoxyphenyl)-6-ethyl-3,6-dihydro-1,3,4-oxadiazin-2-on
3-(4-Picolinoylamino-benzyl)-5-(3-fluormethoxy-4-methoxyphenyl)-6-ethyl-3,6-dihydro-1,3,4-oxadiazin-2-on
3-(4-Picolinoylamino-benzyl)-5-(3-methoxy-4-ethoxyphenyl)-ethyl-3,6-dihydro-1,3,4-oxadiazin-2-on
3-(4-Picolinoylamino-benzyl)-5-(3-ethoxy-4-methoxyphenyl)-6-ethyl-3,6-dihydro-1,3,4-oxadiazin-2-on
3-(4-Picolinoylamino-benzyl)-5-(3-hydroxy-4-methoxyphenyl)-6-ethyl-3,6-dihydro-1,3,4-oxadiazin-2-on
3-(4-Picolinoylamino-benzyl)-5-(4-methylsulfonylphenyl)-6-ethyl-3,6-dihydro-1,3,4-oxadiazin-2-on
3-(4-Picolinoylamino-benzyl)-5-(4-methylenoxyphenyl)-6-ethyl-3,6-dihydro-1,3,4-oxadiazin-2-on
3-(4-Picolinoylamino-benzyl)-5-(3-cyclopentyloxy-4-methoxyphenyl)-6-ethyl-3,6-dihydro-1,3,4-oxadiazin-2-on
3-(3-Picolinoylamino-benzyl)-5-(3-cyclopentyloxy-4-methoxyphenyl)-6-ethyl-3,6-dihydro-1,3,4-oxadiazin-2-on
3-(4-Picolinoylamino-phenethyl)-5-(3,4-dimethoxyphenyl)-3,6-dihydro-1,3,4-oxadtazin-2-on
3-(4-Picolinoylamino-phenethyl)-5-(3,4-dimethoxyphenyl)-6-ethyl-3,6-dihydro-1,3,4-oxadiazin-2-on.

Analog erhält man durch Umsetzung der oben aufgeführten "Aminderivate" mit Furan-2-carbonsäurechlorid die nachstehenden Verbindungen
2-(4-(Furan-2-carbonylamino)-benzyl)-6-(3,4-dimethoxyphenyl)-2,3,4,5-tetrahydro-pyridazin-3-on
2-(3-(Furan-2-carbonylamino)-benzyl)-6-(3,4-dimethoxyphenyl)-2,3,4,5-tetrahydro-pyridazin-3-on
2-(2-(Furan-2-carbonylamino)-benzyl)-6-(3,4-dimethoxyphenyl)-2,3,4,5-tetrahydro-pyridazin-3-on
2-(4-(Furan-2-carbonylamino)-benzyl)-6-(3,4-dimethoxyphenyl)-5-ethyl-2,3,4,5-tetrahydro-pyridazin-3-on;
2-(3-(Furan-2-carbonylamino)-benzyl)-6-(3,4-dimethoxyphenyl)-5-ethyl-2,3,4,5-tetrahydro-pyridazin-3-on
2-(2-(Furan-2-carbonylamino)-benzyl)-6-(3,4-dimethoxyphenyl)-5-ethyl-2,3,4,5-tetrahydro-pyridazin-3-on
2-(4-(Furan-2-carbonylamino)-benzyl)-6-(3-methoxy-4-trifluor-methoxy-phenyl)-5-ethyl-2,3,4,5-tetrahydro-pyridazin-3-on
2-(4-(Furan-2-carbonylamino-benzyl)-6-(3-methoxy-4-difluor-methoxy-phenyl)-5-ethyl-2,3,4,5-tetrahydro-pyridazin-3-on
2-(4-(Furan-2-carbonylamino)-benzyl)-6-(3-methoxy-4-fluor-methoxyphenyl)-5-ethyl-2,3,4,5-tetrahydro-pyridazin-3-on
2-(4-(Furan-2-carbonylamino)-benzyl)-6-(3-difluormethoxy-4-methoxy-phenyl)-5-ethyl-2,3,4,5-tetrahydro-pyridazin-3-on
2-(4-(Furan-2-carbonylamino)-benzyI)-6-(3-trifluormethoxy-4-methoxy-phenyl)-5-ethyl-2,3,4,5-tetrahydro-pyridazin-3-on
2-(4-(Furan-2-carbonylamino)-benzyl)-6-(3-Fluormethoxy-4-methoxy-phenyl)-5-ethyl-2,3,4,5-tetrahydro-pyridazin-3-on
2-(4-(Furan-2-carbonylamino)-benzyl)-6-(3-methoxy-4-ethoxyphenyl)-5-ethyl-2,3,4,5-tetrahydro-pyridazin-3-on
2-(4-(Furan-2-carbonylamino)-benzyl)-6-(3-ethoxy-4-methoxyphenyl)-5-ethyl-2,3,4,5-tetrahydro-pyridazin-3-on
2-(4-(Furan-2-carbonylamino)-benzyl)-6-(3-hydroxy-4-methoxyphenyl)-5-ethyl-2,3,4,5-tetrahydro-pyridazin-3-on
2-(4-(Furan-2-carbonylamino)-benzyl)-6-(4-methylsulfonylphenyl)-5-ethyl-2,3,4,5-tetrahydro-pyridazin-3-on
2-(4-(Furan-2-carbonylamino)-benzyl)-6-(4-methylenoxyphenyl)-5-ethyl-2,3,4,5-tetrahydro-pyridazin-3-on
2-(4-(Furan-2-carbonylamino)-benzyl)-6-(3-cyclopentyloxy-4-methoxy-phenyl)-5-ethyl-2,3,4,5-tetrahydro-pyridazin-3-on
2-(3-(Furan-2-carbonylamino)-benzyl)-6-(3-cyclopentyloxy-4-methoxy-phenyl)-5-ethyl-2,3,4,5-tetrahydro-pyridazin-3-on
2-(4-(Furan-2-carbonylamino)-phenethyl)-6-(3,4-dimethoxyphenyl)-2,3,4,5-tetrahydro-pyridazin-3-on
2-(4-(Furan-2-carbonylamino)-phenethyl)-6-(3,4-dimethoxyphenyl)-5-ethyl-2,3,4,5-tetrahydro-pyridazin-3-on
3-(4-(Furan-2-carbonylamino)-benzyl)-5-(3,4-dimethoxyphenyl)-3,6-dihydro-1,3,4-thiadiazin-2-on
3-(3-(Furan-2-carbonylamino)-benzyl)-5-(3,4-dimethoxyphenyl)-3,6-dihydro-1,3,4-thiadiazin-2-on
3-(2-(Furan-2-carbonylamino)-benzyl)-5-(3,4-dimethoxyphenyl)-3,6-dihydro-1,3,4-thiadiazin-2-on
3-(4-(Furan-2-carbonylamino)-benzyl)-5-(3,4-dimethoxyphenyl)-6-ethyl-3,6-dihydro-1,3,4-thiadiazin-2-on
3-(3-(Furan-2-carbonylamino)-benzyl)-5-(3,4-dimethoxyphenyl)-6-ethyl-3,6-dihydro-1,3,4-thiadiazin-2-on
3-(2-(Furan-2-carbonylamino)-benzyl)-5-(3,4-dimethoxyphenyl)-6-ethyl-3,6-dihydro-1,3,4-thiadiazin-2-on
3-(4-(Furan-2-carbonylamino)-benzyl)-5-(3-methoxy-4-trifluor-methoxy-phenyl)-6-ethyl-3,6-dihydro-1,3,4-thiadiazin-2-on
3-(4-(Furan-2-carbonylamino)-benzyl)-5-(3-methoxy-4-difluor-methoxy-phenyl)-6-ethyl-3,6-dihydro-1,3,4-thiadiazin-2-on
3-(4-(Furan-2-carbonylamino)-benzyl)-5-(3-methoxy-4-fluor-methoxy-phenyl)-6-ethyl-3,6-dihydro-1,3,4-thiadiazin-2-on
3-(4-(Furan-2-carbonylamino)-benzyl)-5-(3-difluormethoxy-4-methoxy-phenyl)-6-ethyl-3,6-dihydro-1,3,4-thiadiazin-2-on
3-(4-Furan-2-carbonylamino)-benzyl)-5-(3-trifluormethoxy-4-methoxy-phenyl)-6-ethyl-3,6-dihydro-1,3,4-thiadiazin-2-on
3-(4-(Furan-2-carbonylamino)-benzyl)-5-(3-fluormethoxy-4-methoxy-phenyl)-6-ethyl-3,6-dihydro-1,3,4-thiadiazin-2-on
3-(4-(Furan-2-carbonylamino)-benzyl)-5-(3-methoxy-4-ethoxyphenyl)-6-ethyl-3,6-dihydro-1,3,4-thiadiazin-2-on
3-(4-(Furan-2-carbonylamino)-benzyl)-5-(3-ethoxy-4-methoxyphenyl)-6-ethyl-3,6-dihydro-1,3,4-thiadiazin-2-on
3-(4-(Furan-2-carbonylamino)-benzyl)-5-(3-hydroxy-4-methoxyphenyl)-6-ethyl-3,6-dihydro-1,3,4-thiadiazin-2-on
3-(4-(Furan-2-carbonylamino)-benzyl)-5-(4-methylsulfonylphenyl)-6-ethyl-3,6-dihydro-1,3,4-thiadiazin-2-on
3-(4-(Furan-2-carbonylamino)-benzyl)-5-(4-methylenoxyphenyl)-6-ethyl-3,6-dihydro-1,3,4-thiadiazin-2-on
3-(4-(Furan-2-carbonylamino)-benzyl)-5-(3-cyclopentyloxy-4-methoxy-phenyl)-6-ethyl-3,6-dihydro-1,3,4-thiadiazin-2-on
3-(3-(Furan-2-carbonylamino)-benzyl)-5-(3-cyclopentyloxy-4-methoxy-phenyl)-6-ethyl-3,6-dihydro-1,3,4-thiadiazin-2-on
3-(4-(Furan-2-carbonylamino)-phenethyl)-5-(3,4-dimethoxyphenyl)-3,6-dihydro-1,3,4-thiadiazin-2-on
3-(4-(Furan-2-carbonylamino)-phenethyl)-5-(3,4-dimethoxyphenyl)-6-ethyl-3,6-dihydro-1,3,4-thiadiazin-2-on
3-(4-(Furan-2-carbonylamino)-benzyl)-5-(3,4-dimethoxyphenyl)-3,6-dihydro-1,3,4-oxadiazin-2-on
3-(3-(Furan-2-carbonylamino)-benzyl)-5-(3,4-dimethoxyphenyl)-3,6-dihydro-1,3,4-oxadiazin-2-on
3-(2-(Furan-2-carbonylamino)-benzyl)-5-(3,4-dimethoxyphenyl)-3,6-dihydro-1,3,4-oxadiazin-2-on
3-(4-(Furan-2-carbonylamino)-benzyl)-5-(3,4-dimethoxyphenyl)-6-ethyl-3,6-dihydro-1,3,4-oxadiazin-2-on
3-(3-(Furan-2-carbonylamino)-benzyl)-5-(3,4-dimethoxyphenyl)-6-ethyl-3,6-dihydro-1,3,4-oxadiazin-2-on
3-(2-(Furan-2-carbonylamino)-benzyl)-5-(3,4-dimethoxyphenyl)-6-ethyl-3,6-dihydro-1,3,4-oxadiazin-2-on
3-(4-(Furan-2-carbonylamino)-benzyl)-5-(3-methoxy-4-trifluor-methoxy-phenyl)-6-ethyl-3,6-dihydro-1,3,4-oxadiazin-2-on
3-(4-(Furan-2-carbonylamino)-benzyl)-5-(3-methoxy-4-difluor-methoxy-phenyl)-6-ethyl-3,6-dihydro-1,3,4-oxadiazin-2-on
3-(4-(Furan-2-carbonylamino)-benzyl)-5-(3-methoxy-4-fluor-methoxy-phenyl)-6-ethyl-3,6-dihydro-1,3,4-oxadiazin-2-on
3-(4-(Furan-2-carbonylamino)-benzyl)-5-(3-difluormethoxy-4-methoxy-phenyl)-6-ethyl-3,6-dihydro-1,3,4-oxadiazin-2-on
3-(4-(Furan-2-carbonylamino)-benzyl)-5-(3-trifluormethoxy-4-methoxy-phenyl)-6-ethyl-3,6-dihydro-1,3,4-oxadiazin-2-on
3-(4-(Furan-2-carbonylamino)-benzyl)-5-(3-fluormethoxy-4-methoxy-phenyl)-6-ethyl-3,6-dihydro-1,3,4-oxadiazin-2-on
3-(4-(Furan-2-carbonylamino)-benzyl)-5-(3-methoxy-4-ethoxyphenyl)-6-ethyl-3,6-dihydro-1,3,4-oxadiazin-2-on
3-(4-(Furan-2-carbonylamino)-benzyl)-5-(3-ethoxy-4-methoxyphenyl)-6-ethyl-3,6-dihydro-1,3,4-oxadiazin-2-on
3-(4-(Furan-2-carbonylamino)-benzyl)-5-(3-hydroxy-4-methoxyphenyl)-6-ethyl-3,6-dihydro-1,3,4-oxadiazin-2-on
3-(4-(Furan-2-carbonylamino)-benzyl)-5-(4-methylsulfonylphenyl)-6-ethyl-3,6-dihydro-1,3,4-oxadiazin-2-on
3-(4-(Furan-2-carbonylamino)-benzyl)-5-(4-methylenoxyphenyl)-6-ethyl-3,6-dihydro-1,3,4-oxadiazin-2-on
3-(4-(Furan-2-carbonylamino)-benzyl)-5-(3-cyclopentyloxy-4-methoxyphenyl)-6-ethyl-3,6-dihydro-1,3,4-oxadiazin-2-on
3-(3-(Furan-2-carbonylamino)-benzyl)-5-(3-cyclopentyloxy-4-methoxyphenyl)-6-ethyl-3,6-dihydro-1,3,4-oxadiazin-2-on
3-(4-(Furan-2-carbonylamino)-phenethyl)-5-(3,4-dimethoxyphenyl)-3,6-dihydro-1,3,4-oxadiazin-2-on
3-(4-(Furan-2-carbonylamino)-phenethyl)-5-(3,4-dimethoxyphenyl)-6-ethyl-3,6-dihydro-1,3,4-oxadiazin-2-on.

Analog erhält man durch Umsetzung der oben aufgeführten "Aminderivate" mit Pyrrol-2-carbonsäurechlorid die nachstehenden Verbindungen
2-(4-(Pyrrol-2-carbonylamino)-benzyl)-6-(3,4-dimethoxyphenyl)-2,3,4,5-tetrahydro-pyridazin-3-on
2-(3-(Pyrrol-2-carbonylamino)-benzyl)-6-(3,4-dimethoxyphenyl)-2,3,4,5-tetrahydro-pyridazin-3-on
2-(2-(Pyrrol-2-carbonylamino)-benzyl)-6-(3,4-dimethoxyphenyl)-2,3,4,5-tetrahydro-pyridazin-3-on
2-(4-(Pyrrol-2-carbonylamino)-benzyl)-6-(3,4-dimethoxyphenyl)-5-ethyl-2,3,4,5-tetrahydro-pyridazin-3-on;
2-(3-(Pyrrol-2-carbonylamino)-benzyl)-6-(3,4-dimethoxyphenyl)-5-ethyl-2,3,4,5-tetrahydro-pyridazin-3-on
2-(2-(Pyrrol-2-carbonylamino)-benzyl)-6-(3,4-dimethoxyphenyl)-5-ethyl-2,3,4,5-tetrahydro-pyridazin-3-on
2-(4-(Pyrrol-2-carbonylamino)-benzyl)-6-(3-methoxy-4-trifluor-methoxyphenyl)-5-ethyl-2,3,4,5-tetrahydro-pyridazin-3-on
2-(4-(Pyrrol-2-carbonylamino)-benzyl)-6-(3-methoxy-4-difluor-methoxyphenyl)-5-ethyl-2,3,4,5-tetrahydro-pyridazin-3-on
2-(4-(Pyrrol-2-carbonylamino)-benzyl)-6-(3-methoxy-4-fluor-methoxy-phenyl)-5-ethyl-2,3,4,5-tetrahydro-pyridazin-3-on
2-(4-(Pyrrol-2-carbonylamino)-benzyl)-6-(3-difluormethoxy-4-methoxy-phenyl)-5-ethyl-2,3,4,5-tetrahydro-pyridazin-3-on
2-(4-(Pyrrol-2-carbonylamino)-benzyl)-6-(3-trifluormethoxy-4-methoxy-phenyl)-5-ethyl-2,3,4,5-tetrahydro-pyridazin-3-on
2-(4-(Pyrrol-2-carbonylamino)-benzyl)-6-(3-Fluormethoxy-4-methoxy-phenyl)-5-ethyl-2,3,4,5-tetrahydro-pyridazin-3-on
2-(4-(Pyrrol-2-carbonylamino)-benzyl)-6-(3-methoxy-4-ethoxyphenyl)-5-ethyl-2,3,4,5-tetrahydro-pyridazin-3-on
2-(4-(Pyrrol-2-carbonylamino)-benzyl)-6-(3-ethoxy-4-methoxyphenyl)-5-ethyl-2,3,4,5-tetrahydro-pyridazin-3-on
2-(4-(Pyrrol-2-carbonylamino)-benzyl)-6-(3-hydroxy-4-methoxyphenyl)-5-ethyl-2,3,4,5-tetrahydro-pyridazin-3-on
2-(4-(Pyrrol-2-carbonylamino)-benzyl)-6-(4-methylsulfonylphenyl)-5-ethyl-2,3,4,5-tetrahydro-pyridazin-3-on
2-(4-(Pyrrol-2-carbonylamino)-benzyl)-6-(4-methylenoxyphenyl)-5-ethyl-2,3,4,5-tetrahydro-pyridazin-3-on
2-(4-(Pyrrol-2-carbonylamino)-benzyl)-6-(3-cyclopentyloxy-4-methoxy-phenyl)-5-ethyl-2,3,4,5-tetrahydro-pyridazin-3-on
2-(3-(Pyrrol-2-carbonylamino)-benzyl)-6-(3-cyclopentyloxy-4-methoxy-phenyl)-5-ethyl-2,3,4,5-tetrahydro-pyridazin-3-on
2-(4-(Pyrrol-2-carbonylamino)-phenethyl)4-(3,4-dimethoxyphenyl)-2,3,4,5-tetrahydro-pyridazin-3-on
2-(4-(Pyrrol-2-carbonylamino)-phenethyl)-6-(3,4-dimethoxyphenyl)-5-ethyl-2,3,4,5-tetrahydro-pyridazin-3-on
3-(4-(Pyrrol-2-carbonylamino)-benzyl)-5-(3,4-dimethoxyphenyl)-3,6-dihydro-1,3,4-thiadiazin-2-on
3-(3-(Pyrrol-2-carbonylamino)-benzyl)-5-(3,4-dimethoxyphenyl)-3,6-dihydro-1,3,4-thiadiazin-2-on
3-(2-(Pyrrol-2-carbonylamino)-benzyl)-5-(3,4-dimethoxyphenyl)-3,6-dihydro-1,3,4-thiadiazin-2-on
3-(4-(Pyrrol-2-carbonylamino)-benzyl)-5-(3,4-dimethoxyphenyl)-6-ethyl-3,6-dihydro-1,3,4-thiadiazin-2-on
3-(3-(Pyrrol-2-carbonylamino)-benzyl)-5-(3,4-dimethoxyphenyl)-6-ethyl-3,6-dihydro-1,3,4-thiadiazin-2-on
3-(2-(Pyrrol-2-carbonylamino)-benzyl)-5-(3,4-dimethoxyphenyl)-6-ethyl-3,6-dihydro-1,3,4-thiadiazin-2-on
3-(4-(Pyrrol-2-carbonylamino)-benzyl)-5-(3-methoxy-4-trifluor-methoxy-phenyl)-6-ethyl-3,6-dihydro-1,3,4-thiadiazin-2-on
3-(4-(Pyrrol-2-carbonylamino)-benzyl)-5-(3-methoxy-4-difluor-methoxy-phenyl)-6-ethyl-3,6-dihydro-1,3,4-thiadiazin-2-on
3-(4-(Pyrrol-2-carbonylamino)-benzyl)-5-(3-methoxy-4-fluor-methoxy-phenyl)-6-ethyl-3,6-dihydro-1,3,4-thiadiazin-2-on
3-(4-(Pyrrol-2-carbonylamino)-benzyl)-5-(3-difluormethoxy-4-methoxy-phenyl)-6-ethyl-3,6-dihydro-1,3,4-thiadiazin-2-on
3-(4-(Pyrrol-2-carbonylamino)-benzyl)-5-(3-trifluormethoxy-4-methoxy-phenyl)-6-ethyl-3,6-dihydro-1,3,4-thiadiazin-2-on
3-(4-(Pyrrol-2-carbonylamino)-benzyl)-5-(3-fluormethoxy-4-methoxy-phenyl)-6-ethyl-3,6-dihydro-1,3,4-thiadiazin-2-on
3-(4-(Pyrrol-2-carbonylamino)-benzyl)-5-(3-Methoxy-4-ethoxyphenyl)-6-ethyl-3,6-dihydro-1,3,4-thiadiazin-2-on
3-(4-(Pyrrol-2-carbonylamino)-benzyl)-5-(3-ethoxy-4-methoxyphenyl)-6-ethyl-3,6-dihydro-1,3,4-thiadiazin-2-on
3-(4-(Pyrrol-2-carbonylamino)-benzyl)-5-(3-hydroxy-4-methoxyphenyl)-6-ethyl-3,6-dihydro-1,3,4-thiadiazin-2-on
3-(4-(Pyrrol-2-carbonylamino)-benzyl)-5-(4-methylsulfonylphenyl)-6-ethyl-3,6-dihydro-1,3,4-thiadiazin-2-on
3-(4-(Pyrrol-2-carbonylamino)-benzyl)-5-(4-methylenoxyphenyl)-6-ethyl-3,6-dihydro-1,3,4-thiadiazin-2-on
3-(4-(Pyrrol-2-carbonylamino)-benzyl)-5-(3-cyclopentyloxy-4-methoxy-phenyl)-6-ethyl-3,6-dihydro-1,3,4-thiadiazin-2-on
3-(3-(Pyrrol-2-carbonylamino)-benzyl)-5-(3-cyclopentyloxy-4-methoxy-phenyl)-6-ethyl-3,6-dihydro-1,3,4-thiadiazin-2-on
3-(4-(Pyrrol-2-carbonylamino)-phenethyl)-5-(3,4-dimethoxyphenyl)-3,6-dihydro-1,3,4-thiadiazin-2-on
3-(4-(Pyrrol-2-carbonylamino)-phenethyl)-5-(3,4-dimethoxyphenyl)-6-ethyl-3,6-dihydro-1,3,4-thiadiazin-2-on
3-(4-(Pyrrol-2-carbonylamino)-benzyl)-5-(3,4-dimethoxyphenyl)-3,6-dihydro-1,3,4-oxadiazin-2-on
3-(3-(Pyrrol-2-carbonylamino)-benzyl)-5-(3,4-dimethoxyphenyl)-3,6-dihydro-1,3,4-oxadiazin-2-on
3-(2-(Pyrrol-2-carbonylamino)-benzyl)-5-(3,4-dimethoxyphenyl)-3,6-dihydro-1,3,4-oxadiazin-2-on
3-(4-(Pyrrol-2-carbonylamino)-benzyl)-5-(3,4-dimethoxyphenyl)-6-ethyl-3,6-dihydro-1,3,4-oxadiazin-2-on
3-(3-(Pyrrol-2-carbonylamino)-benzyl)-5-(3,4-dimethoxyphenyl)-6-ethyl-3,6-dihydro-1,3,4-oxadiazin-2-on
3-(2-(Pyrrol-2-carbonylamino)-benzyl)-5-(3,4-dimethoxyphenyl)-6-ethyl-3,6-dihydro-1,3,4-oxadiazin-2-on
3-(4-(Pyrrol-2-carbonylamino)-benzyl)-5-(3-methoxy-4-trifluor-methoxy-phenyl)-6-ethyl-3,6-dihydro-1,3,4-oxadiazin-2-on
3-(4-(Pyrrol-2-carbonylamino)-benzyl)-5-(3-methoxy-4-difluor-methoxy-phenyl)-6-ethyl-3,6-dihydro-1,3,4-oxadiazin-2-on
3-(4-(Pyrrol-2-carbonylamino)-benzyl)-5-(3-methoxy-4-fluor-methoxy-phenyl)-6-ethyl-3,6-dihydro-1,3,4-oxadiazin-2-on
3-(4-(Pyrrol-2-carbonylamino)-benzyl)-5-(3-difluormethoxy-4-methoxy-phenyl)-6-ethyl-3,6-dihydro-1,3,4-oxadiazin-2-on
3-(4-(Pyrrol-2-carbonylamino)-benzyl)-5-(3-trifluomethoxy-4-methoxy-phenyl)-6-ethyl-3,6-dihydro-1,3,4-oxadiazin-2-on
3-(4-(Pyrrol-2-carbonylamino)-benzyl)-5-(3-fluormethoxy-4-methoxy-phenyl)-6-ethyl-3,6-dihydro-1,3,4-oxadiazin-2-on
3-(4-(Pyrrol-2-carbonylamino)-benzyl)-5-(3-methoxy-4-ethoxyphenyl)-6-ethyl-3,6-dihydro-1,3,4-oxadiazin-2-on
3-(4-(Pyrrol-2-carbonylamino)-benzyl)-5-(3-ethoxy-4-methoxyphenyl)-6-ethyl-3,6-dihydro-1,3,4-oxadiazin-2-on
3-(4-(Pyrrol-2-carbonylamino)-benzyl)-5-(3-hydroxy-4-methoxyphenyl)-6-ethyl-3,6-dihydro-1,3,4-oxadiazin-2-on
3-(4-(Pyrrol-2-carbonylamino)-benzyl)-5-(4-methylsulfonylphenyl)-6-ethyl-3,6-dihydro-1,3,4-oxadiazin-2-on
3-(4-(Pyrrol-2-carbonylamino)-benzyl)-5-(4-methylenoxyphenyl)-6-ethyl-3,6-dihydro-1,3,4-oxadiazin-2-on
3-(4-(Pyrrol-2-carbonylamino)-benzyl)-5-(3-cyclopentyloxy-4-methoxy-phenyl)-6-ethyl-3,6-dihydro-1,3,4-oxadiazin-2-on
3-(3-(Pyrrol-2-carbonylamino)-benzyl)-5-(3-cyclopentyloxy-4-methoxy-phenyl)-6-ethyl-3,6-dihydro-1,3,4-oxadiazin-2-on
3-(4-(Pyrrol-2-carbonylamino)-phenethyl)-5-(3,4-dimethoxyphenyl)-3,6-dihydro-1,3,4-oxadiazin-2-on
3-(4-(Pyrrol-2-carbonylamino)-phenethyl)-5-(3,4-dimethoxyphenyl)-6-ethyl-3,6-dihydro-1,3,4-oxadiazin-2-on.

Analog erhält man durch Umsetzung der oben aufgeführten "Aminderivate" mit Thiophen-2-carbonsäurechlorid die nachstehenden Verbindungen
2-(4-(Thiophen-2-carbonylamino)-benzyl)4-(3,4-dimethoxyphenyl)-2,3,4,5-tetrahydro-pyridazin-3-on
2-(3-(Thiophen-2-carbonylamino)-benzyl)-6-(3,4-dimethoxyphenyl)-2,3,4,5-tetrahydro-pyridazin-3-on
2-(2-(Thiophen-2-carbonylamino)-benzyl)-6-(3,4-dimethoxyphenyl)-2,3,4,5-tetrahydro-pyridazin-3-on
2-(4-(Thiophen-2-carbonylamino)-benzyl)-6-(3,4-dimethoxyphenyl)-5-ethyl-2,3,4,5-tetrahydro-pyridazin-3-on;
2-(3-(Thiophen-2-carbonylamino)-benzyl)4-(3,4-dimethoxyphenyl)-5-ethyl-2,3,4,5-tetrahydro-pyridazin-3-on
2-(2-(Thiophen-2-carbonylamino)-benzyl)-6-(3,4-dimethoxyphenyl)-5-ethyl-2,3,4,5-tetrahydro-pyridazin-3-on
2-(4-(Thiophen-2-carbonylamino)-benzyl)-6-(3-methoxy-4-trifluor-methoxyphenyl)-5-ethyl-2,3,4,5-tetrahydro-pyridazin-3-on
2-(4-(Thiophen-2-carbonylamino)-benzyl)4-(3-methoxy-4-difluor-methoxyphenyl)-5-ethyl-2,3,4,5-tetrahydro-pyridazin-3-on
2-(4-(Thiophen-2-carbonylamino)-benzyl)-6-(3-methoxy-4-fluor-methoxyphenyl)-5-ethyl-2,3,4,5-tetrahydro-pyridazin-3-on
2-(4-(Thiophen-2-carbonylamino)-benzyl)-6-(3-difluormethoxy-4-methoxyphenyl)-5-ethyl-2,3,4,5-tetrahydro-pyridazin-3-on
2-(4-(Thiophen-2-carbonylamino)-benzyl)-6-(3-trifluormethoxy-4-methoxyphenyl)-5-ethyl-2,3,4,5-tetrahydro-pyridazin-3-on
2-(4-(Thiophen-2-carbonylamino)-benzyl)-6-(3-fluormethoxy-4-methoxyphenyl)-5-ethyl-2,3,4,5-tetrahydro-pyridazin-3-on
2-(4-(Thiophen-2-carbonylamino)-benzyl)-6-(3-Methoxy-4-ethoxy-phenyl)-5-ethyl-2,3,4,5-tetrahydro-pyridazin-3-on
2-(4-(Thiophen-2-carbonylamino)-benzyl)-6-(3-ethoxy-4-methoxy-phenyl)-5-ethyl-2,3,4,5-tetrahydro-pyridazin-3-on
2-(4-(Thiophen-2-carbonylamino)-benzyl)-6-(3-hydroxy-4-methoxyphenyl)-5-ethyl-2,3,4,5-tetrahydro-pyridazin-3-on
2-(4-(Thiophen-2-carbonylamino)-benzyl)-6-(4-methylsulfonylphenyl)-5-ethyl-2,3,4,5-tetrahydro-pyridazin-3-on
2-(4-(Thiophen-2-carbonylamino)-benzyl)-6-(4-methylenoxypheny)-5-ethyl-2,3,4,5-tetrahydro-pyridazin-3-on
2-(4-(Thiophen-2-carbonylamino)-benzyl)-6-(3-cyclopentyloxy-4-methoxyphenyl)-5-ethyl-2,3,4,5-tetrahydro-pyridazin-3-on
2-(3-(Thiophen-2-carbonylamino)-benzyl)-6-(3-cyclopentyloxy-4-methoxyphenyl)-5-ethyl-2,3,4,5-tetrahydro-pyridazin-3-on
2-(4-(Thiophen-2-carbonylamino)-phenethyl)-6-(3,4-dimethoxyphenyl)-2,3,4,5-tetrahydro-pyridazin-3-on
2-(4-(Thiophen-2-carbonylamino)-phenethyl)-6-(3,4-dimethoxyphenyl)-5-ethyl-2,3,4,5-tetrahydro-pyridazin-3-on
3-(4-(Thiophen-2-carbonylamino)-benzyl)-5-(3,4-dimethoxyphenyl)-3,6-dihydro-1,3,4-thiadiazin-2-on, F. 186°
3-(3-(Thiophen-2-carbonylamino)-benzyl)-5-(3,4-dimethoxyphenyl)-3,6-dihydro-1,3,4-thiadiazin-2-on
3-(2-(Thiophen-2-carbonylamino)-benzyl)-5-(3,4-dimethoxyphenyl)-3,6-dihydro-1,3,4-thiadiazin-2-on
3-(4-(Thiophen-2-carbonylamino)-benzyl)-5-(3,4-dimethoxyphenyl)-6-ethyl-3,6-dihydro-1,3,4-thiadiazin-2-on
3-(3-(Thiophen-2-carbonylamino)-benzyl)-5-(3,4-dimethoxyphenyl)-6-ethyl-3,6-dihydro-1,3,4-thiadiazin-2-on
3-(2-(Thiophen-2-carbonylamino)-benzyl)-5-(3,4-dimethoxyphenyl)-6-ethyl-3,6-dihydro-1,3,4-thiadiazin-2-on
3-(4-(Thiophen-2-carbonylamino)-benzyl)-5-(3-methoxy-4-trifluor-methoxyphenyl)-6-ethyl-3,6-dihydro -1,3,4-thiadiazin-2-on
3-(4-(Thiophen-2-carbonylamino)-benzyl)-5-(3-methoxy-4-difluor-methoxyphenyl)-6-ethyl-3,6-dihydro-1,3,4-thiadiazin-2-on
3-(4-(Thiophen-2-carbonylamino)-benzyl)-5-(3-methoxy-4-fluor-methoxyphenyl)-6-ethyl-3,6-dihydro-1,3,4-thiadiazin-2-on
3-(4-(Thiophen-2-carbonylamino)-benzyl)-5-(3-difluormethoxy-4-methoxyphenyl)-6-ethyl-3,6-dihydro-1,3,4-thiadiazin-2-on
3-(4-(Thiophen-2-carbonylamino)-benzyl)-5-(3-trifluormethoxy-4-methoxyphenyl)-6-ethyl-3,6-dihydro-1,3,4-thiadiazin-2-on
3-(4-(Thiophen-2-carbonylamino)-benzyl)-5-(3-fluormethoxy-4-methoxyphenyl)-6-ethyl-3,6-dihydro-1,3,4-thiadiazin-2-on
3-(4-(Thiophen-2-carbonylamino)-benzyl)-5-(3-methoxy-4-ethoxy-phenyl)-6-ethyl-3,6-dihydro-1,3,4-thiadiazin-2-on
3-(4-(Thiophen-2-carbonylamino)-benzyl)-5-(3-ethoxy-4-methoxy-phenyl)-6-ethyl-3,6-dihydro-1,3,4-thiadiazin-2-on
3-(4-(Thiophen-2-carbonylamino)-benzyl)-5-(3-hydroxy-4-methoxy-phenyl)-6-ethyl-3,6-dihydro-1,3,4-thiadiazin-2-on
3-(4-(Thiophen-2-carbonylamino)-benzyl)-5-(4-methylsulfonylphenyl)-6-ethyl-3,6-dihydro-1,3,4-thiadiazin-2-on
3-(4-(Thiophen-2-carbonylamino)-benzyl)-5-(4-methylenoxyphenyl)-6-ethyl-3,6-dihydro-1,3,4-thiadiazin-2-on
3-(4-(Thiophen-2-carbonylamino)-benzyl)-5-(3-cyclopentyloxy-4-methoxy-phenyl)-6-ethyl-3,6-dihydro-1,3,4-thiadiazin-2-on
3-(3-(Thiophen-2-carbonylamino)-benzyl)-5-(3-cyclopentyloxy-4-methoxy-phenyl)-6-ethyl-3,6-dihydro-1,3,4-thiadiazin-2-on
3-(4-(thiophen-2-carbonylamino)-phenethyl)-5-(3,4-dimethoxyphenyl-3,6-dihydro-1,3,4-thiadiazin-2-on
3-(4-(Thiophen-2-carbonylamino)-phenethyl)-5-(3,4-dimethoxyphenyl)-6-ethyl-3,6-dihydro-1,3,4-thiadiazin-2-on
3-(4-(Thiophen 2-carbonylamino)-benzyl)-5-(3,4-dimethoxyphenyl)-3,6-dihydro-1,3,4-oxadiazin-2-on
3-(3-(Thiophen-2-carbonylamino)-benzyl)-5-(3,4-dimethoxyphenyl)-3,6-dihydro-1,3,4-oxadiazin-2-on
3-(2-(Thiophen-2-carbonylamino)-benzyl)-5-(3,4-dimethoxyphenyl)-3,6-dihydro-1,3,4-oxadiazin-2-on
3-(4-(Thiophen-2-carbonylamino)-benzyl)-5-(3,4-dimethoxyphenyl)-6-ethyl-3,6-dihydro-1,3,4-oxadiazin-2-on
3-(3-(Thiophen-2-carbonylamino)-benzyl)-5-(3,4-dimethoxyphenyl)-6-ethyl-3,6-dihydro-1,3,4-oxadiazin-2-on
3-(2-(Thiophen-2-carbonylamino)-benzyl)-5-(3,4-dimethoxyphenyl)-6-ethyl-3,6-dihydro-1,3,4-oxadiazin-2-on
3-(4-(Thiophen-2-carbonylamino)-benzyl)-5-(3-methoxy-4-trifluor-methoxyphenyl)-6-ethyl-3,6-dihydro-1,3,4-oxadiazin-2-on
3-(4-(Thiophen-2-carbonylamino)-benzyl)-5-(3-methoxy-4-difluormethoxyphenyl)-6-ethyl-3,6-dihydro-1,3,4-oxadiazin-2-on
3-(4-(Thiophen-2-carbonylamino)-benzyl)-5-(3-methoxy-4-fluor-methoxyphenyl)-6-ethyl-3,6-dihydro-1,3,4-oxadiazin-2-on
3-(4-(Thiophen-2-carbonylamino)-benzyl)-5-(3-difluormethoxy-4-methoxyphenyl)-6-ethyl-3,6-dihydro-1,3,4-oxadiazin-2-on
3-(4-(Thiophen-2-carbonylamino)-benzyl)-5-(3-trifluormethoxy-4-methoxyphenyl)-6-ethyl-3,6-dihydro-1,3,4-oxadiazin-2-on
3-(4-(Thiophen-2-carbonylamino)-benzyl)-5-(3-fluormethoxy-4-methoxyphenyl)-6-ethyl-3,6-dihydro-1,3,4-oxadiazin-2-on
3-(4-(Thiophen-2-carbonylamino)-benzyl)-5-(3-Methoxy-4-ethoxy-phenyl)-6-ethyl-3,6-dihydro-1,3,4-oxadiazin-2-on
3-(4-(Thiophen-2-carbonylamino)-benzyl)-5-(3-ethoxy-4-methoxy-phenyl)-6-ethyl-3,6-dihydro-1,3,4-oxadiazin-2-on
3-(4-(Thiophen-2-carbonylamino)-benzyl)-5-(3-hydroxy-4-methoxy-phenyl)-6-ethyl-3,6-dihydro-1,3,4-oxadiazin-2-on
3-(4-(Thiophen-2-carbonylamino)-benzyl)-5-(4-methylsulfonylphenyl)-6-ethyl-3,6-dihydro-1,3,4-oxadiazin-2-on
3-(4-(Thiophen-2-carbonylamino)-benzyl)-5-(4-methylenoxyphenyl)-6-ethyl-3,6-dihydro-1,3,4-oxadiazin-2-on
3-(4-(Thiophen-2-carbonylamino)-benzyl)-5-(3-cyclopentyloxy-4-methoxy-phenyl)-6-ethyl-3,6-dihydro-,1,3,4-oxadiazin-2-on
3-(3-(Thiophen-2-carbonylamino)-benzyl)-5-(3-cyclopentyloxy-4-methoxy-phenyl)-6-ethyl-3,6-dihydro-1,3,4-oxadiazin-2-on
3-(4-(Thiophen-2-carbonylamino)-phenethyl)-5-(3,4-dimethoxyphenyl)-3,6-dihydro-1,3,4-oxadiazin-2-on
3-(4-(Thiophen-2-carbonylamino)-phenethyl)-5-(3,4-dimethoxyphenyl)-6-ethyl-3,6-dihydro-1,3,4-oxadiazin-2-on.

Analog erhält man durch Umsetzung der oben aufgeführten "Amin-derivate" mit Pyrazin-2-carbonsäurechlorid die nachstehenden Verbindungen
2-(4-Pyrazincarbonylamino-benzyl)-6-(3,4-dimethoxyphenyl)-2,3,4,5-tetrahydro-pyridazin-3-on, F. 197°
2-(3-Pyrazincarbonylamino-benzyl)-6-(3,4-dimethoxyphenyl)-2,3,4,5-tetrahydro-pyridazin-3-on
2-(2-Pyrazincarbonylamino-benzyl)-6-(3,4-dimethoxyphenyl)-2,3,4,5-tetrahydro-pyridazin-3-on
2-(4-Pyrazincarbonylamino-benzyl)-6-(3,4-dimethoxyphenyl)-5-ethyl-2,3,4,5-tetrahydro-pyridazin-3-on:
2-(3-Pyrazincarbonylamino-benzyl)4-(3,4-dimethoxyphenyl)-5-ethyl-2,3,4,5-tetrahydro-pyridazin-3-on
2-(2-Pyrazincarbonylamino-benzyl)-6-(3,4-dimethoxyphenyl)-5-ethyl-2,3,4,5-tetrahydro-pyridazin-3-on
2-(4-Pyrazincarbonylamino-benzyl)-6-(3-methoxy-4-trifluor-methoxy-phenyl)-5-ethyl-2,3,4,5-tetrahydro-pyridazin-3-on
2-(4-Pyrazincarbonylamino-benzyl)-6-(3-methoxy-4-difluor-methoxy-phenyl)-5-ethyl-2,3,4,5-tetrahydro-pyridazin-3-on
2-(4-Pyrazincarbonylamino-benzyl)-6-(3-methoxy-4-fluor-methoxy-phenyl)-5-ethyl-2,3,4,5-tetrahydro-pyridazin-3-on
2-(4-Pyrazincarbonylamino-benzyl)-6-(3-difluormethoxy-4-methoxy-phenyl)-5-ethyl-2,3,4,5-tetrahydro-pyridazin-3-on
2-(4-Pyrazincarbonylamino-benzyl)-6-(3-trifluormethoxy-4-methoxyphenyl)-5-ethyl-2,3,4,5-tetrahydro-pyridazin-3-on
2-(4-Pyrazincarbonylamino-benzyl)-6-(3-fluormethoxy-4-methoxy-phenyl)-5-ethyl-2,3,4,5-tetrahydro-pyridazin-3-on
2-(4-Pyrazincarbonylamino-benzyl)-6-(3-methoxy-4-ethoxy-phenyl)-5-ethyl-2,3,4,5-tetrahydro-pyridazin-3-on
2-(4-Pyrazincarbonylamino-benzyl)-6-(3-ethoxy-4-methoxy-phenyl)-5-ethyl-2,3,4,5-tetrahydro-pyridazin-3-on
2-(4-Pyrazincarbonylamino-benzyl)-6-(3-hydroxy-4-methoxy-phenyl)-5-ethyl-2,3,4,5-tetrahydro-pyridazin 3-on
2-(4-Pyrazincarbonylamino-benzyl)-6-(4-methylsulfonylphenyl)-5-ethyl-2,3,4,5-tetrahydro-pyridazin-3-on
2-(4-Pyrazincarbonylamino-benzyl)-6-(4-methylenoxyphenyl)-5-ethyl-2,3,4,5-tetrahydro-pyridazin-3-on
2-(4-Pyrazincarbonylamino-benzyl)-6-(3-cyclopentyloxy-4-methoxy-phenyl)-5-ethyl-2,3,4,5-tetrahydro-pyridazin-3-on
2-(3-Pyrazincarbonylamino-benzyl)-6-(3-cyclopentyloxy-4-methoxy-phenyl)-5-ethyl-2,3,4,5-tetrahydro-pyridazin-3-on
2-(4-Pyrazincarbonylamino-phenethyl)-6-(3,4-dimethoxyphenyl)-2,3,4,5-tetrahydro-pyridazin-3-on
2-(4-Pyrazincarbonylamino-phenethyl)-6-(3,4-dimethoxyphenyl)-5-ethyl-2,3,4,5-tetrahydro-pyridazin-3-on
3-(4-Pyrazincarbonylamino-benzyl)-5-(3,4-dimethoxyphenyl)-3,6-dihydro-1,3,4-thiadiazin-2-on, F. 202°
3-(3-Pyrazincarbonylamino-benzyl)-5-(3,4-dimethoxyphenyl)-3,6-dihydro-1,3,4-thiadiazin-2-on
3-(2-Pyrazincarbonylamino-benzyl)-5-(3,4-dimethoxyphenyl)-3,6-dihydro-1,3,4-thiadiazin-2-on
3-(4-Pyrazincarbonylamino-benzyl)-5-(3,4-dimethoxyphenyl)-6-ethyl-3,6-dihydro-1,3,4-thiadiazin-2-on
3-(3-Pyrazincarbonylamino-benzyl)-5-(3,4-dimethoxyphenyl)-6-ethyl-3,6-dihydro-1,3,4-thiadiazin-2-on
3-(2-Pyrazincarbonylamino-benzyl)-5-(3,4-dimethoxyphenyl)-6-ethyl-3,6-dihydro-1,3,4-thiadiazin-2-on
3-(4-Pyrazincarbonylamino-benzyl)-5-(3-methoxy-4-trifluor-methoxy-phenyl)-6-ethyl-3,6-dihydro-1,3,4-thiadiazin-2-on
3-(4-Pyrazincarbonylamino-benzyl)-5-(3-methoxy-4-difluor-methoxy-phenyl)-6-ethyl-3,6-dihydro-1,3,4-thiadiazin-2-on
3-(4-Pyrazincarbonylamino-benzyl)-5-(3-methoxy-4-fluor-methoxy-phenyl)-6-ethyl-3,6-dihydro-1,3,4-thiadiazin-2-on
3-(4-Pyrazincarbonylamino-benzyl)-5-(3-difluormethoxy-4-methoxy-phenyl)-6-ethyl-3,6-dihydro-1,3,4-thiadiazin-2-on
3-(4-Pyrazincarbonylamino-benzyl)-5-(3-trifluormethoxy-4-methoxy-phenyl)-6-ethyl-3,6-dihydro-1,3,4-thiadiazin-2-on
3-(4-Pyrazincarbonylamino-benzyl)-5-(3-fluormethoxy-4-methoxy-phenyl)-6-ethyl-3,6-dihydro-1,3,4-thiadiazin-2-on
3-(4-Pyrazincarbonylamino-benzyl)-5-(3-methoxy-4-ethoxy-phenyl)-6-ethyl-3,6-dihydro-1,3,4-thiadiazin-2-on
3-(4-Pyrazincarbonylamino-benzyl)-5-(3-ethoxy-4-methoxy-phenyl)-6-ethyl-3,6-dihydro-1,3,4-thiadiazin-2-on
3-(4-Pyrazincarbonylamino-benzyl)-5-(3-hydroxy-4-methoxy-phenyl)-6-ethyl-3,6-dihydro-1,3,4-thiadiazin-2-on
3-(4-Pyrazincarbonylamino-benzyl)-5-(4-methylsulfonylphenyl)-6-ethyl-3,6-dihydro-1,3,4-thiadiazin-2-on
3-(4-Pyrazincarbonylamino-benzyl)-5-(4-methylenoxyphenyl)-6-ethyl-3,6-dihydro-1,3,4 thiadiazin-2-on
3-(4-Pyrazincarbonylamino-benzyl)-5-(3-cyclopentyloxy-4-methoxy-phenyl)-6-ethyl-3,6-dihydro-1,3,4-thiadiazin-2-on
3-(3-Pyrazincarbonylamino-benzyl)-5-(3-cyclopentyloxy-4-methoxy-phenyl)-6-ethyl-3,6-dihydro-1,3,4-thiadiazin-2-on
3-(-4-Pyrazincarbonylamino-phenethyl)-5,3,4-dimethoxyphenyl)-3,6-dihydro-1,3,4-thiadiazin-2-on
3-(4-Pyrazincarbonylamino-phenethyl)-5-(3,4-dimethoxyphenyl)-6-ethyl-3,6-dihydro-1,3,4-thiadiazin-2-on
3-(4-Pyrazincarbonylamino-benzyl)-5-(3,4-dimethoxyphenyl)-3,6-dihydro-1,3,4-oxadiazin-2-on
3-(3-Pyrazincarbonylamino-benzyl)-5-(3,4-dimethoxyphenyl)-3,6-dihydro-1,3,4-oxadiazin-2-on
3-(2-Pyrazincarbonylamino-benzyl)-5-(3,4-dimethoxyphenyl)-3,6-dihydro-1,3,4-oxadiazin-2-on
3-(4-Pyrazincarbonylamino-benzyl)-5-(3,4-dimethoxyphenyl)-6-ethyl-3,6-dihydro-1,3,4-oxadiazin-2-on
3-(3-Pyrazincarbonylamino-benzyl)-5-(3,4-dimethoxyphenyl)-6-ethyl-3,6-dihydro-1,3,4-oxadiazin-2-on
3-(2-Pyrazincarbonylamino-benzyl)-5-(3,4-dimethoxyphenyl)-6-ethyl-3,6-dihydro-1,3,4-oxadiazin-2-on
3-(4-Pyrazincarbonylamino-benzyl)-5-(3-methoxy-4-trifluor-methoxy-phenyl)-6-ethyl-3,6-dihydro-1,3,4-oxadiazin-2-on
3-(4-Pyrazincarbonylamino-benzyl)-5-(3-methoxy-4-difluor-methoxy-phenyl)-6-ethyl-3,6-dihydro-1,3,4-oxadiazin-2-on
3-(4-Pyrazincarbonylamino-benzyl)-5-(3-methoxy-4-fluor-methoxy-phenyl)-6-ethyl-3,6-dihydro-1,3,4-oxadiazin-2-on
3-(4-Pyrazincarbonylamino-benzyl)-5-(3-difluormethoxy-4-methoxy-phenyl)-6-ethyl-3,6-dihydro-1,3,4-oxadiazin-2-on
3-(4-Pyrazincarbonylamino-benzyl)-5-(3-trifluormethoxy-4-methoxy-phenyl)-6-ethyl-3,6-dihydro-1,3,4-oxadiazin-2-on
3-(4-Pyrazincarbonylamino-benzyl)-5-(3 fluormethoxy-4-methoxy-phenyl)-6-ethyl-3,6-dihydro-1,3,4-oxadiazin-2-on
3-(4-Pyrazincarbonylamino-benzyl)-5-(3-Methoxy-4-ethoxy-phenyl)-6-ethyl-3,6-dihydro-1,3,4-oxadiazin-2-on
3-(4-Pyrazincarbonylamino-benzyl)-5-(3-ethoxy-4-methoxy-phenyl)-6-ethyl-3,6-dihydro-1,3,4-oxadiazin-2-on
3-(4-Pyrazincarbonylamino-benzyl)-5-(3-hydroxy-4-methoxy-phenyl)-6-ethyl-3,6-dihydro-1,3,4-oxadiazin-2-on
3-(4-Pyrazincarbonylamino-benzyl)-5-(4-methylsulfonylphenyl)-6-ethyl-3,6-dihydro-1,3,4-oxadiazin-2-on
3-(4-Pyrazincarbonylamino-benzyl)-5-(4-methylenoxyphenyl)-6-ethyl-3,6-dihydro-1,3,4-oxadiazin 2-on
3-(4-Pyrazincarbonylamino-benzyl)-5-(3-cyclopentyloxy-4-methoxy-phenyl)-6-ethyl-3,6-dihydro-1,3,4-oxadiazin-2-on
3-(3-Pyrazincarbonylamino-benzyl)-5-(3-cyclopentyloxy-4-methoxy-phenyl)-6-ethyl-3,6-dihydro-1,3,4-oxadiazin-2-on
3-(4-Pyrazincarbonylamino-phenethyl)-5-(3,4-dimethoxyphenyl)-3,6-dihydro-1,3,4-oxadiazin-2-on
3-(4-Pyrazincarbonylamino-phenethyl)-5-(3,4-dimethoxyphenyl)-6-ethyl-3,6-dihydro-1,3,4-oxadiazin-2-on.

Analog erhält man durch Umsetzung der oben aufgeführten "Aminderivate" mit 6-Chlor-pyridazin-3-carbonsäurechlorid die nachstehenden Verbindungen
2-(4-(6-Chlor-pyridazin-3-carbonylamino)-benzyl)-6-(3,4-dimethoxy-phenyl)-2,3,4,5-tetrahydro-pyridazin-3-on
2-(3-(6-Chlor-pyridazin-3-carbonylamino)-benzyl)-6-(3,4-dimethoxy-phenyl)-2,3,4,5-tetrahydro-pyridazin-3-on
2-(2-(6-Chlor-pyridazin-3-carbonylamino)-benzyl)-6-(3,4-dimethoxy-phenyl)-2,3,4,5-tetrahydro-pyridazin-3-on
2-(4-(6-Chlor-pyridazin-3-carbonylamino)-benzyl)-6-(3,4-dimethoxy-phenyl)-5-ethyl-2,3,4,5-tetrahydro-pyridazin-3-on;
2-(3-(6-Chlor-pyridazin-3-carbonylamino)-benzyl)-6-(3,4-dimethoxy-phenyl)-5-ethyl-2,3,4,5-tetrahydro-pyridazin-3-on
2-(2-(6-Chlor-pyridazin-3-carbonylamino)-benzyl)-6-(3,4-dimethoxy-phenyl)-5-ethyl-2,3,4,5-tetrahydro-pyridazin-3-on
2-(4-(6-Chlor-pyridazin-3-carbonylamino)-benzyl)-6-(3-methoxy-4-trifluor-methoxyphenyl)-5-ethyl-2,3,4,5-tetrahydro-pyridazin-3-on
2-(4-(6-Chlor-pyridazin-3-carbonylamino)-benzyl)-6-(3-methoxy-4-difluor-methoxyphenyl)-5-ethyl-2,3,4,5-tetrahydro-pyridazin-3-on
2-(4-(6-Chlor-pyridazin-3-carbonylamino)-benzyl)-6-(3-methoxy-4-fluor-methoxyphenyl)-5-ethyl-2,3,4,5 tetrahydro-pyridazin-3-on
2-(4-(6-Chlor-pyridazin-3-carbonylamino)-benzyl)-6-(3-difluormethoxy-4-methoxyphenyl)-5-ethyl-2,3,4,5-tetrahydro-pyridazin-3-on
2-(4-(6-Chlor-pyridazin-3-carbonylamino)-benzyl)-6-(3-trifluormethoxy-4-methoxyphenyl)-5-ethyl-2,3,4,5-tetrahydro-pyridazin-3-on
2-(4-(6-Chlor-pyridazin-3-carbonylamino)-benzyl)-6-(3-fluormethoxy-4-methoxyphenyl)-5-ethyl-2,3,4,5-tetrahydro-pyridazin-3-on
2-(4-(6-Chlor-pyridazin-3-carbonylamino)-benzyl)-6-(3-methoxy-4-ethoxy-phenyl)-5-ethyl-2,3,4,5-tetrahydro-pyridazin-3-on
2-(4-(6-Chlor-pyridazin-3-carbonylamino)-benzyl)-6-(3-ethoxy-4-methoxy-phenyl)-5-ethyl-2,3,4,5-tetrahydro-pyridazin-3-on
2-(4-(6-Chlor-pyridazin-3-carbonylamino)-benzyl)-6-(3-hydroxy-4-methoxy-phenyl)-5-ethyl-2,3,4,5-tetrahydro-pyridazin-3-on
2-(4-(6-Chlor-pyridazin-3-carbonylamino)-benzyl)-6-(4-methylsulfonylphenyl)-5-ethyl-2,3,4,5-tetrahydro-pyridazin-3-on
2-(4-(6-Chlor-pyridazin-3-carbonylamino)-benzyl)-6-(4-methylenoxy-phenyl)-5-ethy(-2,3,4,5-tetrahydro-pyridazin-3-on
2-(4-(6-Chlor-pyridazin-3-carbonylamino)-benzyl)-6-(3-cyclopentyloxy-4-methoxyphenyl)-5-ethyl-2,3,4,5-tetrahydro-pyridazin-3-on
2-(3-(6-Chlor-pyridazin-3-carbonylamino)-benzyl)-6-(3-cyclopentyloxy-4-methoxyphenyl)-5-ethyl-2,3,4,5-tetrahydro-pyridazin-3-on
2-(4-(6-Chlor-pyridazin-3-carbonylamino)-phenethyl)-6-(3,4-dimethoxy-phenyl)-2,3,4,5-tetrahydro-pyridazin-3-on
2-(4-(6-Chlor-pyridazin-3-carbonylamino)-phenethyl)-6-(3,4-dimethoxyphenyl)-5-ethyl-2,3,4,5-tetrahydro-pyridazin-3-on
3-(4-(6-Chlor-pyridazin-3-carbonylamino)-benzyl)-5-(3,4-dimethoxy-phenyl)-3,6-dihydro-1,3,4-thiadiazin-2-on
3-(3-(6-Chlor-pyridazin-3-carbonylamino)-benzyl)-5-(3,4-dimethoxyphenyl)-3,6-dihydro-1,3,4-thiadiazin-2-on
3-(2-(6-Chlor-pyridazin-3-carbonylamino)-benzyl)-5-(3,4-dimethoxy-phenyl)-3,6-dihydro-1,3,4-thiadiazin-2-on
3-(4-(6-Chlor-pyridazin-3-carbonylamino)-benzyl)-5-(3,4-dimethoxy-phenyl)-6-ethyl-3,6-dihydro-1,3,4-thiadiazin-2-on
3-(3-(6-Chlor-pyridazin-3-carbonylamino)-benzyl)-5-(3,4-dimethoxy-phenyl)-6-ethyl-3,6-dihydro-1,3,4-thiadiazin-2-on
3-(2-(6-Chlor-pyridazin-3-carbonylamino)-benzyl)-5-(3,4-dimethoxy-phenyl)-6-ethyl-3,6-dihydro-1,3,4-thiadiazin-2-on
3-(4-(6-Chlor-pyridazin-3-carbonylamino)-benzyl)-5-(3-methoxy-4-trifluor-methoxyphenyl)-6-ethyl-3,6-dihydro-1,3,4-thiadiazin-2-on
3-(4-(6-Chlor-pyridazin-3-carbonylamino)-benzyl)-5-(3-methoxy-4-difluor-methoxyphenyl)-6-ethyl-3,6-dihydro-1,3,4-thiadiazin-2-on
3-(4-(6-Chlor-pyridazin-3-carbonylamino)-benzyl)-5-(3-methoxy-4-fluor-methoxyphenyl)-6-ethyl-3,6-dihydro-1,3,4-thiadiazin-2-on
3-(4-(6-Chlorpyridazin-3-carbonylamino)-benzyl)-5-(3-difluormethoxy-4-methoxyphenyl)-6-ethyl-3,6-dihydro-1,3,4-thiadiazin-2-on
3-(4-(6-Chlor-pyridazin-3-carbonylamino)-benzyl)-5-(3-trifluormethoxy-4-methoxyphenyl)4-ethyl-3,6-dihydro-1,3,4-thiadiazin-2-on
3-(4-(6-Chlor-pyridazin-3-carbonylamino)-benzyl)-5-(3-fluormethoxy-4-methoxyphenyl)-6-ethyl-3,6-dihydro-1,3,4-thiadiazin-2-on
3-(4-(6-Chlor-pyridazin-3-carbonylamino)-benzyl)-5-(3-methoxy-4-ethoxy-phenyl)-6-ethyl-3,6-dihydro-1,3,4-thiadiazin-2-on
3-(4-(6-Chlor-pyridazin-3-carbonylamino)-benzyl)-5-(3-ethoxy-4-methoxy-phenyl)4-ethyl-3,6-dihydro-1,3,4-thiadiazin-2-on
3-(4-(6-Chlor-pyridazin-3-carbonylamino)-benzyl)-5-(3-hydroxy-4-methoxy-phenyl)-6-ethyl-3,6-dihydro-1,3,4-thiadiazin-2-on
3-(4-(6-Chlor-pyridazin-3-carbonylamino)-benzyl)-5-(4-methylsulfonylphenyl)-6-ethyl-3,6-dihydro-1,3,4-thiadiazin-2-on
3-(4-(6-Chlor-pyridazin-3-carbonylamino)-benzyl)-5-(4-methylenoxy-phenyl)-6-ethyl-3,6-dihydro 1,3,4-thiadiazin-2-on
3-(4-(6-Chlor-pyridazin-3-carbonylamino)-benzyl)-5-(3-cyclopentyloxy-4-methoxy-phenyl)-6-ethyl-3,6-dihydro-1,3,4-thiadiazin-2-on
3-(3-(6-Chlor-pyridazin-3-carbonylamino)-benzyl)-5-(3-cyclopentyloxy-4-methoxy-phenyl)-6-ethyl-3,6-dihydro-1,3,4-thiadiazin-2-on
3-(4-(6-Chlor-pyridazin-3-carbonylamino)-phenethyl)-5-(3,4-dimethoxy-phenyl)-3,6-dihydro-1,3,4-thiadiazin-2-on
3-(4-(6-Chlor-pyridazin-3-carbonylamino)-phenethyl)-5-(3,4-dimethoxy-phenyl)-6-ethyl-3,6-dihydro-1,3,4-thiadiazin-2-on
3-(4-(6-Chlor-pyridazin-3-carbonylamino)-benzyl)-5-(3,4-dimethoxy-phenyl)-3,6-dihydro-1,3,4-oxadiazin-2-on
3-(3-(6-Chlor-pyridazin-3-carbonylamino)-benzyl)-5-(3,4-dimethoxyphenyl)-3,6-dihydro-1,3,4-oxadiazin-2-on
3-(2-(6-Chlor-pyridazin-3-carbonylamino)-benzyl)-5-(3,4-dimethoxy-phenyl)-3,6-dihydro-1,3,4-oxadiazin-2-on
3-(4-(6-Chlor-pyridazin-3-carbonylamino)-benzyl)-5-(3,4-dimethoxyphenyl)-6-ethyl-3,6-dihydro-1,3,4-oxadiazin-2-on
3-(3-(6-Chlor-pyridazin-3-carbonylamino)-benzyl)-5-(3,4-dimethoxyphenyl)-6-ethyl-3,6-dihydro-1,3,4-oxadiazin-2-on
3-(2-(6-Chlor-pyridazin-3-carbonylamino)-benzyl)-5-(3,4-dimethoxyphenyl)-6-ethyl-3,6-dihydro-1,3,4-oxadiazin-2-on
3-(4-(6-Chlor-pyridazin-3-carbonylamino)-benzyl)-5-(3-methoxy-4-trifluor-methoxyphenyl)-6-ethyl-3,6-dihydro-1,3,4-oxadiazin-2-on
3-(4-(6-Chlor-pyridazin-3-carbonylamino)-benzyl)-5-(3-methoxy-4-difluor-methoxyphenyl)-6-ethyl-3,6-dihydro-1,3,4-oxadiazin-2-on
3-(4-(6-Chlor-pyridazin-3-carbonylamino)-benzyl)-5-(3-methoxy-4-fluor-methoxyphenyl)-6-ethyl-3,6-dihydro-1,3,4-oxadiazin-2-on
3-(4-(6-Chlor-pyridazin-3-carbonylamino)-benzyl)-5-(3-difluormethoxy-4-methoxyphenyl)-6-ethyl-3,6-dihydro-1,3,4-oxadiazin-2-on
3-(4-(6-Chlor-pyridazin-3-carbonylamino)-benzyl)-5-(3-trifluormethoxy-4-methoxyphenyl)-6-ethyl-3,6-dihydro-1,3,4-oxadiazin-2-on
3-(4-(6-Chlor-pyridazin-3-carbonylamino)-benzyl)-5-(3-fluormethoxy-4-methoxyphenyl)-6-ethyl-3,6-dihydro-1,3,4-oxadiazin-2-on
3-(4-(6-Chlor-pyridazin-3-carbonylamino)-benzyl)-5-(3-methoxy-4-ethoxy-phenyl)-6-ethyl-3,6-dihydro-1,3,4-oxadiazin-2-on
3-(4-(6-Chlor-pyridazin-3-carbonylamino)-benzyl)-5-(3-ethoxy-4-methoxy-phenyl)-6-ethyl-3,6-dihydro-1,3,4-oxadiazin-2-on
3-(4-(6-Chlor-pyridazin-3-carbonylamino)-benzyl)-5-(3-hydroxy-4-methoxy-phenyl)-6-ethyl-3,6-dihydro-1,3,4-oxadiazin-2-on
3-(4-(6-Chlor-pyridazin-3-carbonylamino)-benzyl)-5-(4-methylsulfonylphenyl)-6-ethyl-3,6-dihydro-1,3,4-oxadiazin-2-on
3-(4-(6-Chlor-pyridazin-3-carbonylamino)-benzyl)-5-(4-methylenoxyphenyl)-6-ethyl-3,6-dihydro-1,3,4-oxadiazin-2-on
3-(4-(6-Chlor-pyridazin-3-carbonylamino)-benzyl)-5-(3-cyclopentyloxy-4-methoxy-phenyl)-6-ethyl-3,6-dihydro-1,3,4-oxadiazin-2-on
3-(3-(6-Chlor-pyridazin-3-carbonylamino)-benzyl)-5-(3-cyclopentyloxy-4-methoxy-phenyl)-6-ethyl-3,6-dihydro-1,3,4-oxadiazin-2-on
3-(4-(6-Chlor-pyridazin-3-carbonylamino)-phenethyl)-5-(3,4-dimethoxyphenyl)-3,6-dihydro-1,3,4-oxadiazin-2-on
3-(4-(6-Chlor-pyridazin-3-carbonylamino)-phenethyl)-5-(3,4-dimethoxy-phenyl)-6-ethyl-3,6-dihydro-1,3,4-oxadiazin-2-on.

Analog erhält man durch Umsetzung der oben aufgeführten "Aminderivate" mit Imidazol-4-carbonsäurechlorid die nachstehenden Verbindungen
2-(4-(Imidazol-4-carbonylamino)-benzyl)-6-(3,4-dimethoxyphenyl)-2,3,4,5-tetrahydro-pyridazin-3-on
2-(3-(Imidazol-4-carbonylamino)-benzyl)-6-(3,4-dimethoxyphenyl)-2,3,4,5-tetrahydro-pyridazin-3-on
2-(2-(Imidazol-4-carbonylamino)-benzyl)-6-(3,4-dimethoxyphenyl)-2,3,4,5-tetrahydro-pyridazin-3-on
2-(4-(Imidazol-4-carbonylamino)-benzyl)-6-(3,4-dimethoxyphenyl)-5-ethyl-2,3,4,5-tetrahydro-pyridazin-3-on;
2-(3-(Imidazol-4-carbonylamino)-benzyl)-6-(3,4-dimethoxyphenyl)-5-ethyl-2,3,4,5-tetrahydro-pyridazin-3-on
2-(2-(Imidazol-4-carbonylamino)-benzyl)-6-(3,4-dimethoxyphenyl)-5-ethyl-2,3,4,5-tetrahydro-pyridazin-3-on
2-(4-(Imidazol-4-carbonylamino)-benzyl)-6-(3-methoxy-4-trifluor-methoxyphenyl)-5-ethyl-2,3,4,5-tetrahydro-pyridazin-3-on
2-(4-(Imidazol-4-carbonylamino)-benzyl)-6-(3-methoxy-4-difluor-methoxyphenyl)-5-ethyl-2,3,4,5-tetrahydro-pyridazin-3-on
2-(4-(Imidazol-4-carbonylamino)-benzyl)-6-(3-methoxy-4-fluor-methoxy-phenyl)-5-ethyl-2,3,4,5-tetrahydro-pyridazin-3-on
2-(4-(Imidazol-4-carbonylamino)-benzyl)-6-(3-difluormethoxy-4-methoxyphenyl)-5-ethyl-2,3,4,5-tetrahydro-pyridazin-3-on
2-(4-(Imidazol-4-carbonylamino)-benzyl)-6-(3-trifluormethoxy-4-methoxyphenyl)-5-ethyl-2,3,4,5-tetrahydro-pyridazin-3-on
2-(4-(Imidazol-4-carbonylamino)-benzyl)4-(3-fluormethoxy-4-methoxy-phenyl)-5-ethyl-2,3,4,5-tetrahydro-pyridazin-3-on
2-(4-(Imidazol-4-carbonylamino)-benzyl)-6-(3-methoxy-4-ethoxy-phenyl)-5-ethyl-2,3,4,5-tetrahydro-pyridazin-3-on
2-(4-(Imidazol-4-carbonylamino)-benzyl)-6-(3-ethoxy-4-methoxy-phenyl)-5-ethyl-2,3,4,5-tetrahydro-pyridazin-3-on
2-(4-(Imidazol-4-carbonylamino)-benzyl)-6-(3-hydroxy-4-methoxy-phenyl)-5-ethyl-2,3,4,5-tetrahydro-pyridazin-3-on
2-(4-(Imidazol-4-carbonylamino)-benzyl)4-(4-methylsutfonylphenyl)-5-ethyl-2,3,4,5-tetrahydro-pyridazin-3-on
2-(4-(Imidazol-4-carbonylamino)-benzyl)-6-(4-methytenoxyphenyl)-5-ethyl-2,3,4,5-tetrahydro-pyridazin-3-on
2-(4-(Imidazol-4-carbonylamino)-benzyl)-6-(3-cyclopentyloxy-4-methoxyphenyl)-5-ethyl-2,3,4,5-tetrahydro-pyridazin-3-on
2-(3-(Imidazol-4-carbonylamino)-benzyl)-6-(3-cyclopentyloxy-4-methoxyphenyl)-5-ethyl-2,3,4,5-tetrahydro-pyridazin-3-on
2-(4-(Imidazol-4-carbonylamino)-phenethyl)-6-(3,4-dimethoxyphenyl)-2,3,4,5-tetrahydro-pyridazin-3-on
2-(4-(Imidazol-4-carbonylamino)-phenethyl)-6-(3,4-dimethoxyphenyl)-5-ethyl-2,3,4,5-tetrahydro-pyridazin-3-on
3-(4-(Imidazol-4-carbonylamino)-benzyl)-5-(3,4-dimethoxyphenyl)-3,6-dihydro-1,3,4-thiadiazin-2-on
3-(3-(lmidazol-4-carbonylamino)-benzyl)-5-(3,4-dimethoxyphenyl)-3,6-dihydro-1,3,4-thiadiazin-2-on
3-(2-(Imidazol-4-carbonylamino)-benzyl)-5-(3,4-dimethoxyphenyl)-3,6-dihydro-1,3,4-thiadiazin-2-on
3-(4-(Imidazol-4-carbonylamino)-benzyl)-5-(3,4-dimethoxyphenyl)-6-ethyl-3,6-dihydro-1,3,4-thiadiazin-2-on
3-(3-(Imidazol-4-carbonylamino)-benzyl)-5-(3,4-dimethoxyphenyl)-6-ethyl-3,6-dihydro-1,3,4-thiadiazin-2-on
3-(2-(lmidazol-4-carbonylamino)-benzyl)-5-(3,4-dimethoxyphenyl)-6-ethyl-3,6-dihydro-1,3,4-thiadiazin-2-on
3-(4-(Imidazol-4-carbonylamino)-benzyl)-5-(3-methoxy-4-trifluor-methoxyphenyl)-6-ethyl-3,6-dihydro-1,3,4-thiadiazin-2-on
3-(4-(lmidazol-4-carbonylamino)-benzyl)-5-(3-methoxy-4-difluor-methoxyphenyl)-6-ethyl-3,6-dihydro-1,3,4-thiadiazin-2-on
3-(4-(Imidazol-4-carbonylamino)-benzyl)-5-(3-methoxy-4-fluor-methoxy-phenyl)-6-ethyl-3,6-dihydro-1,3,4-thiadiazin-2-on
3-(4-(Imidazol-4-carbonylamino)-benzyl)-5-(3-difluormethoxy-4-methoxyphenyl)-6-ethyl-3,6-dihydro-1,3,4-thiadiazin-2-on
3-(4-(Imidazol-4-carbonylamino)-benzyl)-5-(3-trifluormethoxy-4-methoxyphenyl)-6-ethyl-3,6-dihydro-1,3,4-thiadiazin-2-on
3-(4-(lmidazol-4-carbonylamino)-benzyl)-5-(3-fluormethoxy-4-methoxy-phenyl)-6-ethyl-3,6-dihydro-1,3,4-thiadiazin-2-on
3-(4-(Imidazol-4-carbonylamino)-benzyl)-5-(3-methoxy-4-ethoxy-phenyl)-6-ethyl-3,6-dihydro-1,3,4-thiadiazin-2-on
3-(4-(Imidazol-4-carbonylamino)-benzyl)-5-(3-ethoxy-4-methoxyphenyl)-6-ethyl-3,6-dihydro-1,3,4-thiadiazin-2-on
3-(4-(Imidazol-4-carbonylamino)-benzyl)-5-(3-hydroxy-4-methoxy-phenyl)-6-ethyl-3,6-dihydro-1,3,4-thiadiazin-2-on
3-(4-(Imidazol-4-carbonylamino)-benzyl)-5-(4-methylsulfonylphenyl)-6-ethyl-3,6-dihydro-1,3,4-thiadiazin-2-on
3-(4-(Imidazol-4-carbonylamino)-benzyl)-5-(4-methylenoxyphenyl)-6-ethyl-3,6-dihydro-1,3,4-thiadiazin-2-on
3-(4-(lmidazol-4-carbonylamino)-benzyl)-5-(3-cyclopentyloxy-4-methoxy-phenyl)-6-ethyl-3,6-dihydro-1,3,4-thiadiazin-2-on
3-(3-(Imidazol-4-carbonylamino)-benzyl)-5-(3-cyclopentyloxy-4-methoxy-phenyl)-6-ethyl-3,6-dihydro-1,3,4-thiadiazin-2-on
3-(4-(Imidazol-4-carbonylamino)-phenethyl)-5-(3,4-dimethoxyphenyl)-3,6-dihydro-1,3,4-thiadiazin-2-on
3-(4-(Imidazol-4-carbonylamino)-phenethyl)-5-(3,4-dimethoxyphenyl)-6-ethyl-3,6-dihydro-1,3,4-thiadiazin-2-on
3-(4-(Imidazol-4-carbonylamino)-benzyl)-5-(3,4-dimethoxyphenyl)-3,6-dihydro-1,3,4-oxadiazin-2-on
3-(3-(Imidazol-4-carbonylamino)-benzyl)-5-(3,4-dimethoxyphenyl)-3,6-dihydro-1,3,4-oxadiazin-2-on
3-(2-(Imidazol-4-carbonylamino)-benzyl)-5-(3,4-dimethoxyphenyl)-3,6-dihydro-1,3,4-oxadiazin-2-on
3-(4-(Imidazol-4-carbonylamino)-benzyl)-5-(3,4-dimethoxyphenyl)-6-ethyl-3,6-dihydro-1,3,4-oxadiazin-2-on
3-(3-(Imidazol-4-carbonylamino)-benzyl)-5-(3,4-dimethoxyphenyl)-6-ethyl-3,6-dihydro-1,3,4-oxadiazin-2-on
3-(2-(Imidazol-4-carbonylamino)-benzyl)-5-(3,4-dimethoxyphenyl)-6-ethyl-3,6-dihydro-1,3,4-oxadiazin-2-on
3-(4-(Imidazol-4-carbonylamino)-benzyl)-5-(3-methoxy-4-trifluor-methoxyphenyl)-6-ethyl-3,6-dihydro-1,3,4-oxadiazin-2-on
3-(4-(Imidazol-4-carbonylamino)-benzyl)-5-(3-methoxy-4-difluor-methoxyphenyl)-6-ethyl-3,6-dihydro-1,3,4-oxadiazin-2-on
3-(4-(Imidazol-4-carbonylamino)-benzyl)-5-(3-methoxy-4-fluor-methoxy-phenyl)-6-ethyl-3,6-dihydro-1,3,4-oxadiazin-2-on
3-(4-(Imidazol-4-carbonylamino)-benzyl)-5-(3-difluormethoxy-4-methoxyphenyl)-6-ethyl-3,6-dihydro-1,3,4-oxadiazin-2-on
3-(4-(Imidazol-4-carbonylamino)-benzyl)-5-(3-trifluormethoxy-4-methoxyphenyl)-6-ethyl-3,6-dihydro-1,3,4-oxadiazin-2-on
3-(4-(Imidazol-4-carbonylamino)-benzyl)-5-(3-fluormethoxy-4-methoxy-phenyl)-6-ethyl-3,6-dihydro-1,3,4-oxadiazin-2-on
3-(4-(Imidazol-4-carbonylamino)-benzyl)-5-(3-methoxy-4-ethoxy-phenyl)-6-ethyl-3,6-dihydro-1,3,4-oxadiazin-2-on
3-(4-(Imidazol-4-carbonylamino)-benzyl)-5-(3-ethoxy-4-methoxy-phenyl)-6-ethyl-3,6-dihydro-1,3,4-oxadiazin-2-on
3-(4-(Imidazol-4-carbonylamino)-benzyl)-5-(3-hydroxy-4-methoxy-phenyl)-6-ethyl-3,6-dihydro-1,3,4-oxadiazin-2-on
3-(4-(Imidazol-4-carbonylamino)-benzyl)-5-(4-methylsulfonylphenyl)-6-ethyl-3,6-dihydro-1,3,4-oxadiazin-2-on
3-(4-(Imidazol-4-carbonylamino)-benzyl)-5-(4-methylenoxyphenyl)-6-ethyl-3,6-dihydro-1,3,4-oxadiazin-2-on
3-(4-(Imidazol-4-carbonylamino)-benzyl)-5-(3-cyclopentyloxy-4-methoxy-phenyl)-6-ethyl-3,6-dihydro-1,3,4-oxadiazin-2-on
3-(3-(Imidazol-4-carbonylamino)-benzyl)-5-(3-cyclopentyloxy-4-methoxy-phenyl)-6-ethyl-3,6-dihydro-1,3,4-oxadiazin-2-on
3-(4-(Imidazol-4-carbonylamino)-phenethyl)-5-(3,4-dimethoxyphenyl)-3,6-dihydro-1,3,4-oxadiazin-2-on
3-(4-(Imidazol-4-carbonylamino)-phenethyl)-5-(3,4-dimethoxyphenyl)-6-ethyl-3,6-dihydro-1,3,4-oxadiazin-2-on.

Analog erhält man durch Umsetzung der oben aufgeführten "Aminderivate" mit 2,4-Dimethyl-thiazol-5-carbonsäurechlorid die nachstehenden Verbindungen
2-(4-(2,4-Dimethyl-thiazol-5-carbonylamino)-benzyl)-6-(3,4-dimethoxy-phenyl)-2,3,4,5-tetrahydro-pyridazin-3-on
2-(3-(2,4-Dimethyl-thiazol-5-carbonylamino)-benzyl)-6-(3,4-dimethoxy-phenyl)-2,3,4,5-tetrahydro-pyridazin-3-on
2-(2-(2,4-Dimethyl-thiazol-5-carbonylamino)-benzyl)-6-(3,4-dimethoxy-phenyl)-2,3,4,5-tetrahydro-pyridazin-3-on
2-(4-(2,4-Dimethyl-thiazol-5-carbonylamino)-benzyl)-6-(3,4-dimethoxy-phenyl)-5-ethyl 2,3,4,5-tetrahydro-pyridazin-3-on;
2-(3-(2,4-Dimethyl-thiazol-5-carbonylamino)-benzyl)-6-(3,4-dimethoxy-phenyl)-5-ethyl-2,3,4,5-tetrahydro-pyridazin-3-on
2-(2-(2,4-Dimethyl-thiazol-5-carbonylamino)-benzyl)4-(3,4-dimethoxy-phenyl)-5-ethyl-2,3,4,5-tetrahydro-pyridazin-3-on
2-(4-(2,4-Dimethyl-thiazol-5-carbonylamino)-benzyl)-6-(3-methoxy-4-trifluor-methoxyphenyl)-5-ethyl-2,3,4,5-tetrahydro-pyridazin-3-on
2-(4-(2,4-Dimethyl-thiazol-5-carbonylamino)-benzyl)-6-(3-methoxy-4-difluor-methoxyphenyl)-5-ethyl-2,3,4,5-tetrahydro-pyridazin-3-on
2-(4-(2,4-Dimethyl-thiazol-5-carbonylamino)-benzyl)-6-(3-methoxy-4-fluor-methoxyphenyl)-5-ethyl-2,3,4,5-tetrahydro-pyridazin-3-on
2-(4-(2,4-Dimethyl-thiazol-5-carbonylamino)-benzyl)-6-(3-difluor-methoxy-4-methoxyphenyl)-5-ethyl-2,3,4,5-tetrahydro-pyridazin-3-on
2-(4-(2,4-Dimethyl-thiazol-5-carbonylamino)-benzyl)-6-(3-trifluormethoxy-4-methoxyphenyl)-5-ethyl-2,3,4,5-tetrahydro-pyridazin-3-on
2-(4-(2,4-Dimethyl-thiazol-5-carbonylamino)-benzyl)-6-(3-fluormethoxy-4-methoxyphenyl)-5-ethyl-2,3,4,5-tetrahydro-pyridazin-3-on
2-(4-(2,4-Dimethyl-thiazol-5-carbonylamino)-benzyl)-6-(3-methoxy-4-ethoxy-phenyl)-5-ethyl-2,3,4,5-tetrahydro-pyridazin-3-on
2-(4-(2,4-Dimethyl-thiazol-5-carbonylamino)-benzyl)-6-(3-ethoxy-4-methoxy-phenyl)-5-ethyl-2,3,4,5-tetrahydro-pyridazin-3-on
2-(4-(2,4-Dimethyl-thiazol-5-carbonylamino)-benzyl)-6-(3-hydroxy-4-methoxy-phenyl)-5-ethyl-2,3,4,5-tetrahydro-pyridazin-3-on
2-(4-(2,4-Dimethyl-thiazol-5-carbonylamino)-benzyl)-6-(4-methylsulfonylphenyl)-5-ethyl-2,3,4,5-tetrahydro-pyridazin-3-on
2-(4-(2,4-Dimethyl-thiazol-5-carbonylamino)-benzyl)-6-(4-methylenoxy-phenyl)-5-ethyl-2,3,4,5-tetrahydro-pyridazin-3-on
2-(4-(2,4-Dimethyl-thiazol-5-carbonylamino)-benzyl)-6-(3-cyclopentyloxy-4-methoxyphenyl)-5-ethyl-2,3,4,5-tetrahydro-pyridazin-3-on
2-(3-(2,4-Dimethyl-thiazol-5-carbonylamino)-benzyl)-6-(3-cyclopentyl-oxy-4-methoxyphenyl)-5-ethyl-2,3,4,5-tetrahydro-pyridazin-3-on
2-(4-(2,4-Dimethyl-thiazol-5-carbonylamino)-phenethyl)-6-(3,4-dimethoxyphenyl)-2,3,4,5-tetrahydro-pyridazin-3-on
2-(4-(2,4-Dimethyl-thiazol-5-carbonylamino)-phenethyl)-6-(3,4-dimethoxyphenyl)-5-ethyl-2,3,4,5-tetrahydro-pyridazin-3-on
3-(4-(2,4-Dimethyl-thiazol-5-carbonylamino)-benzyl)-5-(3,4-dimethoxy-phenyl)-3,6-dihydro-1,3,4-thiadiazin-2-on
3-(3-(2,4-Dimethyl-thiazol-5-carbonylamino)-benzyl)-5-(3,4-dimethoxy-phenyl)-3,6-dihydro-1,3,4-thiadiazin-2-on
3-(2-(2,4-Dimethyl-thiazol-5-carbonylamino)-benzyl)-5-(3,4-dimethoxyphenyl)-3,6-dihydro-1,3,4-thiadiazin-2-on
3-(4-(2,4-Dimethyl-thiazol-5-carbonylamino)-benzyl)-5-(3,4-dimethoxy-phenyl)-6-ethyl-3,6-dihydro-1,3,4-thiadiazin-2-on
3-(3-(2,4-Dimethyl-thiazol-5-carbonylamino)-benzyl)-5-(3,4-dimethoxyphenyl)-6-ethyl-3,6-dihydro-1,3,4-thiadiazin-2-on
3-(2-(2,4-Dimethyl-thiazol-5-carbonylamino)-benzyl)-5-(3,4-dimethoxyphenyl)-6-ethyl-3,6-dihydro-1,3,4-thiadiazin-2-on
3-(4-(2,4-Dimethyl-thiazol-5-carbonylamino)-benzyl)-5-(3-methoxy-4-trifluor-methoxyphenyl)-6-ethyl-3,6-dihydro-1,3,4-thiadiazin-2-on
3-(4-(2,4-Dimethyl-thiazol-5-carbonylamino)-benzyl)-5-(3-methoxy-4-difluor-methoxyphenyl)-6-ethyl-3,6-dihydro-1,3,4-thiadiazin-2-on
3-(4-(2,4-Dimethyl-thiazol-5-carbonylamino)-benzyl)-5-(3-methoxy-4-fluor-methoxyphenyl)-6-ethyl-3,6-dihydro-1,3,4-thiadiazin-2-on
3-(4-(2,4-Dimethyl-thiazol-5-carbonylamino)-benzyl)-5-(3-difluor-methoxy-4-methoxyphenyl)-6-ethyl-3,6-dihydro-1,3,4-thiadiazin-2-on
3-(4-(2,4 -Dimethyl-thiazol-5-carbonylamino)-benzyl)-5-(3-trifluormethoxy-4-methoxyphenyl)-6-ethyl-3,6-dihydro-1,3,4-thiadiazin-2-on
3-(4-(2,4-Dimethyl-thiazol-5-carbonylamino)-benzyl)-5-(3-fluormethoxy-4-methoxyphenyl)-6-ethyl-3,6-dihydro-1,3,4-thiadiazin-2-on
3-(4-(2,4-Dimethyl-thiazol-5-carbonylamino)-benzyl)-5-(3-methoxy-4-ethoxy-phenyl)4-ethyl-3,6-dihydro-1,3,4-thiadiazin-2-on
3-(4-(2,4-Dimethyl-thiazol-5-carbonylamino)-benzyl)-5-(3-ethoxy-4-methoxy-phenyl)-6-ethyl-3,6-dihydro-1,3,4-thiadiazin-2-on
3-(4-(2,4-Dimethyl-thiazol-5-carbonylamino)-benzyl)-5-(3-hydroxy-4-methoxy-phenyl)-6-ethyl-3,6-dihydro-1,3,4-thiadiazin-2-on
3-(4-(2,4-Dimethyl-thiazol-5-carbonylamino)-benzyl)-5-(4-methylsulfonylphenyl)-6-ethyl-3,6-dihydro-1,3,4-thiadiazin-2-on
3-(4-(2,4-Dimethyl-thiazol-5-carbonylamino)-benzyl)-5-(4-methylenoxy-phenyl)-6-ethyl-3,6-dihydro-1,3,4-thiadiazin-2-on
3-(4-(2,4-Dimethyl-thiazol-5-carbonylamino)-benzyl)-5-(3-cyclopentyloxy-4-methoxy-phenyl)-6-ethyl-3,6-dihydro-1,3,4-thiadiazin-2-on
3-(3-(2,4-Dimethyl-thiazol-5-carbonylamino)-benzyl)-5-(3-cyclopentyloxy-4-methoxy-phenyl)-6-ethyl-3,6-dihydro-1,3,4-thiadiazin-2-on
3-(4-(2,4-Dimethyl-thiazol-5-carbonylamino)-phenethyl)-5-(3,4-dimethoxyphenyl)-3,6-dihydro-1,3,4-thiadiazin-2-on
3-(4-(2,4-Dimethyl-thiazol-5-carbonylamino)-phenethyl)-5-(3,4-dimethoxyphenyl)-6-ethyl-3,6-dihydro-1,3,4-thiadiazin-2-on
3-(4-(2,4-Dimethyl-thiazol-5-carbonylamino)-benzyl)-5-(3,4-dimethoxyphenyl)-3,6-dihydro-1,3,4-oxadiazin-2-on
3-(3-(Imidazol-4-carbonylamino)-benzyl)-5-(3,4-dimethoxyphenyl)-3,6-dihydro-1,3,4-oxadiazin-2-on
3-(2-(2,4-Dimethyl-thiazol-5-carbonylamino)-benzyl)-5-(3,4-dimethoxyphenyl)-3,6-dihydro-1,3,4-oxadiazin-2-on
3-(4-(2,4-Dimethyl-thiazol-5-carbonylamino)-benzyl)-5-(3,4-dimethoxyphenyl)-6-ethyl-3,6-dihydro-1,3,4-oxadiazin-2-on
3-(3-(2,4-Dimethyl-thiazol-5-carbonylamino)-benzyl)-5-(3,4-dimethoxy-phenyl)-6-ethyl-3,6-dihydro-1,3,4-oxadiazin-2-on
3-(2-(2,4-Dimethyl-thiazol-5-carbonylamino)-benzyl)-5-(3,4-dimethoxyphenyl)4-ethyl-3,6-dihydro-1,3,4-oxadiazin-2-on
3-(4-(2,4-Dimethyl-thiazol-5-carbonylamino)-benzyl)-5-(3-methoxy-4-trifluor-methoxyphenyl)-6-ethyl-3,6-dihydro-1,3,4-oxadiazin-2-on
3-(4-(2,4-Dimethyl-thiazol-5-carbonylamino)-benzyl)-5-(3-methoxy-4-difluor-methoxyphenyl)-6-ethyl-3,6-dihydro-1,3,4-oxadiazin-2-on
3-(4-(2,4-Dimethyl-thiazol-5-carbonylamino)-benzyl)-5-(3-methoxy-4-fluor-methoxyphenyl)-6-ethyl-3,6-dihydro-1,3,4-oxadiazin-2-on
3-(4-(2,4-Dimethyl-thiazol-5-carbonylamino)-benzyl)-5-(3-difluor-methoxy-4-methoxyphenyl)-6-ethyl-3,6-dihydro-1,3,4-oxadiazin-2-on
3-(4-(2,4-Dimethyl-thiazol-5-carbonylamino)-benzyl)-5-(3-trifluor-methoxy-4-methoxyphenyl)-6-ethyl-3,6-dihydro-1,3,4-oxadiazin-2-on
3-(4-(2,4-Dimethyl-thiazol-5-carbonylamino)-benzyl)-5-(3-fluormethoxy-4-methoxyphenyl)-6-ethyl-3,6-dihydro-1,3,4-oxadiazin-2-on
3-(4-(2,4-Dimethyl-thiazol-5-carbonylamino)-benzyl)-5-(3-methoxy-4-ethoxy-phenyl)-6-ethyl-3,6-dihydro-1,3,4-oxadiazin-2-on
3-(4-(2,4-Dimethyl-thiazol-5-carbonylamino)-benzyl)-5-(3-ethoxy-4-methoxy-phenyl)-6-ethyl-3,6-dihydro-1,3,4-oxadiazin-2-on
3-(4-(2,4-Dimethyl-thiazol-5-carbonylamino)-benzyl)-5-(3-hydroxy-4-methoxy-phenyl)-6-ethyl-3,6-dihydro-1,3,4-oxadiazin-2-on
3-(4-(2,4-Dimethyl-thiazol-5-carbonylamino)-benzyl)-5-(4-methylsulfonylphenyl)-6-ethyl-3,6-dihydro-1,3,4-oxadiazin-2-on
3-(4-(2,4-Dimethyl-thiazol-5-carbonylamino)-benzyl)-5-(4-methylenoxy-phenyl)-6-ethyl-3,6-dihydro-1,3,4-oxadiazin-2-on
3-(4-(2,4-Dimethyl-thiazol-5-carbonylamino)-benzyl)-5-(3-cyclopentyl-oxy-4-methoxy-phenyl)-6-ethyl-3,6-dihydro-1,3,4-oxadiazin-2-on
3-(3-(2,4-Dimethyl-thiazol-5-carbonylamino)-benzyl)-5-(3-cyclopentyl-oxy-4-methoxy-phenyl)-6-ethyl-3,6-dihydro-1,3,4-oxadiazin-2-on
3-(4-(2,4-Dimethyl-thiazol-5-carbonylamino)-phenethyl)-5-(3,4-dimethoxyphenyl)-3,6-dihydro-1,3,4-oxadiazin-2-on
3-(4-(2,4-Dimethyl-thiazol-5-carbonylamino)-phenethyl)-5-(3,4-dimethoxyphenyl)-6-ethyl-3,6-dihydro -1,3,4-oxadiazin-2-on.

Analog erhält man durch Umsetzung der oben aufgeführten "Aminderivate" mit lsoxazol Isoxazol-5-carbonsäurechlorid die nachstehenden Verbindungen
2-(4-(Isoxazol-5-carbonylamino)-benzyl)-6-(3,4-dimethoxyphenyl)-2,3,4,5-tetrahydro-pyridazin-3-on, F. 217°
2-(3-(Isoxazol-5-carbonylamino)-benzyl)-6-(3,4-dimethoxyphenyl)-2,3,4,5-tetrahydro-pyridazin-3-on
2-(2-(Isoxazol-5-carbonylamino)-benzyl)-6-(3,4-dimethoxyphenyl)-2,3,4,5-tetrahydro-pyridazin-3-on
2-(4-(lsoxazol-5-carbonylamino)-benzyl)-6-(3,4-dimethoxyphenyl)-5-ethyl-2,3,4,5-tetrahydro-pyridazin-3-on;
2-(3-(lsoxazol-5-carbonylamino)-benzyl)-6-(3,4-dimethoxyphenyl)-5-ethyl-2,3,4,5-tetrahydro-pyridazin-3-on
2-(2-(Isoxazol-5-carbonylamino)-benzyl)-6-(3,4-dimethoxyphenyl)-5-ethyl-2,3,4,5-tetrahydro-pyridazin-3-on
2-(4-(lsoxazol-5-carbonylamino)-benzyl)-6-(3-methoxy-4-trifluor-methoxyphenyl)-5-ethyl-2,3,4,5-tetrahydro-pyridazin-3-on
2-(4-(Isoxazol-5-carbonylamino)-benzyl)-6-(3-methoxy-4-difluormethoxyphenyl)-5-ethyl-2,3,4,5-tetrahydro-pyridazin-3-on
2-(4-(Isoxazol-5-carbonylamino)-benzyl)-6-(3-methoxy-4-fluor-methoxy-phenyl)-5-ethyl-2,3,4,5-tetrahydro-pyridazin-3-on
2-(4-(Isoxazol-5-carbonylamino)-benzyl)-6-(3-difluormethoxy-4-methoxyphenyl)-5-ethyl-2,3,4,5-tetrahydro-pyridazin-3-on
2-(4-(Isoxazol-5-carbonylamino)-benzyl)-6-(3-trifluormethoxy-4-methoxyphenyl)-5-ethyl-2,3,4,5-tetrahydro-pyridazin-3-on
2-(4-(Isoxazol-5-carbonylamino)-benzyl)-6-(3-fluormethoxy-4-methoxyphenyl)-5-ethyl-2,3,4,5-tetrahydro-pyridazin-3-on
2-(4-(Isoxazol-5-carbonylamino)-benzyl)-6-(3-methoxy-4-ethoxyphenyl)-5-ethyl-2,3,4,5-tetrahydro-pyridazin-3-on
2-(4-(Isoxazol-5-carbonylamino)-benzyl)-6-(3-ethoxy-4-methoxy-phenyl)-5-ethyl-2,3,4,5-tetrahydro-pyridazin-3-on
2-(4-(Isoxazol-5-carbonylamino)-benzyl)-6-(3-hydroxy-4-methoxy-phenyl)-5-ethyl-2,3,4,5-tetrahydro-pyridazin-3-on
2-(4-(lsoxazol-5-carbonylamino)-benzyl)-6-(4-methylsulfonylphenyl)-5-ethyl-2,3,4,5-tetrahydro-pyridazin-3-on
2-(4-(Isoxazol-5-carbonylamino)-benzyl)-6-(4-methylenoxyphenyl)-5-ethyl-2,3,4,5-tetrahydro-pyridazin-3-on
2-(4-(Isoxazol-5-carbonylamino)-benzyl)-6-(3-cyclopentyloxy-4-methoxyphenyl)-5-ethyl-2,3,4,5-tetrahydro-pyridazin-3-on
2-(3-(lsoxazol-5-carbonylamino)-benzyl)-6-(3-cyclopentyloxy-4-methoxyphenyl)-5-ethyl-2,3,4,5-tetrahydro-pyridazin-3-on
2-(4-(lsoxazol-5-carbonylamino)-phenethyl)-6-(3,4-dimethoxyphenyl)-2,3,4,5-tetrahydro-pyridazin-3-on
2-(4-(Isoxazol-5-carbonylamino)-phenethyl)-6-(3,4-dimethoxyphenyl)-5-ethyl-2,3,4,5-tetrahydro-pyridazin-3-on
3-(4-(Isoxazol-5-carbonylamino)-benzyl)-5-(3,4-dimethoxyphenyl)-3,6-dihydro-1,3,4-thiadiazin-2-on, F. 196°
3-(3-(Isoxazol-5-carbonylamino)-benzyl)-5-(3,4-dimethoxyphenyl)-3,6-dihydro-1,3,4-thiadiazin-2-on
3-(2-(Isoxazol-5-carbonylamino)-benzyl)-5-(3,4-dimethoxyphenyl)-3,6-dihydro-1,3,4-thiadiazin-2-on
3-(4-(Isoxazol-5-carbonylamino)-benzyl)-5-(3,4-dimethoxyphenyl)-6-ethyl-3,6-dihydro-1,3,4-thiadiazin-2-on
3-(3-(Isoxazol-5-carbonylamino)-benzyl)-5-(3,4-dimethoxyphenyl)-6-ethyl-3,6-dihydro-1,3,4-thiadiazin-2-on
3-(2-(Isoxazol-5-carbonylamino)-benzyl)-5-(3,4-dimethoxyphenyl)-6-ethyl-3,6-dihydro-1,3,4-thiadiazin-2-on
3-(4-(Isoxazol-5-carbonylamino)-benzyl)-5-(3-methoxy-4-trifluor-methoxyphenyl)-6-ethyl-3,6-dihydro-1,3,4-thiadiazin-2-on
3-(4-(Isoxazol-5-carbonylamino)-benzyl)-5-(3-methoxy-4-difluormethoxyphenyl)-6-ethyl-3,8-dihydro-1,3,4-thiadiazin-2-on
3-(4-(Isoxazol-5-carbonylamino)-benzyl)-5-(3-methoxy-4-fluor-methoxyphenyl)-6-ethyl-3,6-dihydro-1,3,4-thiadiazin-2-on
3-(4-(Isoxazol-5-carbonylamino)-benzyl)-5-(3-difluormethoxy-4-methoxyphenyl)-6-ethyl-3,6-dihydro-1,3,4-thiadiazin-2-on
3-(4-(Isoxazol-5-carbonylamino)-benzyl)-5-(3-trifluormethoxy-4-methoxyphenyl)-6-ethyl-3,6-dihydro-1,3,4-thiadiazin-2-on
3-(4-(Isoxazol-5-carbonylamino)-benzyl)-5-(3-fluormethoxy-4-methoxyphenyl)-6-ethyl-3,6-dihydro-1,3,4-thiadiazin-2-on
3-(4-(lsoxazol-5-carbonylamino)-benzyl)-5-(3-methoxy-4-ethoxyphenyl)-6-ethyl-3,6-dihydro-1,3,4-thiadiazin-2-on
3-(4-(Isoxazol-5-carbonylamino)-benzyl)-5-(3-ethoxy-4-methoxy-phenyl)-6-ethyl-3,6-dihydro-1,3,4-thiadiazin-2-on
3-(4-(Isoxazol-5-carbonylamino)-benzyl)-5-(3-hydroxy-4-methoxyphenyl)-6-ethyl-3,6-dihydro-1,3,4-thiadiazin-2-on
3-(4-(lsoxazol-5-carbonylamino)-benzyl)-5-(4-methylsulfonylphenyl)-6-ethyl-3,6-dihydro-1,3,4-thiadiazin-2-on
3-(4-(Isoxazol-5-carbonylamino)-benzyl)-5-(4-methylenoxyphenyl)-6-ethyl-3,6-dihydro-1,3,4-thiadiazin-2-on
3-(4-(Isoxazol-5-carbonylamino)-benzyl)-5-(3-cyclopentyloxy-4-methoxy-phenyl)-6-ethyl-3,6-dihydro-1,3,4-thiadiazin-2-on
3-(3-(Isoxazol-5-carbonylamino)-benzyl)-5-(3-cyclopentyloxy-4-methoxy-phenyl)-6-ethyl-3,6-dihydro-1,3,4-thiadiazin-2-on
3-(4-(lsoxazol-5-carbonylamino)-phenethyl)-5-(3,4-dimethoxyphenyl)-3,6-dihydro-1,3,4-thiadiazin-2-on
3-(4-(Isoxazol-5-carbonylamino)-phenethyl)-5-(3,4-dimethoxyphenyl)-6-ethyl-3,6-dihydro-1,3,4-thiadiazin-2-on
3-(4-(lsoxazol-5-carbonylamino)-benzyl)-5-(3,4-dimethoxyphenyl)-3,6-dihydro-1,3,4-oxadiazin-2-on
3-(3-(Isoxazol-5-carbonylamino)-benzyl)-5-(3,4-dimethoxyphenyl)-3,6-dihydro-1,3,4-oxadiazin-2-on
3-(2-(Isoxazol-5-carbonylamino)-benzyl)-5-(3,4-dimethoxyphenyl)-3,6-dihydro-1,3,4-oxadiazin-2-on
3-(4-(Isoxazol-5-carbonylamino)-benzyl)-5-(3,4-dimethoxyphenyl)-6-ethyl-3,6-dihydro-1,3,4-oxadiazin-2-on
3-(3-(Isoxazol-5-carbonylamino)-benzyl)-5-(3,4-dimethoxyphenyl)-6-ethyl-3,6-dihydro-1,3,4-oxadiazin-2-on
3-(2-(lsoxazol-5-carbonylamino)-benzyl)-5-(3,4-dimethoxyphenyl)-6-ethyl-3,6-dihydro-1,3,4-oxadiazin-2-on
3-(4-(Isoxazol-5-carbonylamino)-benzyl)-5-(3-methoxy-4-trifluormethoxyphenyl)-6-ethyl-3,6-dihydro-1,3,4-oxadiazin-2-on
3-(4-(Isoxazol-5-carbonylamino)-benzyl)-5-(3-methoxy-4-difluormethoxyphenyl)-6-ethyl-3,6-dihydro-1,3,4-oxadiazin-2-on
3-(4-(lsoxazol-5-carbonylamino)-benzyl)-5-(3-methoxy-4-fluor-methoxyphenyl)-6-ethyl-3,6-dihydro-1,3,4-oxadiazin-2-on
3-(4-(Isoxazol-5-carbonylamino)-benzyl)-5-(3-difluormethoxy-4-methoxyphenyl)-6-ethyl-3,6-dihydro-1,3,4-oxadiazin-2-on
3-(4-(Isoxazol-5-carbonylamino)-benzyl)-5-(3-trifluormethoxy-4-methoxyphenyl)-6-ethyl-3,6-dihydro-1,3,4-oxadiazin-2-on
3-(4-(lsoxazol-5-carbonylamino)-benzyl)-5-(3-fluormethoxy-4-methoxyphenyl)-6-ethyl-3,6-dihydro-1,3,4-oxadiazin-2-on
3-(4-(lsoxazol-5-carbonylamino)-benzyl)-5-(3-methoxy-4-ethoxyphenyl)-6-ethyl-3,6-dihydro-1,3,4-oxadiazin-2-on
3-(4-(Isoxazol-5-carbonylamino)-benzyl)-5-(3-ethoxy-4-methoxyphenyl)-6-ethyl-3,6-dihydro-1,3,4-oxadiazin-2-on
3-(4-(Isoxazol-5-carbonylamino)-benzyl)-5-(3-hydroxy-4-methoxyphenyl)-6-ethyl-3,6-dihydro-1,3,4-oxadiazin-2-on
3-(4-(lsoxazol-5-carbonylamino)-benzyl)-5-(4-methylsulfonylphenyl)-6-ethyl-3,6-dihydro-1,3,4-oxadiazin-2-on
3-(4-(lsoxazol-5-carbonylamino)-benzyl)-5-(4-methylenoxyphenyl)-6-ethyl-3,6-dihydro-1,3,4-oxadiazin-2-on
3-(4-(Isoxazol-5-carbonylamino)-benzyl)-5-(3-cyclopentyloxy-4-methoxy-phenyl)-6-ethyl-3,6-dihydro-1,3,4-oxadiazin-2-on
3-(3-(Isoxazol-5-carbonylamino)-benzyl)-5-(3-cyclopentyloxy-4-methoxy-phenyl)-6-ethyl-3,6-dihydro-1,3,4-oxadiazin-2-on
3-(4-(Isoxazol-5-carbonylamino)-phenethyl)-5-(3,4-dimethoxyphenyl)-3,6-dihydro-1,3,4-oxadiazin-2-on
3-(4-(Isoxazol-5-carbonylamino)-phenethyl)-5-(3,4-dimethoxyphenyl)4-ethyl-3,6-dihydro-1,3,4-oxadiazin-2-on.

Analog erhält man durch Umsetzung der oben aufgeführten "Aminderivate" mit Oxazol-5-carbonsäurechlorid die nachstehenden Verbindungen
2-(4-(Oxazol-5-carbonylamino)-benzyl)-6-(3,4-dimethoxyphenyl)-2,3,4,5-tetrahydro-pyridazin-3-on
2-(3-(Oxazol-5-carbonylamino)-benzyl)-6-(3,4-dimethoxyphenyl)-2,3,4,5-tetrahydro-pyridazin-3-on
2-(2-(Oxazol-5-carbonylamino)-benzyl)-6-(3,4-dimethoxyphenyl)-2,3,4,5-tetrahydro-pyridazin-3-on
2-(4-(Oxazol-5-carbonylamino)-benzyl)-6-(3,4-dimethoxyphenyl)-5-ethyl-2,3,4,5-tetrahydro-pyridazin-3-on;
2-(3-(Oxazol-5-carbonylamino)-benzyl)-6-(3,4-dimethoxyphenyl)-5-ethyl-2,3,4,5-tetrahydro-pyridazin-3-on
2-(2-(Oxazol-5-carbonylamino)-benzyl)-6-(3,4-dimethoxyphenyl)-5-ethyl-2,3,4,5-tetrahydro-pyridazin-3-on
2-(4-(Oxazol-5-carbonylamino)-benzyl)-6-(3-methoxy-4-trifluor-methoxyphenyl)-5-ethyl-2,3,4,5-tetrahydro-pyridazin-3-on
2-(4-(Oxazol-5-carbonylamino)-benzyl)-6-(3-methoxy-4-difluor-methoxyphenyl)-5-ethyl-2,3,4,5-tetrahydro-pyridazin-3-on
2-(4-(Oxazol-5-carbonylamino)-benzyl-6-(3-methoxy-4 fluor-methoxyphenyl)-5-ethyl-2,3,4,5-tetrahydro-pyridazin-3-on
2-(4-(Oxazol-5-carbonylamino)-benzyl)-6-(3-difluormethoxy-4-methoxyphenyl)-5-ethyl-2,3,4,5-tetrahydro-pyridazin-3-on
2-(4-(Oxazol-5-carbonylamino)-benzyl)-6-(3-trifluormethoxy-4-methoxyphenyl)-5-ethyl-2,3,4,5-tetrahydro-pyridazin-3-on
2-(4-(Oxazol-5-carbonylamino)-benzyl)-6-(3-fluormethoxy-4-methoxyphenyl)-5-ethyl-2,3,4,5-tetrahydro-pyridazin-3-on
2-(4-(Oxazol-5-carbonylamino)-benzyl)-6-(3-methoxy-4-ethoxy-phenyl)-5-ethyl-2,3,4,5-tetrahydro-pyridazin-3-on
2-(4-(Oxazol-5-carbonylamino)-benzyl)-6-(3-ethoxy-4-methoxy-phenyl)-5-ethyl-2,3,4,5-tetrahydro-pyridazin-3-on
2-(4-(Oxazol-5-carbonylamino)-benzyl)-6-(3-hydroxy-4-methoxy-phenyl)-5-ethyl-2,3,4,5-tetrahydro-pyridazin-3-on
2-(4-(Oxazol-5-carbonylamino)-benzyl)-6-(4-methylsulfonylphenyl)-5-ethyl-2,3,4,5-tetrahydro-pyridazin-3-on
2-(4-(Oxazol-5-carbonylamino)-benzyl)-6-(4-methylenoxyphenyl)-5-ethyl-2,3,4,5-tetrahydro-pyridazin-3-on
2-(4-(Oxazol-5-carbonylamino)-benzyl)-6-(3-cyclopentyloxy-4-methoxy-phenyl)-5-ethyl-2,3,4,5-tetrahydro-pyridazin-3-on
2-(3-(Oxazol-5-carbonylamino)-benzyl)-6-(3-cyclopentyloxy-4-methoxyphenyl)-5-ethyl-2,3,4,5-tetrahydro-pyridazin-3-on
2-(4-(Oxazol-5-carbonylamino)-phenethyl)-6-(3,4-dimethoxyphenyl)-2,3,4,5-tetrahydro-pyridazin-3-on
2-(4-(Oxazol-5-carbonylamino)-phenethyl)-6-(3,4-dimethoxyphenyl)-5-ethyl-2,3,4,5-tetrahydro-pyridazin-3-on
3-(4-(Oxazol-5-carbonylamino)-benzyl)-5-(3,4-dimethoxyphenyl)-3,6-dihydro-1,3,4-thiadiazin-2-on
3-3-(Oxazol-5-carbonylamino)-benzyl)-5-(3,4-dimethoxyphenyl)-3,6-dihydro-1,3,4-thiadiazin-2-on
3-(2-(Oxazol-5-carbonylamino)-benzyl)-5-(3,4-dimethoxyphenyl)-3,6-dihydro-1,3,4-thiadiazin-2-on
3-(4-(Oxazol-5-carbonylamino)-benzyl)-5-(3,4-dimethoxyphenyl)-6-ethyl-3,6-dihydro-1,3,4-thiadiazin-2-on
3-(3-(Oxazol-5-carbonylamino)-benzyl)-5-(3,4-dimethoxyphenyl)-6-ethyl-3,6-dihydro-1,3,4-thiadiazin-2-on
3-(2-(Oxazol-5-carbonylamino)-benzyl)-5-(3,4-dimethoxyphenyl)-6-ethyl-3,6-dihydro-1,3,4-thiadiazin-2-on
3-(4-(Oxazol-5-carbonylamino)-benzyl)-5-(3-methoxy-4-trifluor-methoxyphenyl)-6-ethyl-3,6-dihydro-1,3,4-thiadiazin-2-on
3-(4-(Oxazol-5-carbonylamino)-benzyl)-5-(3-methoxy-4-difluor-methoxyphenyl)-6-ethyl-3,6-dihydro-1,3,4-thiadiazin-2-on
3-(4-(Oxazol-5-carbonylamino)-benzyl)-5-(3-methoxy-4-fluor-methoxyphenyl)-6-ethyl-3,6-dihydro-1,3,4-thiadiazin-2-on
3-(4-(Oxazol-5-carbonylamino)-benzyl)-5-(3-difluormethoxy-4-methoxyphenyl)-6-ethyl-3,6-dihydro-1,3,4-thiadiazin-2-on
3-(4-(Oxazol-5-carbonylamino)-benzyl)-5-(3-trifluormethoxy-4-methoxyphenyl)-6-ethyl-3,6-dihydro-1,3,4-thiadiazin-2-on
3-(4-(Oxazol-5-carbonylamino)-benzyl)-5-(3-fluormethoxy-4-methoxyphenyl)-6-ethyl-3,6-dihydro-1,3,4-thiadiazin-2-on
3-(4-(Oxazol-5-carbonylamino)-benzyl)-5-(3-methoxy-4-ethoxy-phenyl)-6-ethyl-3,6-dihydro-1,3,4-thiadiazin-2-on
3-(4-(Oxazol-5-carbonylamino)-benzyl)-5-(3-ethoxy-4-methoxy-phenyl)-6-ethyl-3,6-dihydro-1,3,4-thiadiazin-2-on
3-(4-(Oxazol-5-carbonylamino)-benzyl)-5-(3-hydroxy-4-methoxyphenyl)-6-ethyl-3,6-dihydro-1,3,4 thiadiazin-2-on
3-(4-(Oxazol-5-carbonylamino)-benzyl)-5-(4-methylsulfonylphenyl)-6-ethyl-3,6-dihydro-1,3,4-thiadiazin-2-on
3-(4-(Oxazol-5-carbonylamino)-benzyl)-5-(4-methylenoxyphenyl)-6-ethyl-3,6-dihydro-1,3,4-thiadiazin-2-on
3-(4-(Oxazol-5-carbonylamino)-benzyl) 5-(3-cyclopentyloxy-4-methoxyphenyl)-6-ethyl-3,6-dihydro-1,3,4-thiadiazin-2-on
3-(3-(Oxazol-5-carbonylamino)-benzyl)-5-(3-cyclopentyloxy-4-methoxyphenyl)-6-ethyl-3,6-dihydro-1,3,4-thiadiazin-2-on
3-(4-(Oxazol-5-carbonylamino)-phenethyl)-5-(3,4-dimethoxyphenyl)-3,6-dihydro-1,3,4-thiadiazin-2-on
3-(4-(Oxazol-5-carbonylamino)-phenethyl)-5-(3,4-dimethoxyphenyl)-6-ethyl-3,6-dihydro-1,3,4-thiadiazin-2-on
3-(4-(Oxazol-5-carbonylamino)-benzyl)-5-(3,4-dimethoxyphenyl)-3,6-dihydro-1,3,4-oxadiazin-2-on
3-(3-(Oxazol-5-carbonylamino)-benzyl)-5-(3,4-dimethoxyphenyl)-3,6-dihydro-1,3,4-oxadiazin-2-on
3-(2-(Oxazol-5-carbonylamino)-benzyl)-5-(3,4-dimethoxyphenyl)-3,6-dihydro-1,3,4-oxadiazin-2-on
3-(4-(Oxazol-5-carbonylamino)-benzyl)-5-(3,4-dimethoxyphenyl)-6-ethyl-3,6-dihydro-1,3,4-oxadiazin-2-on
3-(3-(Oxazol-5-carbonylamino)-benzyl)-5-(3,4-dimethoxyphenyl)-6-ethyl-3,6-dihydro-1,3,4-oxadiazin-2-on
3-(2-(Oxazol-5-carbonylamino)-benzyl)-5-(3,4-dimethoxyphenyl)-6-ethyl-3,6-dihydro-1,3,4-oxadiazin-2-on
3-(4-(Oxazol-5-carbonylamino)-benzyl)-5-(3-methoxy-4-trifluor-methoxy-phenyl)-6-ethyl-3,6-dihydro-1,3,4-oxadiazin-2-on
3-(4-(Oxazol-5-carbonylamino)-benzyl)-5-(3-methoxy-4-difluor-methoxyphenyl)-6-ethyl-3,6-dihydro-1,3,4-oxadiazin-2-on
3-(4-(Oxazol-5-carbonylamino)-benzyl)-5-(3-methoxy-4-fluor-methoxyphenyl)-6-ethyl-3,6-dihydro-1,3,4-oxadiazin-2-on
3-(4-(Oxazol-5-carbonylamino)-benzyl)-5-(3-difluormethoxy-4-methoxyphenyl)-6-ethyl-3,6-dihydro-1,3,4-oxadiazin-2-on
3-(4-(Oxazol-5-carbonylamino)-benzyl)-5-(3-trifluormethoxy-4-methoxyphenyl)-6-ethyl-3,6-dihydro-1,3,4-oxadiazin-2-on
3-(4-(Oxazol-5-carbonylamino)-benzyl)-5-(3-fluormethoxy-4-methoxy-phenyl)-6-ethyl-3,6-dihydro-1,3,4-oxadiazin-2-on
3-(4-(Oxazol-5-carbonylamino)-benzyl)-5-(3-methoxy-4-ethoxy-phenyl)-6-ethyl-3,6-dihydro-1,3,4-oxadiazin-2-on
3-(4-(Oxazol-5-carbonylamino)-benzyl)-5-(3-ethoxy-4-methoxy-phenyl)-6-ethyl-3,6-dihydro-1,3,4-oxadiazin-2-on
3-(4-(Oxazol-5-carbonylamino)-benzyl)-5-(3-hydroxy-4-methoxy-phenyl)-6-ethyl-3,6-dihydro-1,3,4-oxadiazin-2-on
3-(4-(Oxazol-5-carbonylamino)-benzyl)-5-(4-methylsulfonylphenyl)-6-ethyl-3,6-dihydro-1,3,4-oxadiazin-2-on
3-(4-(Oxazol-5-carbonylamino)-benzyl)-5-(4-methylenoxyphenyl)-6-ethyl-3,6-dihydro-1,3,4-oxadiazin-2-on
3-(4-(Oxazol-5-carbonylamino)-benzyl)-5-(3-cyclopentyloxy-4-methoxy-phenyl)-6-ethyl-3,6-dihydro-1,3,4-oxadiazin-2-on
3-(3-(Oxazol-5-carbonylamino)-benzyl)-5-(3-cyclopentyloxy-4-methoxy-phenyl)-6-ethyl-3,6-dihydro -1,3,4-oxadiazin-2-on
3-(4-(Oxazol-5-carbonylamino)-phenethyl)-5-(3,4-dimethoxyphenyl)-3,6-dihydro-1,3,4-oxadiazin-2-on
3-(4-(Oxazol-5-carbonylamino)-phenethyl)-5-(3,4-dimethoxyphenyl)-6-ethyl-3,6-dihydro-1,3,4-oxadiazin-2-on.

Analog erhält man durch Umsetzung der oben aufgeführten "Aminderivate" mit Pyrimidin-2-carbonsäurechlorid die nachstehenden Verbindungen
2-(4-Pyrimidin-2-carbonylamino)-benzyl)-6-(3,4-dimethoxyphenyl)-2,3,4,5-tetrahydro-pyridazin-3-on
2-(3-(Pyrimidin-2-carbonylamino)-benzyl)-6-(3,4-dimethoxyphenyl)-2,3,4,5-tetrahydro-pyridazin-3-on
2-(2-Pyrimidin-2-carbonylamino)-benzyl)-6-(3,4-dimethoxyphenyl)-2,3,4,5-tetrahydro-pyridazin-3-on
2-(4-(Pyrimidin-2-carbonylamino)-benzyl)-6-(3,4-dimethoxyphenyl)-5-ethyl-2,3,4,5-tetrahydro-pyridazin-3-on;
2-(3-(Pyrimidin-2-carbonylamino)-benzyl)-6-(3,4-dimethoxyphenyl)-5-ethyl-2,3,4,5-tetrahydro-pyridazin-3-on
2-(2-(Pyrimidin-2-carbonylamino)-benzyl)-6-(3,4-dimethoxyphenyl)-5-ethyl-2,3,4,5-tetrahydro-pyridazin-3-on
2-(4-(Pyrimidin-2-carbonylamino)-benzyl)-6-(3-methoxy-4-trifluor-methoxyphenyl)-5-ethyl-2,3,4,5-tetrahydro-pyridazin-3-on
2-(4-(Pyrimidin-2-carbonylamino)-benzyl)-6-(3-methoxy-4-difluor-methoxyphenyl)-5-ethyl-2,3,4,5-tetrahydro-pyridazin-3-on
2-(4-(Pyrimidin-2-carbonylamino)-benzyl)-6-(3-methoxy-4-fluormethoxyphenyl)-5-ethyl-2,3,4,5-tetrahydro-pyridazin-3-on
2-(4-(Pyrimidin-2-carbonylamino)-benzyl)-6-(3-difluormethoxy-4-methoxyphenyl)-5-ethyl-2,3,4,5-tetrahydro-pyridazin-3-on
2-(4-(Pyrimidin-2-carbonylamino)-benzyl)-6-(3-trifluormethoxy-4-methoxyphenyl)-5-ethyl-2,3,4,5-tetrahydro-pyridazin-3-on
2-(4-(Pyrimidin-2-carbonylamino)-benzyl)-6-(3-fluormethoxy-4-methoxy-phenyl)-5-ethyl-2,3,4,5-tetrahydro-pyridazin-3-on
2-(4-(Pyrimidin-2-carbonylamino)-benzyl)-6-(3-methoxy-4-ethoxy-phenyl)-5-ethyl-2,3,4,5-tetrahydro-pyridazin-3-on
2-(4-(Pyrimidin-2-carbonylamino)-benzyl)-6-(3-ethoxy-4-methoxy-phenyl)-5-ethyl-2,3,4,5-tetrahydro-pyridazin-3-on
2-(4-(Pyrimidin-2-carbonylamino)-benzyl)-6-(3-hydroxy-4-methoxy-phenyl)-5-ethyl-2,3,4,5-tetrahydro-pyridazin-3-on
2-(4-(Pyrimidin2-carbonylamino)-benzyl-6-(4-methylsulfonylphenyl)-5-ethyl-2,3,4,5-tetrahydro-pyridazin-3-on
2-(4-(Pyrimidin-2-carbonylamino)-benzyl)-6-(4-methylenoxyphenyl)-5-ethyl-2,3,4,5-tetrahydro-pyridazin-3-on
2-(4-(Pyrimidin-2-carbonylamino)-benzyl)-6-(3-cyclopentyloxy-4-methoxyphenyl)-5-ethyl-2,3,4,5-tetrahydro-pyridazin-3-on
2-(3-(Pyrimidin-2-carbonylamino)-benzyl)-6-(3-cyclopentyloxy-4-methoxyphenyl)-5-ethyl-2,3,4,5-tetrahydro-pyridazin-3-on
2-(4-(Pyrimidin-2-carbonylamino)-phenethyl)-6-(3,4-dimethoxyphenyl)-2,3,4,5-tetrahydro-pyridazin-3-on
2-(4-(Pyrimidin-2-carbonylamino)-phenethyl)-6-(3,4-dimethoxyphenyl)-5-ethyl-2,3,4,5-tetrahydro-pyridazin-3-on
3-(4-(Pyrimidin-2-carbonylamino)-benzyl)-5-(3,4-dimethoxyphenyl)-3,6-dihydro-1,3,4-thiadiazin-2-on
3-(3-(Pyrimidin-2-carbonylamino)-benzyl)-5-(3,4-dimethoxyphenyl)-3,6-dihydro-1,3,4-thiadiazin-2-on
3-(2-(Pyrimidin-2-carbonylamino)-benzyl)-5-(3,4-dimethoxyphenyl)-3,6-dihydro-1,3,4-thiadiazin-2-on
3-(4-(Pyrimidin-2-carbonylamino)-benzyl)-5-(3,4-dimethoxyphenyl)-6-ethyl-3,6-dihydro-1,3,4-thiadiazin-2-on
3-(3-(Pyrimidin-2-carbonylamino)-benzyl)-5-(3,4-dimethoxyphenyl)-6-ethyl-3,6-dihydro-1,3,4-thiadiazin-2-on
3-(2-(Pyrimidin-2-carbonylamino)-benzyl)-5-(3,4-dimethoxyphenyl)-6-ethyl-3,6-dihydro-1,3,4-thiadiazin-2-on
3-(4-(Pyrimidin-2-carbonylamino)-benzyl)-5-(3-methoxy-4-trifluor-methoxyphenyl)-6-ethyl-3,6-dihydro-1,3,4-thiadiazin-2-on
3-(4-(Pyrimidin-2-carbonylamino)-benzyl)-5-(3-methoxy-4-difluor-methoxyphenyl)-6-ethyl-3,6-dihydro-1,3,4-thiadiazin-2-on
3-(4-(Pyrimidin-2-carbonylamino)-benzyl)-5-(3-methoxy-4-fluor-methoxyphenyl)-6-ethyl-3,6-dihydro-1,3,4-thiadiazin-2-on
3-(4-(Pyrimidin-2-carbonylamino)-benzyl)-5-(3-difluormethoxy-4-methoxyphenyl)-6-ethyl-3,6-dihydro-1,3,4-thiadiazin-2-on
3-(4-(Pyrimidin-2-carbonylamino)-benzyl)-5-(3-trifluormethoxy-4-methoxyphenyl)-6-ethyl-3,6-dihydro-1,3,4-thiadiazin-2-on
3-(4-(Pyrimidin-2-carbonylamino)-benzyl)-5-(3-fluormethoxy-4-methoxy-phenyl)-6-ethyl-3,6-dihydro-1,3,4-thiadiazin-2-on
3-(4-(Pyrimidin-2-carbonylamino)-benzyl)-5-(3-methoxy-4-ethoxy-phenyl)-6-ethyl-3,6-dihydro-1,3,4-thiadiazin-2-on
3-(4-(Pyrimidin-2-carbonylamino)-benzyl)-5-(3-ethoxy-4-methoxy-phenyl)-6-ethyl-3,6-dihydro-1,3,4-thiadiazin-2-on
3-(4-(Pyrimidin-2-carbonylamino)-benzyl)-5-(3-hydroxy-4-methoxy-phenyl)-6-ethyl-3,6-dihydro-1,3,4-thiadiazin-2-on
3-(4-(Pyrimidin-2-carbonylamino)-benzyl)-5-(4-methylsulfonylphenyl)-6-ethyl-3,6-dihydro-1,3,4-thiadiazin-2-on
3-(4-(Pyrimidin-2-carbonylamino)-benzyl)-5-(4-methylenoxyphenyl)-6-ethyl-3,6-dihydro-1,3,4-thiadiazin-2-on
3-(4-(Pyrimidin-2-carbonylamino)-benzyl)-5-(3-clopentyloxy-4-methoxy-phenyl)-6-ethyl-3,6-dihydro-1,3,4-thiadiazin-2-on
3-(3-(Pyrimidin-2-carbonylamino)-benzyl)-5-(3-cyclopentyloxy-4-methoxy-phenyl)-6-ethyl-3,6-dihydro-1,3,4-thiadiazin-2-on
3-(4-(Pyrimidin-2-carbonylamino)-phenethyl)-5-(3,4-dimethoxyphenyl)-3,6-dihydro-1,3,4-thiadiazin-2-on
3-(4-(Pyrimidin-2-carbonylamino)-phenethyl)-5-(3,4-dimethoxyphenyl)-6-ethyl-3,6-dihydro-1,3,4-thiadiazin-2-on
3-(4-(Pyrimidin-2-carbonylamino)-benzyl)-5-(3,4-dimethoxyphenyl)-3,6-dihydro-1,3,4-oxadiazin-2-on
3-(3-(Pyrimidin-2-carbonylamino)-benzyl)-5-(3,4-dimethoxyphenyl)-3,6-dihydro-1,3,4-oxadiazin-2-on
3-(2-(Pyrimidin-2-carbonylamino)-benzyl)-5-(3,4-dimethoxyphenyl)-3,6-dihydro-1,3,4-oxadiazin-2-on
3-(4-(Pyrimidin-2-carbonylamino)-benzyl)-5-(3,4-dimethoxyphenyl)-6-ethyl-3,6-dihydro-1,3,4-oxadiazin-2-on
3-(3-(Pyrimidin-2-carbonylamino)-benzyl)-5-(3,4-dimethoxyphenyl)-6-ethyl-3,6-dihydro-1,3,4-oxadiazin-2-on
3-(2-(Pyrimidin-2-carbonylamino)-benzyl)-5-(3,4-dimethoxyphenyl)-6-ethyl-3,6-dihydro-1,3,4-oxadiazin-2-on
3-(4-(Pyrimidin-2-carbonylamino)-benzyl)-5-(3-methoxy-4-trifluormethoxyphenyl)-6-ethyl-3,6-dihydro-1,3,4-oxadiazin-2-on
3-(4-(Pyrimidin-2-carbonylamino)-benzyl)-5-(3-methoxy-4-difluor-methoxyphenyl)-6-ethyl-3,6-dihydro-1,3,4-oxadiazin-2-on
3-(4-(Pyrimidin-2-carbonylamino)-benzyl)-5-(3-methoxy-4-fluor-methoxyphenyl)-6-ethyl-3,6-dihydro-1,3,4-oxadiazin-2-on
3-(4-(Pyrimidin-2-carbonylamino)-benzyl)-5-(3-difluormethoxy-4-methoxyphenyl)4-ethyl-3,6-dihydro-1,3,4-oxadiazin-2-on
3-(4-(Pyrimidin-2-carbonylamino)-benzyl)-5-(3-trifluormethoxy-4-methoxyphenyl)-6-ethyl-3,6-dihydro-1,3,4-oxadiazin-2-on
3-(4-(Pyrimidin-2-carbonylamino)-benzyl)-5-(3-fluormethoxy-4-methoxy-phenyl)-6-ethyl-3,6-dihydro-1,3,4-oxadiazin-2-on
3-(4-(Pyrimidin-2-carbonylamino)-benzyl)-5-(3-methoxy-4-ethoxy-phenyl)-6-ethyl-3,6-dihydro-1,3,4-oxadiazin-2-on
3-(4-(Pyrimidin-2-carbonylamino)-benzyl)-5-(3-ethoxy-4-methoxy-phenyl)-6-ethyl-3,6-dihydro-1,3,4-oxadiazin-2-on
3-(4-(Pyrimidin-2-carbonylamino)-benzyl)-5-(3-hydroxy-4-methoxyphenyl)-6-ethyl-3,6-dihydro-1,3,4-oxadiazin-2-on
3-(4-(Pyrimidin-2-carbonylamino)-benzyl)-5-(4-methylsulfonylphenyl)-6-ethyl-3,6-dihydro-1,3,4-oxadiazin-2-on
3-(4-(Pyrimidin-2-carbonylamino)-benzyl)-5-(4-methylenoxyphenyl)-6-ethyl-3,6-dihydro-1,3,4-oxadiazin-2-on
3-(4-(Pyrimidin-2-carbonylamino)-benzyl)-5-(3-cyclopentyloxy-4-methoxy-phenyl)-6-ethyl-3,6-dihydro-1,3,4-oxadiazin-2-on
3-(3-(Pyrimidin-2-carbonylamino)-benzyl)-5-(3-cyclopentyloxy-4-methoxy-phenyl)-6-ethyl-3,6-dihydro-1,3,4-oxadiazin-2-on
3-(4-(Pyrimidin-2-carbonylamino)-phenethyl)-5-(3,4-dimethoxyphenyl)-3,6-dihydro-1,3,4-oxadiazin-2-on
3-(4-(Pyrimidin-2-carbonylamino)-phenethyl)-5-(3,4-dimethoxyphenyl)-6-ethyl-3,6-dihydro -1,3,4-oxadiazin-2-on.

Analog erhält man durch Umsetzung der oben aufgeführten "Aminderivate" mit Pyrazol-3-carbonsäurechlorid die nachstehenden Verbindungen
2-(4-(Pyrazol-3-carbonylamino)-benzyl)-6-(3,4-dimethoxyphenyl)-2,3,4,5-tetrahydro-pyridazin-3-on
2-(3-(Pyrazol-3-carbonylamino)-benzyl)-6-(3,4-dimethoxyphenyl)-2,3,4,5-tetrahydro-pyridazin-3-on
2-(2-(Pyrazol-3-carbonylamino)-benzyl)-6-(3,4-dimethoxyphenyl)-2,3,4,5-tetrahydro-pyridazin-3-on
2-(4-(Pyrazol-3-carbonylamino)-benzyl)-6-(3,4-dimethoxyphenyl)-5-ethyl-2,3,4,5-tetrahydro-pyridazin-3-on;
2-(3-(Pyrazol-3-carbonylamino)-benzyl)-6-(3,4-dimethoxyphenyl)-5-ethyl-2,3,4,5-tetrahydro-pyridazin-3-on
2-(2-(Pyrazol-3-carbonylamino)-benzyl)-6-(3,4-dimethoxyphenyl)-5-ethyl-2,3,4,5-tetrahydro-pyridazin-3-on
2-(4-(Pyrazol-3-carbonylamino)-benzyl)-6-(3-methoxy-4-trifluormethoxyphenyl)-5-ethyl-2,3,4,5-tetrahydro-pyridazin-3-on
2-(4-(Pyrazol-3-carbonylamino)-benzyl)-6-(3-methoxy-4-difluormethoxyphenyl)-5-ethyl-2,3,4,5-tetrahydro-pyridazin-3-on
2-(4-(Pyrazol-3-carbonylamino)-benzyl)-6-(3-methoxy-4-fluor-methoxyphenyl)-5-ethyl-2,3,4,5-tetrahydro-pyridazin-3-on
2-(4-(Pyrazol-3-carbonylamino)-benzyl)-6-(3-difluormethoxy-4-methoxyphenyl)-5-ethyl-2,3,4,5-tetrahydro-pyridazin-3-on
2-(4-(Pyrazol-3-carbonylamino)-benzyl)-6-(3-trtfluormethoxy-4-methoxyphenyl)-5-ethyl-2,3,4,5-tetrahydro-pyridazin-3-on
2-(4-(Pyrazol-3-carbonylamino)-benzyl)-6-(3-fluormethoxy-4-methoxyphenyl)-5-ethyl-2,3,4,5-tetrahydro-pyridazin-3-on
2-(4-(Pyrazol-3-carbonylamino)-benzyl)-6-(3-methoxy-4-ethoxy-phenyl)-5-ethyl-2,3,4,5-tetrahydro-pyridazin-3-on
2-(4-(Pyrazol-3-carbonylamino)-benzyl)-6-(3-ethoxy-4-methoxy-phenyl)-5-ethyl-2,3,4,5-tetrahydro-pyridazin-3-on
2-(4-(Pyrazol-3-carbonylamino)-benzyl)-6-(3-hydroxy-4-methoxyphenyl)-5-ethyl-2,3,4,5-tetrahydro-pyridazin-3-on
2-(4-(Pyrazol-3-carbonylamino)-benzyl)-6-(4-methylsulfonylphenyl)-5-ethyl-2,3,4,5-tetrahydro-pyridazin-3-on
2-(4-(Pyrazol-3-carbonylamino)-benzyl)-6-(4-methylenoxyphenyl)-5-ethyl-2,3,4,5-tetrahydro-pyridazin-3-on
2-(4-(Pyrazol-3-carbonylamino)-benzyl)-6-(3-cyclopentyloxy-4-methoxyphenyl)-5-ethyl-2,3,4,5-tetrahydro-pyridazin-3-on
2-(3-(Pyrazol-3-carbonylamino)-benzyl)-6-(3-cyclopentyloxy-4-methoxyphenyl)-5-ethyl-2,3,4,5-tetrahydro-pyridazin-3-on
2-(4-(Pyrazol-3-carbonylamino)-phenethyl)-6-(3,4-dimethoxyphenyl)-2,3,4,5-tetrahydro-pyridazin-3-on
2-(4-(Pyrazol-3-carbonylamino)-phenethyl)-6-(3,4-dimethoxyphenyl)-5-ethyl-2,3,4,5-tetrahydro-pyridazin-3-on
3-(4-(Pyrazol-3-carbonylamino)-benzyl)-5-(3,4-dimethoxyphenyl)-3,6-dihydro-1,3,4-thiadiazin-2-on
3-(3-(Pyrazol-3-carbonylamino)-benzyl)-5-(3,4-dimethoxyphenyl)-3,6-dihydro-1,3,4-thiadiazin-2-on
3-(2-(Pyrazol-3-carbonylamino)-benzyl)-5-(3,4-dimethoxyphenyl)-3,6-dihydro-1,3,4-thiadiazin-2-on
3-(4-(Pyrazol-3-carbonylamino)-benzyl)-5-(3,4-dimethoxyphenyl)-6-ethyl-3,6-dihydro-1,3,4-thiadiazin-2-on
3-(3-(Pyrazol-3-carbonylamino)-benzyl)-5-(3,4-dimethoxyphenyl)-6-ethyl-3,6-dihydro-1,3,4-thiadiazin-2-on
3-(2-(Pyrazol-3-carbonylamino)-benzyl)-5-(3,4-dimethoxyphenyl)-6-ethyl-3,6-dihydro-1,3,4-thiadiazin-2-on
3-(4-(Pyrazol-3-carbonylamino)-benzyl)-5-(3-methoxy-4-trifluormethoxyphenyl)-6-ethyl-3,6-dihydro-1,3,4-thiadiazin-2-on
3-(4-(Pyrazol-3-carbonylamino)-benzyl)-5-(3-methoxy-4-difluormethoxyphenyl)-6-ethyl-3,6-dihydro-1,3,4-thiadiazin-2-on
3-(4-(Pyrazol-3-carbonylamino)-benzyl-5-(3-methoxy-4-fluor-methoxyphenyl)-6-ethyl-3,6-dihydro-1,3,4-thiadiazin-2-on
3-(4-(Pyrazol-3-carbonylamino)-benzyl)-5-(3-difluormethoxy-4-methoxyphenyl)-6-ethyl-3,6-dihydro-1,3,4-thiadiazin-2-on
3-(4-(Pyrazol-3-carbonylamino)-benzyl)-5-(3-trifluormethoxy-4-methoxyphenyl)-6-ethyl-3,6-dihydro-1,3,4-thiadiazin-2-on
3-(4-(Pyrazol-3-carbonylamino)-benzyl)-5-(3 fluormethoxy-4-methoxyphenyl)-6-ethyl-3,6-dihydro -1,3,4-thiadiazin-2-on
3-(4-(Pyrazol-3-carbonylamino)-benzyl)-5-(3-methoxy-4-ethoxy-phenyl)-6-ethyl-3,6-dihydro-1,3,4-thiadiazin-2-on
3-(4-(Pyrazol-3-carbonylamino)-benzyl)-5-(3-ethoxy-4-methoxy-phenyl)-6-ethyl-3,6-dihydro-1,3,4-thiadiazin-2-on
3-(4-(Pyrazol-3-carbonylamino)-benzyl)-5-(3-hydroxy-4-methoxyphenyl)-6-ethyl-3,6-dihydro-1,3,4-thiadiazin-2-on
3-(4-(Pyrazol-3-carbonylamino)-benzyl)4-(4-methylsulfonylphenyl)-6-ethyl-3,6-dihydro-1,3,4-thiadiazin-2-on
3-(4-(Pyrazol-3-carbonylamino)-benzyl)-5-(4-methylenoxyphenyl)-6-ethyl-3,6-dihydro-1,3,4-thiadiazin-2-on
3-(4-(Pyrazol-3-carbonylamino)-benzyl)-5-(3-cyclopentyloxy-4-methoxyphenyl)-6-ethyl-3,6-dihydro-1,3,4-thiadiazin-2-on
3-(3-(Pyrazol-3-carbonylamino)-benzyl)-5-(3-cyclopentyloxy-4-methoxyphenyl)-6-ethyl-3,6-dihydro-1,3,4-thiadiazin-2-on
3-(4-(Pyrazol-3-carbonylamino)-phenethyl)-5-(3,4-dimethoxyphenyl)-3,6-dihydro-1,3,4-thiadiazin-2-on
3-(4-(Pyrazol-3-carbonylamino)-phenethyl)-5-(3,4-dimethoxyphenyl)-6-ethyl-3,6-dihydro-1,3,4-thiadiazin-2-on
3-(4-(Pyrazol-3-carbonylamino)-benzyl)-5-(3,4-dimethoxyphenyl)-3,6-dihydro-1,3,4-oxadiazin-2-on
3-(3-(Pyrazol-3-carbonylamino)-benzyl)-5-(3,4-dimethoxyphenyl)-3,6-dihydro-1,3,4-oxadiazin-2-on
3-(2-(Pyrazol-3-carbonylamino)-benzyl)-5-(3,4-dimethoxyphenyl)-3,6-dihydro-1,3,4-oxadiazin-2-on
3-(4-(Pyrazol-3-carbonylamino)-benzyl)-5-(3,4-dimethoxyphenyl)-6-ethyi-3,6-dihydro-1,3,4-oxadiazin-2-on
3-(3-(Pyrazol-3-carbonylamino)-benzyl)-5-(3,4-dimethoxyphenyl)-6-ethyl-3,6-dihydro-1,3,4-oxadiazin-2-on
3-(2-(Pyrazol-3-carbonylamino)-benzyl)-5-(3,4-dimethoxyphenyl)-6-ethyl-3,6-dihydro-1,3,4-oxadiazin-2-on
3-(4-(Pyrazol-3-carbonylamino)-benzyl)-5-(3-methoxy-4-trifluor-methoxyphenyl)-6-ethyl-3,6-dihydro-1,3,4-oxadiazin-2-on
3-(4-(Pyrazol-3-carbonylamino)-benzyl)-5-(3-methoxy-4-difluormethoxyphenyl)-6-ethyl-3,6-dihydro-1,3,4-oxadiazin-2-on
3-(4-(Pyrazol-3-carbonylamino)-benzyl)-5-(3-methoxy-4-fluor-methoxy-phenyl)-6-ethyl-3,6-dihydro-1,3,4-oxadiazin-2-on
3-(4-(Pyrazol-3-carbonylamino)-benzyl)-5-(3-difluormethoxy-4-methoxyphenyl)-6-ethyl-3,6-dihydro-1,3,4-oxadiazin-2-on
3-(4-(Pyrazol-3-carbonylamino)-benzyl)-5-(3-trifluormethoxy-4-methoxyphenyl)-6-ethyl-3,6-dihydro-1,3,4-oxadiazin-2-on
3-(4-(Pyrazol-3-carbonylamino)-benzyl)-5-(3-fluormethoxy-4-methoxyphenyl)-6-ethyl-3,6-dihydro-1,3,4-oxadiazin-2-on
3-(4-(Pyrazol-3-carbonylamino)-benzyl)-5-(3-methoxy-4-ethoxy-phenyl)-6-ethyl-3,6-dihydro-1,3,4-oxadiazin-2-on
3-(4-(Pyrazol-3-carbonylamino)-benzyl)-5-(3-ethoxy-4-methoxy-phenyl)-6-ethyl-3,6-dihydro-1,3,4-oxadiazin-2-on
3-(4-(Pyrazol-3-carbonylamino)-benzyl)-5-(3-hydroxy-4-methoxyphenyl)-6-ethyl-3,6-dihydro-1,3,4-oxadiazin-2-on
3-(4-(Pyrazol-3-carbonylamino)-benzyl)-5-(4-methylsulfonylphenyl)-6-ethyl-3,6-dihydro-1,3,4-oxadiazin-2-on
3-(4-(Pyrazol-3-carbonylamino)-benzyl)-5-(4-methylenoxyphenyl)-6-ethyl-3,6-dihydro-1,3,4-oxadiazin-2-on
3-(4-(Pyrazol-3-carbonylamino)-benzyl)-5-(3-cyclopentyloxy-4-methoxyphenyl)-6-ethyl-3,6-dihydro-1,3,4-oxadiazin-2-on
3-(3-(Pyrazol-3-carbonylamino)-benzyl)-5-(3-cyclopentyloxy-4-methoxyphenyl)-6-ethyl-3,6-dihydro-1,3,4-oxadiazin-2-on
3-(4-(Pyrazol-3-carbonylamino)-phenethyl)-5-(3,4-dimethoxyphenyl)-3,6-dihydro-1,3,4-oxadiazin-2-on
3-(4-(Pyrazol-3-carbonylamino)-phenethyl)-5-(3,4-dimethoxyphenyl)-6-ethyl-3,6-dihydro-1,3,4-oxadiazin-2-on.

Analog erhält man durch Umsetzung
von 2-(3-Aminobenzyl)-6-(3-ethoxy-4-methoxyphenyl)-2,3,4,5-tetrahydro-pyridazin-3-on mit Nicotinoylchlorid
2-(3-Nicotinoylaminobenzyl)-6-(3-ethoxy-4-methoxyphenyl)-2,3,4,5-tetrahydropyridazin-3-on, F. 204°
von 2-(4-Aminobenzyl)-6-(3-ethoxy-4-methoxyphenyl)-2,3,4,5-tetrahydropyridazin-3-on
mit Isonicotinoylchlorid
2-(4-lsonicotinoylaminobenzyl)-6-(3-ethoxy-4-methoxyphenyl)-2,3,4,5-tetrahydropyridazin-3-on, Hydrochlorid F. 252°
mit Pyrazin-2-carbonsäurechlorid
2-(4-Pyrazincarbonylaminobenzyl)-6-(3-ethoxy-4-methoxyphenyl)-2,3,4,5-tetrahydropyridazin-3-on, F. 202°
mit Isoxazol-5-carbonsäurechlorid
2-(4-(Isoxazol-5-carbonylamino)-benzyl)-6-(3-ethoxy-4-methoxyphenyl)-2,3,4,5-tetrahydropyridazin-3-on, F. 192°
von 2-(4 Aminobenzyl)-6-(3-cyclopentyloxy-4-methoxyphenyl)-2,3,4,5-tetrahydropyridazin-3-on mit Nicotinoylchlorid
2-(4-Nicotinoylaminobenzyl)-6-(3-cyclopentyloxy-4-methoxyphenyl)-2,3,4,5-tetrahydropyridazin-3-on, F. 205°
von 2-(4-Aminobenzyl)-(3,4-dimethoxyphenyl)-2,3,4,5-tetrahydropyridazin-3-on mit Nicotinoylchlorid
2-(4-Nicotinoylaminobenzyl)-6-(3,4-dimethoxyphenyl)-2,3,4,5-tetrahydropyridazin-3-on, Hydrochlorid F. 212°.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel Imit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 l isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen der Formel I 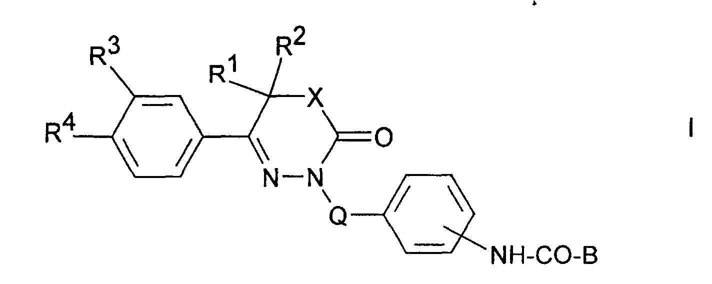 worin
B 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5- Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6- Pyrimidinyl, 1,2,3-Triazol-1-, -4-oder-5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 2-, 3-, 4-, 5- 6- oder 7- Benzofuryl, 2-, 3-, 4-, 5-, 6- oder 7-Benzothienyl, 1-, 2-, 3-, 4-, 5-, 6- oder 7-Indolyl, 1-, 2-, 4- oder 5-Benzimidazolyl, 1-, 3-, 4-, 5-, 6- oder 7-Benzopyrazolyl, 2-, 4-, 5-, 6- oder 7- Benzoxazolyl, 3-, 4-, 5-, 6- oder 7- Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8- Chinazolinyl,
Q fehlt oder Alkylen mit 1-6 C-Atomen,
X CH_{z}, S oder 0,
R¹ und R² jeweils unabhängig voneinander H oder A,
R³ und R⁴ jeweils unabhängig voneinander -OH, OR⁵, -S-R⁵, -SO-R⁵, - SO₂-R⁵, Hal, Methylendioxy, -NO₂, -NH₂; -NHR⁵ oder -NR⁵R⁶,
R⁵ und R⁶ jeweils unabhängig voneinander A, Cycloalkyl mit 3-7 C-Atomen, Methylencycloalkyl mit 4-8 C-Atomen oder Alkenyl mit 2-8 C-Atomen,
A Alkyl mit 1 bis 10 C-Atomen, das durch 1 bis 5 F- und/oder Cl-Atome substituiert sein kann und
Hal F, Cl, Br oder l
bedeuten,
sowie deren physiologisch unbedenklichen Salze.

2. Ein Enantiomer einer Verbindung der Formel I gemäß Anspruch 1.

3. Verbindungen nach Anspruch 1
(a) 3-(4-Picolinoylamino-benzyl)-5-(3,4-dimethoxyphenyl)-6-ethyl-3,6-dihydro-1,3,4-thiadiazin-2-on;
(b) 3-(4-Picolinoylamino-benzyl)-5-(3,4-dimethoxyphenyl)-3,6-dihydro-1,3,4-thiadiazin-2-on;
(c) 3-(4-Nicotinoylamino-benzyl)-5-(3,4-dimethoxyphenyl)-3,6-dihydro-1,3,4-thiadiazin-2-on;
(d) 3-(4-lsonicotinoylamino-benzyl)-5-(3,4-dimethoxyphenyl)-3,6-dihydro-1,3,4-thiadiazin-2-on;
(e) 3-(4-Nicotinoylamino-benzyl)-5-(3,4-dimethoxyphenyl)-6-ethyl-3,6-dihydro-1,3,4-oxadiazin-2-on;
(f) 2-(4-Nicotinoylamino-benzyl)-6-(3,4-dimethoxyphenyl)-5-ethyl-2,3,4,5-tetrahydro-pyridazin-3-on.

4. Verfahren zur Herstellung von Verbindungen der Formel l nach Anspruch 1 sowie deren Salzen, **dadurch gekennzeichnet, daß** man eine Verbindung der Formel II 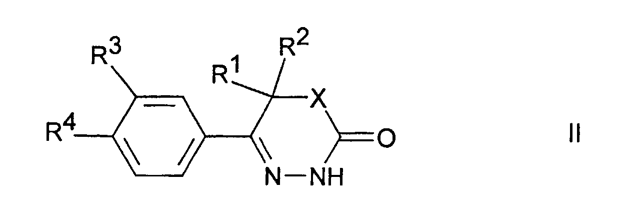 worin
R¹, R², R³_{,} R⁴ und X die angegebenen Bedeutungen haben,
mit einer Verbindung der Formel III 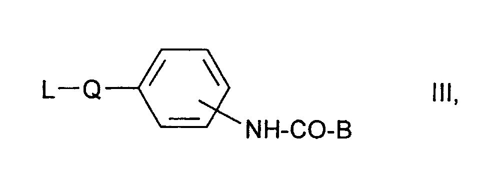 worin
B und Q die angegebenen Bedeutungen haben, und
L Cl, Br, OH oder eine reaktionsfähige veresterte OH-Gruppe bedeutet,
umsetzt,
oder
daß man eine Verbindung der Formel IV 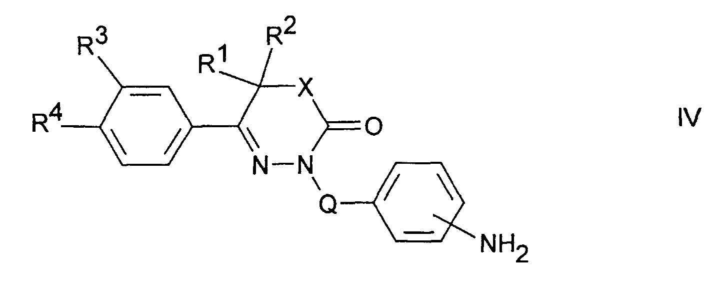 worin
R¹, R², R³_{,} R⁴, Q und X die angegebenen Bedeutungen haben, mit einer Verbindung der Formel V
L-CO-B V
worin
B die angegebene Bedeutung hat, und
L Cl, Br, OH oder eine reaktionsfähige veresterte OH-Gruppe bedeutet,
umsetzt,
und/oder daß man eine basische Verbindung der Formel I durch Behandeln mit einer Säure in eines ihrer Salze überführt.

5. Verfahren zur Herstellung pharmazeutischer Zubereitungen, **dadurch gekennzeichnet, daß** man eine Verbindung der Formel I nach Anspruch 1 und/oder eines ihrer physiologischen unbedenklichen Salze zusammen mit mindestens einem festen, flüssigen oder halbflüssigen Träger- oder Hilfsstoff in eine geeignete Dosierungsform bringt.

6. Pharmazeutische Zubereitung, **gekennzeichnet durch** einen Gehalt an mindestens einer Verbindung der Formel I nach Anspruch 1 und/oder einem ihrer physiologisch unbedenklichen Salze.

7. Verbindungen der Formel I nach Anspruch 1 und ihre physiologisch unbedenklichen Salze zur Bekämpfung von Asthma, Allergien und entzündlichen Krankheiten, Autoimmunerkrankungen und Transplantatabstoßungsreaktionen.

8. Arzneimittel der Formel I nach Anspruch 1 und ihre physiologisch unbedenklichen Salze als Phosphodiesterase IV-Hemmer.

9. Verwendung von Verbindungen der Formel I nach Anspruch 1 und/oder ihre physiologisch unbedenklichen Salze zur Herstellung eines Arzneimittels.

## Claims

1. Compounds of the formula I 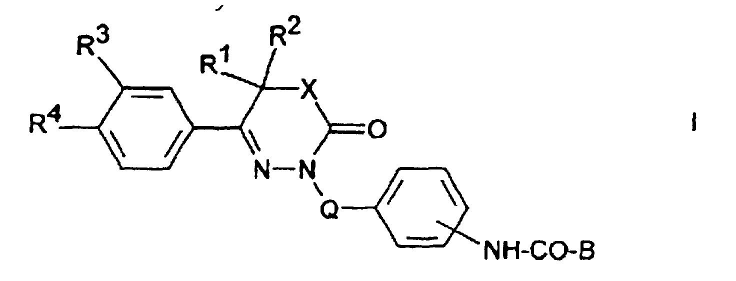 in which
B is 2- or 3-furyl, 2- or 3-thienyl, 1-, 2- or 3-pyrrolyl, 1-, 2-, 4-or 5-imidazolyl, 1-, 3-, 4- or 5-pyrazolyl, 2-, 4- or 5-oxazolyl, 3-, 4- or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, 1,2,3-triazol-1-,-4- or -5-yl, 1,2,4-triazol-1-, -3- or -5-yl, 1-or 5-tetrazolyl, 1,2,3-oxadiazol-4- or -5-yl, 1,2,4-oxadiazol-3- or -5-yl, 1,3,4-thiadiazol-2- or -5-yl, 1,2,4-thiadiazol-3- or -5-yl, 1,2,3-thiadiazol-4- or -5-yl, 3- or 4-pyridazinyl, pyrazinyl, 2-, 3-, 4-, 5-, 6- or 7-benzofuryl, 2-, 3-, 4-, 5-, 6-or 7-benzothienyl, 1-, 2-, 3-, 4-, 5-, 6- or 7-indolyl, 1-, 2-, 4-or 5-benzimidazolyl, 1-, 3-, 4-, 5-, 6- or 7-benzopyrazolyl, 2-, 4-, 5-, 6- or 7-benzoxazolyl, 3-, 4-, 5-, 6- or 7-benzisoxazolyl, 2-, 4-, 5-, 6- or 7-benzothiazolyl, 2-, 4-, 5-, 6- or 7-benzisothiazolyl, 4-, 5-, 6- or 7-benz-2,1,3-oxadiazolyl, 2-, 3-, 4-, 5-, 6-, 7- or 8-quinolyl, 1-, 3-, 4-, 5-, 6-, 7- or 8-isoquinolyl, 3-, 4-, 5-, 6-, 7- or 8-cinnolinyl, 2-, 4-, 5-, 6-, 7-or 8-quinazolinyl,
Q is absent or is alkylene having 1-6 carbon atoms,
X is CH_{z}, S or 0,
R¹ and R² are each, independently of one another, H or A,
R³ and R⁴ are each, independently of one another, -OH, OR⁵, -S-R⁵, - SO-R⁵, -SO₂-R⁵, Hal, methylenedioxy, -NO₂, -NH₂, -NHR⁵ or -NR⁵R⁶,
R⁵ and R⁶ are each, independently of one another, A, cycloalkyl having 3-7 carbon atoms, methylenecycloalkyl having 4-8 carbon atoms or alkenyl having 2-8 carbon atoms,
A is alkyl having from 1 to 10 carbon atoms, which may be substituted by from 1 to 5 F and/or Cl atoms, and
Hal is F, Cl, Br or I,
and physiologically acceptable salts thereof.

2. Enantiomer of a compound of the formula I according to Claim 1.

3. Compounds according to Claim 1
(a) 3-(4-picolinoylaminobenzyl)-5-(3,4-dimethoxyphenyl)-6-ethyl-3,6-dihydro-1,3,4-thiadiazin-2-one;
(b) 3-(4-picolinoylaminobenzyl)-5-(3,4-dimethoxyphenyl)-3,6-dihydro-1,3,4-thiadiazin-2-one;
(c) 3-(4-nicotinoylaminobenzyl)-5-(3,4-dimethoxyphenyl)-3,6-dihydro-1,3,4-thiadiazin-2-one;
(d) 3-(4-isonicotinoylaminobenzyl)-5-(3,4-dimethoxyphenyl)-3,6-dihydro-1,3,4-thiadiazin-2-one;
(e) 3-(4-nicotinoylaminobenzyl)-5-(3,4-dimethoxyphenyl)-6-ethyl-3,6-dihydro-1,3,4-oxadiazin-2-one;
(f) 2-(4-(nicotinoylaminobenzyl)-6-(3,4-dimethoxyphenyl)-5-ethyl-2,3,4,5-tetrahydropyridazin-3-one.

4. Process for the preparation of compounds of the formula I according to Claim 1 and their salts, **characterised in that** a compound of the formula II 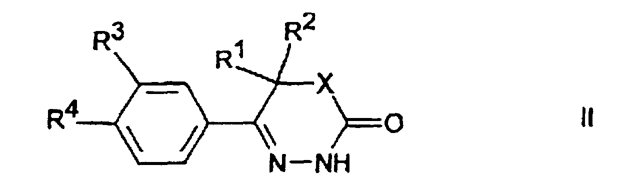 in which
R¹, R², R³, R⁴ and X have the meanings indicated,
is reacted with a compound of the formula III 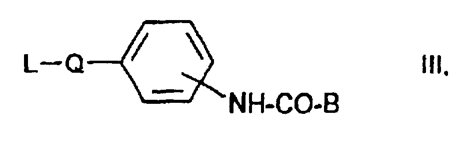 in which
B and Q have the meanings indicated, and
L is Cl, Br, OH or a reactive esterified OH group,
or
**in that** a compound of the formula IV 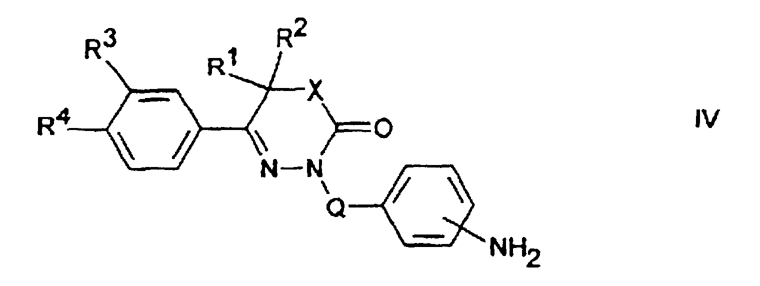 in which
R¹, R², R³, R⁴, Q and X have the meanings indicated,
is reacted with a compound of the formula V
L-CO-B V
in which
B has the meaning indicated, and
L is Cl, Br, OH or a reactive esterified OH group,
and/or **in that** a basic compound of the formula I is converted into one of its salts by treatment with an acid.

5. Process for the preparation of pharmaceutical preparations, **characterised in that** a compound of the formula I according to Claim 1 and/or a physiologically acceptable salt thereof is converted into a suitable dosage form together with at least one solid, liquid or semiliquid excipient or adjuvant.

6. Pharmaceutical preparation, **characterised by** a content of at least one compound of the formula I according to Claim 1 and/or a physiologically acceptable salt thereof.

7. Compounds of the formula I according to Claim 1 and physiologically acceptable salts thereof for combating asthma, allergies and inflammatory diseases, autoimmune diseases and transplant rejection reactions.

8. Medicaments of the formula I according to Claim 1 and physiologically acceptable salts thereof as phosphodiesterase IV inhibitors.

9. Use of compounds of the formula I according to Claim 1 and/or physiologically acceptable salts thereof for the preparation of a medicament.

## Revendications

1. Composés répondant à la formule I 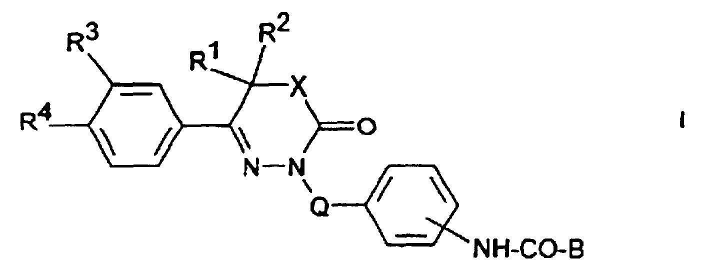 dans laquelle
B représente 2- ou 3-furyle, 2- ou 3-thiényle, 1-, 2- ou 3-pyrrolyle, 1-, 2-, 4- ou 5-imidazolyle, 1-, 3-, 4- ou 5-pyra-zolyle, 2-, 4- ou 5-oxazolyle, 3-, 4- ou 5-isoxazolyle, 2-, 4-ou 5-thiazolyle, 3-, 4- ou 5-isothiazolyle, 2-, 3- ou 4-pyridyle, 2-, 4-, 5- ou 6-pyrimidinyle, 1,2,3-triazol-1-, -4- ou -5-yle, 1,2,4-triazol-1-, -3- ou -5-yle, 1- ou 5-tétrazolyle, 1,2,3-oxadiazol-4- ou -5-yle, 1,2,4-oxadiazol-3- ou -5-yle, 1,3,4-thiadiazol-2- ou -5-yle, 1,2,4-thiadiazol-3- ou -5-yle, 1,2,3-thiadiazol-4- ou -5-yle, 3- ou 4-pyridazinyle, pyrazinyle, 2-, 3-, 4-, 5-, 6- ou 7-benzofuryle, 2-, 3-, 4-, 5-, 6- ou 7-benzothiényle, 1-, 2-, 3-, 4-, 5-, 6- ou 7-indolyle, 1-, 2-, 4- ou 5-benzimidazolyle, 1-, 3-, 4-, 5-, 6- ou 7-benzopyrazolyle, 2-, 4-, 5-, 6- ou 7-benzoxazolyle, 3-, 4-, 5-, 6- ou 7-benzisoxazolyle, 2-, 4-, 5-, 6- ou 7-benzothiazolyle, 2-, 4-, 5-, 6- ou 7-benzisothiazolyle, 4-, 5-, 6- ou 7-benz-2,1,3-oxadiazolyle, 2-, 3-, 4-, 5-, 6-, 7- ou 8-quinolyle, 1-, 3-, 4-, 5-, 6-, 7- ou 8-isoquinolyle, 3-, 4-, 5-, 6-, 7- ou 8-cinnolinyle, 2-, 4-, 5-, 6-, 7- ou 8-quinazolinyle,
Q est absent ou représente alkylène ayant de 1 à 6 atomes de carbone,
X représente CH₂, S ou O,
R¹ et R² représentent chacun, indépendamment l'un de l'autre, H ou A,
R³ et R⁴ représentent chacun, indépendamment l'un de l'autre, -OH, OR⁵, -S-R⁵, -SO-R⁵, -SO₂-R⁵, Hal, méthylènedioxy, - NO₂, -NN₂, -NHR⁵ ou -NR⁵R⁶,
R⁵ et R⁶ représentent chacun, indépendamment l'un de l'autre, A, cycloalkyle ayant de 3 à 7 atomes de carbone, méthylènecycloalkyle ayant de 4 à 8 atomes de carbone ou alkényle ayant de 2 à 8 atomes de carbone,
A représente alkyle ayant de 1 à 10 atomes de carbone, pouvant être substitué par de 1 à 5 atomes F et/ou Cl, et
Hal représente F, Cl, Br ou I,
et les sels physiologiquement acceptables de ceux-ci.

2. Enantiomère d'un composé répondant à la formule I selon la revendication 1.

3. Composés selon la revendication 1
(a) la 3-(4-picolinoylaminobenzyl)-5-(3,4-diméthoxyphényl)-6-éthyl-3,6-dihydro-1,3,4-thiadiazin-2-one;
(b) la 3-(4-picolinoylaminobenzyl)-5-(3,4-diméthoxyphényl)-3,6-dihydro-1,3,4-thiadiazin-2-one;
(c) la 3-(4-nicotinoylaminobenzyl)-5-(3,4-diméthoxyphényl)-3,6-dihydro-1,3,4-thiadiazin-2-one;
(d) la 3-(4-isonicotinoylaminobenzyl)-5-(3,4-diméthoxyphényl)-3,6-dihydro-1,3,4-thiadiazin-2-one;
(e) la 3-(4-nicotinoylaminobenzyl)-5-(3,4-diméthoxyphényl)-6-éthyl-3,6-dihydro-1,3,4-oxadiazin-2-one;
(f) la 2-(4-(nicotinoylaminobenzyl)-6-(3,4-diméthoxyphényl)-5-éthyl-2,3,4,5-tétrahydropyridazin-3-one.

4. Procédé de préparation de composés répondant à la formule I selon la revendication 1 et de leurs sels, **caractérisé en ce qu'**un composé répondant à la formule II 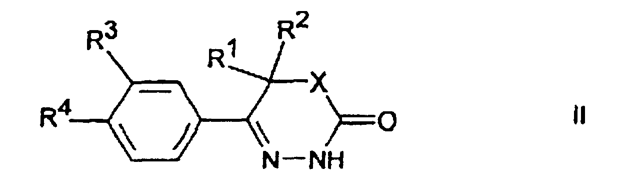 dans laquelle
R¹, R², R³, R⁴ et X ont les significations indiquées,
est réagi avec un composé répondant à la formule III 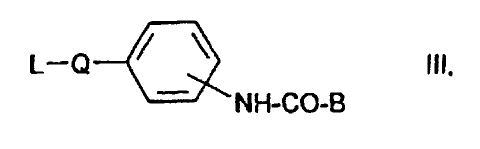 dans laquelle
B et Q ont les significations indiquées, et
L représente Cl, Br, OH ou un groupement OH estérifié réactif,
ou
**en ce qu'**un composé répondant à la formule IV 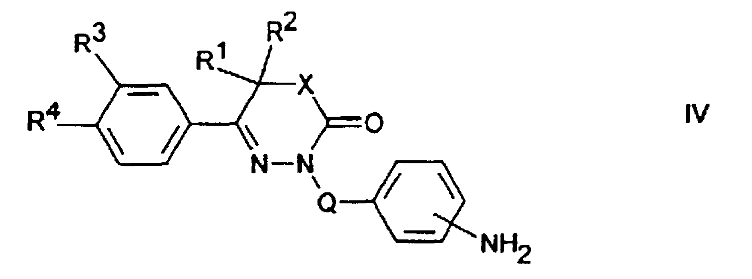 dans laquelle
R¹, R², R³, R⁴, Q et X ont les significations indiquées,
est réagi avec un composé répondant à la formule V
L-CO-B V
dans laquelle
B a la signification indiquée, et
L représente Cl, Br, OH ou un groupement OH estérifié réactif,
et/ou **en ce qu'**un composé basique répondant à la formule I est converti en l'un de ses sels par traitement par un acide.

5. Procédé de préparation de préparations pharmaceutiques, **caractérisé en ce qu'**un composé répondant à la formule I selon la revendication 1 et/ou un sel physiologiquement acceptable de celui-ci est mis sous une forme de dosage convenable conjointement avec au moins un excipient ou adjuvant solide, liquide ou semi-liquide.

6. Préparation pharmaceutique, **caractérisée par** une teneur en au moins un composé répondant à la formule I selon la revendication 1 et/ou un sel physiologiquement acceptable de celui-ci.

7. Composés répondant à la formule l selon la revendication 1 et les sels physiologiquement acceptables de ceux-ci pour lutter contre l'asthme, les allergies et les maladies inflammatoires, les maladies auto-immunes et les réactions de rejet de greffon.

8. Médicaments répondant à la formule I selon la revendication 1 et les sels physiologiquement acceptables de ceux-ci comme inhibiteurs de la phosphodiestérase IV.

9. Utilisation de composés répondant à la formule I selon la revendication 1 et/ou de sels physiologiquement acceptables de ceux-ci pour la préparation d'un médicament.
